# EUROPEAN PATENT APPLICATION

(11) **EP 4 647 489 A1**
(43) Date of publication of application: **12.11.2025**
(21) Application number: 23914595.6
(22) Date of filing: 29.12.2023
(51) Int. Cl.: C12N 1/21, C12N 15/74, A61K 35/74, A61P 35/00

(54) **BACTERIA SECRETING INTERLEUKIN-10**

(30) Priority: 03.01.2023 CN 202310004294
(71) Applicant: Shenzhen Synthetica Pioneering Co., Ltd., Shenzhen, Guangdong 518107 (CN)
(72) Inventor: LIU, Chenli, Shenzhen, Guangdong 518055 (CN); DONG, Yuxuan, Shenzhen, Guangdong 518055 (CN); LI, Yang, Shenzhen, Guangdong 518055 (CN); CHEN, Qian, Shenzhen, Guangdong 518055 (CN)
(74) Representative: Michalski Hüttermann & Partner Patentanwälte mbB
(86) International application number: PCT/CN2023/143031
(87) International publication number: WO 2024/146463

(57) **Abstract**

The present invention relates to a fusion polypeptide comprising an IL10 polypeptide, wherein the fusion polypeptide can be prokaryotic expressed and secreted in bacterial cells. The present invention also relates to modified bacteria having anti-tumor activity and capable of prokaryotically expressing and secreting IL10 polypeptides in tumors, the bacterium comprises essential gene expression cassette controlled by a strictly hypoxia inducible promoter, and the bacteria are missing at least one gene participating in or regulating an endogenous anti-oxidative stress response pathway or a functional expression product thereof. The present invention also relates to a pharmaceutical composition comprising the modified bacteria and anti-tumor use thereof.

## Description

### Technical Field

The present invention relates to the field of genetic engineering. Specifically, the present invention relates to a bacterium with anti-tumor activity that is genetically modified to secrete interleukin-10 in situ, a pharmaceutical composition comprising the modified bacterium and the anti-tumor use thereof.

### Background

Cancer has become one of the most prevalent diseases threatening human life and health, but existing treatments (radiotherapy, chemotherapy, surgery and targeted drugs) all have shortcomings and there is an urgent need to develop new treatment approaches. Traditional bacterial tumor therapies, represented by Coley's toxin, have a history of 150 years, but are known for their unstable efficacy and safety issues. The development of genetic engineering technology has made great progress in reducing the toxicity of bacterial strains, and a series of clinical trials have demonstrated the safety of the attenuated engineered bacteria for human tumor therapy but with limited efficacy, i.e., it is difficult to have both safety and therapeutic efficacy, thus failing to satisfy the needs for tumor therapy. Further optimization for tumor therapy is imperative.

Interleukin 10 (IL-10) is a cytokine mainly produced by Th2 cells. It can inhibit the production of other lymphokines, and is a major anti-inflammatory factor. It has certain therapeutic effects on transplant rejection, inflammation, etc. Interleukin-10 is initially considered as a T cell growth factor derived from B cells (B- TCGF), and thus, it is considered that the function of interleukin-10 is to stimulate the activation of CD8+T cells (MacNeil I A, et al. The Journal of Immunology, 1990). Subsequently, investigators have found that interleukin-10 can also induce the expression of CD3 and CD8 molecules in thymocytes, and activate the cytotoxicity of CD8+T cells (Chen W F, et al. The Journal of Immunology, 1991). In addition, interleukin-10 can enhance proliferation of CD8+T cells by directly stimulating T cell receptor signals via anti-CD3 monoclonal antibodies.

For the use of interleukin-10 in immunotherapy, it was initially found that interleukin-10 knocked out mice were particularly susceptive to chemically induced skin cancer, and it was found that tumorigenesis in interleukin-10 knocked out mice developed faster and early metastasis occurred. Intratumoral CD8+T cells, MHC molecules and granzyme are all inhibited in tumors in interleukin-10 knockout mice. In contrast, in transgenic mice overexpressing interleukin-10, the above molecules were increased upon the overexpression of interleukin-10 (Mumm JB, Emmerich J, Zhang X, Chan I, Wu L, Mauze S, Blaisdell S, Basham B, Dai J, Grein J, Sheppard C, Hong K, Cutler C, Turner S, LaFace D, Kleinschek M, Judo M, Ayanoglu G, Langowski J, Gu D, Paporello B, Murphy E, Sriram V, Naravula S, Desai B, Medicherla S, Seghezzi W, McClanahan T, Cannon-Carlson S, Beebe AM, Oft M. IL-10 elicits IFNγ-dependent tumor immune surveillance. Cancer Cell. 2011 Dec 13;20(6):781-96. doi: 10.1016/j.ccr.2011.11.003. PMID: 22172723). Martin Soft et al. found that the expression of IFN-γ and granzyme in tumors was significantly increased by injecting pegylated interleukin-10 (chemically modified interleukin-10 to prolong the half-life of interleukin-10) into tumor-bearing mice. They also found that pegylated interleukin-10 would activate the persistent immune memory against tumors in vivo, and the mice were protected against the challenge of tumor 8 months after the initial tumor rejection. This persistent tumor immunity may be due to the phosphorylation of STAT1 and STAT3 in CD8+T cells in the tumor induced by interleukin-10, which activates the signaling pathway specific for CD8+T cells in the tumor and stimulates the secretion of cytokine IFN-γ (Mumm JB, Emmerich J, Zhang X, Chan I, Wu L, Mauze S, Blaisdell S, Basham B, Dai J, Grein J, Sheppard C, Hong K, Cutler C, Turner S, LaFace D, Kleinschek M, Judo M, Ayanoglu G, Langowski J, Gu D, Paporello B, Murphy E, Sriram V, Naravula S, Desai B, Medicherla S, Seghezzi W, McClanahan T, Cannon-Carlson S, Beebe AM, Oft M. IL-10 elicits IFNγ-dependent tumor immune surveillance. Cancer Cell. 2011 Dec 13;20(6):781-96. doi: 10.1016/j.ccr.2011.11.003. PMID: 22172723). However, for CD4+ or CD8+ T cells derived from lymphoid organs, interleukin-10 can only induce the phosphorylation of STAT3 therein, but cannot induce the secretion of IFN-γ (Emmerich J, Mumm JB, Chan IH, LaFace D, Truong H, McClanahan T, Gorman DM, Oft M. IL-10 directly activates and expands tumor-resident CD8(+) T cells without de novo infiltration from secondary lymphoid organs. Cancer Res. 2012 Jul 15;72(14):3570-81. doi: 10.1158/0008-5472.CAN-12-0721. Epub 2012 May 11. PMID: 22581824). STAT1 is particularly important in the induction of IFN-γ because it induces the generation of cell-specific transcription factor T-bet, which is a transcription factor for the T cells of Th1 and Tc1 lineages that produce IFN-γ (Afkarian M, Sedy JR, Yang J, Jacobson NG, Cereb N, Yang SY, Murphy TL, Murphy KM. T-bet is a STAT1-induced regulator of IL-12R expression in naive CD4+ T cells. Nat Immunol. 2002 Jun;3(6):549-57. doi: 10.1038/ni794. Epub 2002 May 13. PMID: 12006974.). T-bet works synergistically with TCR-activated transcription factor NFAT to induce the generation of IFN-γ and granulase in cytotoxic T cells (Glimcher LH, Townsend MJ, Sullivan BM, Lord GM. Recent developments in the transcriptional regulation of cytolytic effector cells. Nat Rev Immunol. 2004 Nov;4(11):900-11. doi: 10.1038/nri1490. PMID: 15516969.). Therefore, with the effect of PEGylated interleukin-10, IFN-γ, granzyme and perforin in cytotoxic T cells were increased under the TCR stimulation only. In addition, intratumoral IFN-γ produced by CD8+T cells will be involved in the induction of MHC class I and MHC class II molecules, potentially allowing the antigen presentation in tumors. In human tumors, the expression of MHC molecules is highly associated with the improved prognosis of the patient (Walsh MD, Dent OF, Young JP, Wright CM, Barker MA, Leggett BA, Bokey L, Chapuis PH, Jass JR, Macdonald GA. HLA-DR expression is associated with better prognosis in sporadic Australian clinicopathological Stage C colorectal cancers. Int J Cancer. 2009 Sep 1;125(5):1231-7. doi: 10.1002/ijc.24484. PMID: 19462453.). In summary, interleukin-10 can improve tumor-related inflammatory response and tumor immune deficiency in tumor treatment, which fully indicates that it may represent a new method for treating cancer patients with broad prospects.

Therefore, bacteria with anti-tumor activity capable of secreting interleukin 10 in situ have an improved effect on treating tumors. However, although the bacteria can express IL-10, it is difficult to secrete the expressed IL-10 out of the cell. Therefore, there is a need to improve the expression methods and to prepare bacteria with anti-tumor activity that can secrete IL-10 *in situ.*

### Summary of the Invention

The present invention constructs a fusion polypeptide by linking the amino acid sequence of mature IL-10 to a signal peptide that directs the secretion of proteins out of the cell in prokaryotic cells by a peptide linker comprising 1-10 acidic amino acid residues. The fusion polypeptide of the present disclosure, when expressed in a prokaryotic cell, can be secreted out of the cell.

According to the present invention, a strictly anaerobic bacterium is constructed by means of synthetic biological genetic circuits, which shows therapeutic efficacy against tumors after being administered to an animal body or a human body, and can be cleared by normal tissues and organs in a short period of time, thereby reducing the toxic and side effects on the animal body or the human body due to the long-term retention of the bacterium in vivo, and thus providing a more reliable anti-tumor efficacy and safety.

In a first aspect, the present invention provides a fusion polypeptide, comprising a signal peptide, an interleukin 10 (IL-10) polypeptide, and a peptide linker linking the signal peptide and the IL-10 polypeptide, wherein the peptide linker comprises 1-10 acidic amino acid residues, and wherein when expressed in a cell, the signal peptide directs the secretion of the IL-10 polypeptide out of the cell.

In a second aspect, the present invention provides a polynucleotide encoding the fusion polypeptide of the present invention, a vector comprising the polynucleotide, and a host cell comprising the polynucleotide and/or the vector.

In a third aspect, the present invention provides a method for the prokaryotic expression of an IL-10 polypeptide, comprising:
i) transforming a bacterial cell with the vector of the invention; and
ii) culturing the transformed cell under a condition that enables the expression of IL-10 by the vector.

In a fourth aspect, the present invention provides a modified bacterium, wherein the bacterium, as compared to an unmodified starting strain, comprises one or more expression cassettes for expressing the polynucleotide of the invention and an essential gene, the expression of the essential gene is controlled by a strictly hypoxia inducible promoter, and the bacterium is deficient in at least one gene involved in or regulating an endogenous anti-oxidative stress response pathway or a functional expression product thereof.

The present invention also provides a modified bacterium, wherein the bacterium comprises, as compared to an unmodified starting strain, one or more expression cassettes for expressing the polynucleotide of the present invention and an essential gene, the expression of the essential genes is controlled by a strictly hypoxia inducible promoter, and the bacterium is deficient in at least one gene required for survival in a macrophage or a functional expression product thereof.

The present invention also provides a modified bacterium, wherein the bacterium comprises, as compared to an unmodified starting strain, one or more expression cassettes for expressing the polynucleotide of the present invention and an essential gene, the expression of which is controlled by a strictly hypoxia inducible promoter, and a pH-regulated expression cassette comprising a gene encoding a bacteria-derived hemolysin protein controlled by a promoter active under acidic pH conditions, and the bacterium is deficient in at least one gene involved in or regulating an endogenous anti-oxidative stress response pathway or a functional expression product thereof.

The present invention also provides a modified bacterium, wherein the bacterium comprises, as compared to an unmodified starting strain, one or more expression cassettes for expressing the polynucleotide of the present invention and an essential gene, the expression of which is controlled by a strictly hypoxia inducible promoter, and a pH-regulated expression cassette comprising a gene encoding a bacteria-derived hemolysin protein controlled by a promoter active under acidic pH conditions, wherein the bacterium expresses a wild-type lipopolysaccharide (LPS) and the bacterium is deficient in at least one gene required for survival in a macrophage or a functional expression product thereof.

The present invention also provides a modified Salmonella typhimurium bacterium, wherein the bacterium comprises, as compared to an unmodified starting strain, one or more expression cassettes for expressing the polynucleotides of the present invention and an essential gene, the expression of which is controlled by a strictly hypoxia inducible promoter, and a pH-regulated expression cassette comprising a gene encoding a bacteria-derived hemolysin protein controlled by a promoter active under acidic pH conditions, and the bacterium is deficient in at least one gene involved in or regulating an endogenous anti-oxidative stress response pathway or a functional expression product thereof.

The present invention also provides a modified Salmonella typhimurium bacterium, wherein the bacterium comprises, as compared to an unmodified starting strain, one or more expression cassettes for expressing the polynucleotide of the present invention and an essential gene, the expression of which is controlled by a strictly hypoxia inducible promoter, and a pH-regulated expression cassette comprising a gene encoding a bacteria-derived hemolysin protein controlled by a promoter active under acidic pH conditions, wherein the bacterium expresses a wild-type lipopolysaccharide (LPS), and the bacterium is deficient in at least one gene required for survival in a macrophage or a functional expression product thereof.

In a fifth aspect, the present invention provides a pharmaceutical composition comprising the modified bacterium of the present invention. In one embodiment, the pharmaceutical composition is used for treating a malignant tumor.

The present invention also provides a method for treating a malignant tumor, comprising administering the modified bacterium or the pharmaceutical composition of the present invention to a subject suffering from a malignant tumor.

The present invention provides use of the modified bacterium of the present invention in the manufacture of a medicament for the treatment of a malignant tumor.

### Brief Description of Drawings

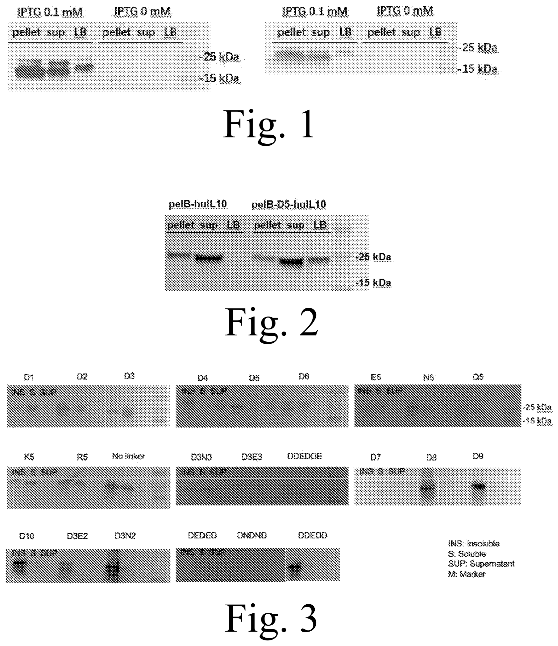

FIG. 1 shows the results of Western Blot showing the effect of the presence and absence of D5 peptide linker on the prokaryotic expression of IL-10. Left: protein expression in the pellet (pellet), lysis supernatant (supernatant) and concentrated LB supernatant (LB) of strain BL21 (DE3)-plasmid-pET22b-pelB-D5-huil10-Amp; Right: protein expression in the pellet (pellet), lysis supernatant (supernatant) and concentrated LB supernatant (LB) of strain BL21 (DE3)-plasmid-pET22b-pelB-huil10-Amp (control without D5 linker peptide) .
FIG. 2 shows the results of Western Blot showing the effect of the presence and absence of D5 peptide linker on the prokaryotic expression of IL-10. Right: protein expression in the pellet (pellet), lysis supernatant (supernatant) and concentrated LB supernatant (LB) of strain BL21 (DE3)-plasmid pSC101-ptac-pelB-D5-huil10-KanR; Left: protein expression in the pellet (pellet), lysis supernatant (supernatant) and concentrated LB supernatant (LB) of strain BL21 (DE3)-plasmid-pSC101-ptac-pelB-huil10-KanR (control without D5 linker peptide).
FIG. 3 shows the results of Western Blot showing the expression of fusion polypeptides containing different peptide linkers by an inducible expression plasmid. For each fusion polypeptide, the insoluble expression product (INS), the soluble product within the bacteria (S), and the secreted product in the supernatant (SUP) were detected separately (in an order from left to right).

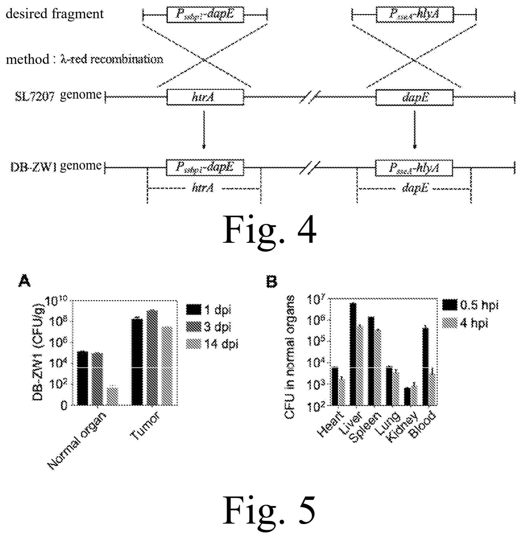

FIG. 4 shows a schematic diagram for the construction of DB-ZW1 strain.
FIG. 5 shows the distribution of DB-ZW1 over time in tumors and tissues.

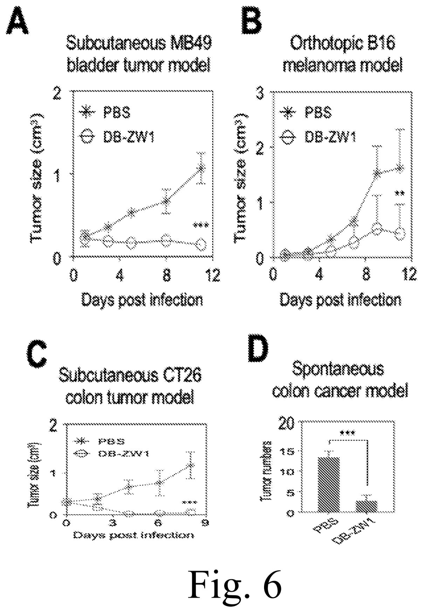

FIG. 6 shows the anti-tumor effects of DB-ZW1 on bladder cancer, melanoma and in situ colon cancer. Among them, FIGs. 6A-C illustrate the effects of DB-ZW1 on the tumor volume of subcutaneous bladder cancer, in situ melanoma, and subcutaneous in situ colon cancer, respectively; and FIG.6D illustrates the effect of DB-ZW1 on the tumor number of in situ colon cancer.

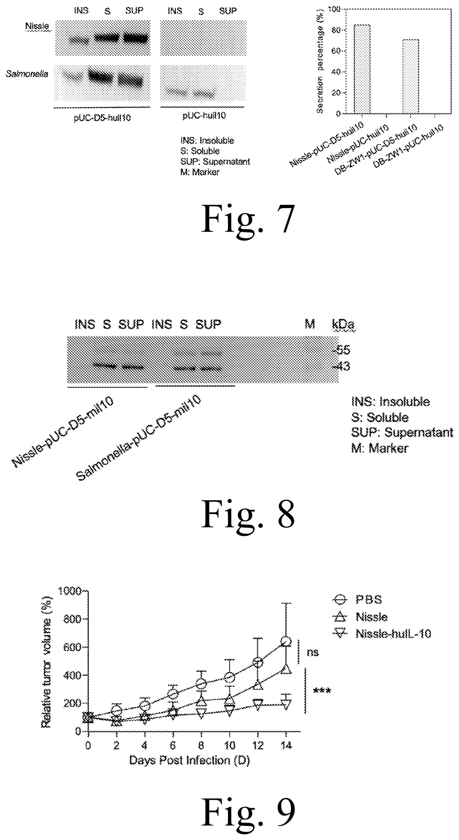

FIG. 7 shows the expression and secretion of human interleukin 10 in E. coli Nissle and Salmonella typhimurium DB-ZW1. The left panel is a Western Blot image of the expression product (INS: insoluble expression product, S: soluble product in the bacteria, and SUP: secreted product in the supernatant), and the right panel is a quantitative analysis of the secreted expression product.
FIG. 8 shows a Western Blot image of the expression and secretion of interleukin 10 by E. coli Nissle and Salmonella typhimurium DB-ZW1 (INS: insoluble expression product, S: soluble product in the bacteria, and SUP: secreted product in the supernatant).
FIG. 9 shows the anti-tumor effect of E. coli Nissle expressing the fusion polypeptide of the present invention.

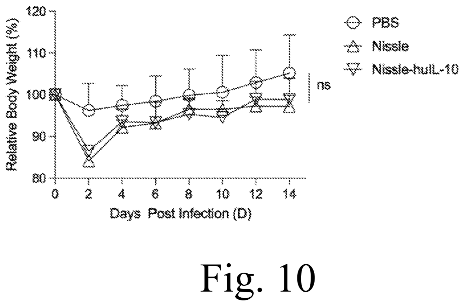

FIG. 10 shows the change in body weight of tumor-bearing mice treated with E. coli Nissle expressing the fusion polypeptide of the present invention.

### Detailed Description of the Invention

Embodiment 1: A fusion polypeptide, comprising a signal peptide, an interleukin 10 (IL-10) polypeptide and a peptide linker linking the signal peptide and a IL-10 polypeptide, wherein the peptide linker comprises 1-10, such as 1, 2, 3, 4, 5, 6, 7, 8, 9 or 10 amino acid residues, and wherein when expressed in a cell, the signal peptide directs the secretion of the IL-10 polypeptide out of the cell.

Embodiment 2: The fusion polypeptide of embodiment 1, wherein the peptide linker comprises
i) 1, 2, 3, 4, 5, 6, 7, 8, 9, or 10 acidic amino acid residues (e.g., D and/or E);
ii) 1, 2, 3, 4, 5, 6, 7, 8, 9, or 10 neutral amino acid residues (e.g., N); and/or
iii) 1, 2, 3, 4, 5, 6, 7, 8, 9, or 10 basic amino acid residues (e.g., K).

Embodiment 3: The fusion polypeptide of embodiment 1 or 2, wherein the peptide linker consists of 1, 2, 3, 4, 5, 6, 7, 8, 9 or 10 glutamic acid residues;
consists of 1, 2, 3, 4, 5, 6, 7, 8, 9, or 10 aspartic acid residues; or
consists of 2, 3, 4, 5, 6, 7, 8, 9 or 10, in total, glutamic acid and aspartic acid residues.

Embodiment 4: The fusion polypeptide of any one of embodiments 1-3, wherein the peptide linker comprises at least one (e.g., 1, 2, 3, 4, or 5) D segment consisting of at least one (e.g., 1, 2, 3, 4, 5, 6, 7, 8, or 9) aspartic acid residue and at least one (e.g., 1, 2, 3, 4, or 5) E segment consisting of at least one (e.g., 1, 2, 3, 4, 5, 6, 7, 8, or 9) glutamic acid residue, and the D segment and the E segment are arranged alternately.

Embodiment 5: The fusion polypeptide of embodiment 4, wherein the peptide linker comprises one D segment and one E segment.

Embodiment 6: The fusion polypeptide of embodiment 5, wherein the peptide linker comprises an amino acid sequence of
D₁E₁,
D₂E₁, D₁E₂,
D₃E₁, D₂E₂, D₁E₃,
D₄E₁, D₃E₂, D₂E₃, D₁E₄,
D₅E₁, D₄E₂, D₃E₃, D₂E₄, D₁E₅,
D₆E₁, D₅E₂, D₄E₃, D₃E₄, D₂E₅, D₁E₆,
D₇E₁, D₆E₂, D₅E₃, D₄E₄, D₃E₅, D₂E₆, D₁E₇,
D₈E₁, D₇E₂, D₆E₃, D₅E₄, D₄E₅, D₃E₆, D₂E₇, D₁E₈,
D₉E₁, D₈E₁, D₇E₂, D₆E₃, D₅E₄, D₄E₅, D₃E₆, D₂E₇ or D₁E₈
E₁D₁,
E₂D₁, E₁D₂,
E₃D₁, E₂D₂, E₁D₃,
E₄D₁, E₃D₂, E₂D₃, E₁D₄,
E₅D₁, E₄D₂, E₃D₃, E₂D₄, E₁D₅,
E₆D₁, E₅D₂, E₄D₃, E₃D₄, E₂D₅, E₁D₆,
E₇D₁, E₆D₂, E₅D₃, E₄D₄, E₃D₅, E₂D₆, E₁D₇,
E₈D₁, E₇D₂, E₆D₃, E₅D₄, E₄D₅, E₃D₆, E₂D₇, E₁D₈,
E₉D₁, E₈D₁, E₇D₂, E₆D₃, E₅D₄, E₄D₅, E₃D₆, E₂D₇ or E₁D₈.

Embodiment 7: The fusion polypeptide of embodiment 1 or 2, wherein the peptide linker comprises 2, 4, 5, 6, 7, 8, 9, or 10, in total, acidic and neutral amino acid residues.

Embodiment 8: The fusion polypeptide of embodiment 1, 2 or 7, wherein the peptide linker consists of 2, 3, 4, 5, 6, 7, 8, 9 or 10 amino acid residues.

Embodiment 9: The fusion polypeptide of embodiment 1, 2, 7, or 8, wherein the peptide linker comprises at least one (e.g., 1, 2, 3, 4, or 5) X segment consisting of at least one (e.g., 1, 3, 4, 5, 6, 7, 8, or 9) acidic amino acid residue and at least one (e.g., 1, 2, 3, 4, or 5) Z segment consisting of at least one (e.g., 1, 2, 4, 5, 6, 7, 8, or 9) neutral amino acid residue, the X segment and the Z segment are arranged alternately.

Embodiment 10: The fusion polypeptide of any one of Embodiment 7-9, wherein the acidic amino acid residue is D, and the neutral amino acid residue is N.

Embodiment 11: The fusion polypeptide of embodiment 10, wherein the peptide linker comprises one X segment and one Z segment.

Embodiment 12: The fusion polypeptide of embodiment 11, wherein the peptide linker comprises an amino acid sequence of
D₁N₁,
D₂N₁, D₁N₂,
D₃N₁, D₂N₂, D₁N₃,
D₄N₁, D₃N₂, D₂N₃, D₁N₄,
D₅N₁, D₄N₂, D₃N₃, D₂N₄, D₁N₅,
D₆N₁, D₅N₂, D₄N₃, D₃N₄, D₂N₅, D₁N₆,
D₇N₁, D₆N₂, D₅N₃, D₄N₄, D₃N₅, D₂N₆, D₁N₇,
D₈N₁, D₇N₂, D₆N₃, D₅N₄, D₄N₅, D₃N₆, D₂N₇, D₁N₈,
D₉N₁, D₈N₁, D₇N₂, D₆N₃, D₅N₄, D₄N₅, D₃N₆, D₂N₇ or D₁N₈
N₁D₁,
N₂D₁, N₁D₂,
N₃D₁, N₂D₂, N₁D₃,
N₄D₁, N₃D₂, N₂D₃, N₁D₄,
N₅D₁, N₄D₂, N₃D₃, N₂D₄, N₁D₅,
N₆D₁, N₅D₂, N₄D₃, N₃D₄, N₂D₅, N₁D₆,
N₇D₁, N₆D₂, N₅D₃, N₄D₄, N₃D₅, N₂D₆, N₁D₇,
N₈D₁, N₇D₂, N₆D₃, N₅D₄, N₄D₅, N₃D₆, N₂D₇, N₁D₈,
N₉D₁, N₈D₁, N₇D₂, N₆D₃, N₅D₄, N₄D₅, N₃D₆, N₂D₇ or N₁D₈.

Embodiment 13: The fusion polypeptide of any one of embodiments 1-12, wherein the IL-10 polypeptide is a human IL-10 polypeptide or a variant thereof.

Embodiment 14: The fusion polypeptide of any one of embodiments 1-13, wherein the IL-10 polypeptide comprises an amino acid sequence of SEQ ID NO: 12, 13, 14, or 15, or an amino acid sequence having at least 60%, 70%, 80%, 90%, 95%, 96%, 97%, 98%, 99% or more identity to SEQ ID NO: 12, 13, 14, or 15, or an amino acid sequence comprising the substitution, insertion, deletion, and/or addition of 1-20, 1-15, 1-10, or 1-5, such as 1, 3, 4, 5, 6, 7, 9, 11, 12, 13, 14, 15, 16, 17, 18, 19, 20, or more amino acid residues, preferably conservative substitutions, of as compared to SEQ ID NO: 12, 13, 14, or 15.

Embodiment 15: The fusion polypeptide of any one of embodiments 1-14, wherein the signal peptide is selected from the group consisting of i) a signal peptide derived from T1SS secretion system, such as HlyA; ii) a signal peptide derived from T2SS secretion system, such as pelB, ompA, ompF, ompC, ompT, PhoA, PhoE, and LPP; and iii) a SicP-S ptP-dependent signal peptide derived from T3SS secretion system, for example, the signal peptide is a pelB signal peptide, in particular, the signal peptide comprises an amino acid sequence of SEQ ID NO: 16, an amino acid sequence having at least 60%, 65%, 70%, 75%, 80%, 85%, 90%, or 95% identity to SEQ ID NO: 16, or an amino acid sequence comprising the substitution, insertion, deletion, and/or addition, preferably conservative substitution, of 1-10 or 1-5, such as 1, 2, 3, 4, 5, 8, 9, or 10 amino acid residues, as compared to SEQ ID NO: 16.

Embodiment 16: The fusion polypeptide of any one of embodiments 1-15, comprising an amino acid sequence of SEQ ID NO: 17, 18, 19, 20, 21, 22, 23 or 24, or an amino acid sequence having at least 60%, 70%, 80%, 90%, 95%, 96%, 97%, 98%, 99% or more identity to SEQ ID NO: 17, 18, 19, 20, 21, 22, 23 or 24, or an amino acid sequence comprising the substitution, insertion, deletion and/or addition, preferably conservative substitution, of 1-20, 1-15, 1-10 or 1-5, such as 1, 3, 4, 5, 6, 7, 9, 11, 12, 13, 14, 15, 16, 17, 18, 19, 20, or more amino acid residues as compared to SEQ ID NO: 17, 18, 19 or 20.

Embodiment 17: A polynucleotide encoding the fusion polypeptide of any one of Embodiment 1-16.

Embodiment 18: An expression cassette comprising the polynucleotide of Embodiment 17 operably linked to a promoter.

Embodiment 19: An expression vector comprising the polynucleotide of embodiment 17 or the expression cassette of embodiment 18.

Embodiment 20: A host cell comprising the polynucleotide of Embodiment 17 or the expression vector of Embodiment 18, for example Escherichia coli such as the Nissle strain and the BL21 (DE3) strain, Salmonella typhimurium such as the SL7207 strain, and Bacillus subtilis.

Embodiment 21: A method for the prokaryotic expression of a IL-10 polypeptide, comprising:
i) transforming a bacterial cell with the expression vector of embodiment 19; and
ii) culturing the transformed cell under a condition that enables the expression of the vector and preferably the secretion of IL-10.

Embodiment 22: The method of embodiment 21, wherein the bacterial cell is a bacterial cell selected from the group consisting of E. coli such as the Nissle strain and the BL21 (DE3) strain, Salmonella typhimurium such as the SL7207 strain, and Bacillus subtilis.

### Embodiment A

Embodiment A1: A modified bacterium, wherein the bacterium comprises, as compared to the unmodified starting strain,
i) the expression cassette of embodiment 18 and a hypoxia regulated essential gene expression cassette, said essential gene expression cassette comprising an essential gene of said bacterium controlled by a strictly hypoxia inducible promoter; or
ii) an expression cassette comprising the polynucleotide of Embodiment 17 and/or an essential gene controlled by a strictly hypoxia inducible promoter,
wherein the bacterium is deficient in at least one gene involved in or regulating an endogenous anti-oxidative stress response pathway or the functional expression product thereof.

Embodiment A2: The modified bacterium of embodiment A1, wherein the strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment A3: The modified bacterium of embodiment A1, wherein the strictly hypoxia inducible promoter is ssbp1 promoter.

Embodiment A4: The modified bacterium of any one of the preceding embodiments A, wherein the expression product of the essential gene is responsible for the synthesis of 2,6-diaminopimelic acid (DAP) in the bacterium, and when cultured under aerobic conditions, the growth of the bacterium is dependent on the additional DAP or an analog thereof added to the medium.

Embodiment A5: The modified bacterium of embodiment A4, wherein the essential gene is selected from dapA, dapB, dapD, dapE, argD, dapF, and any combination thereof.

Embodiment A6: The modified bacterium of embodiment A4, wherein the essential gene is selected from dapA and dapE.

Embodiment A7: The modified bacterium of any one of the preceding embodiments A, wherein the gene involved in or regulating the endogenous anti-oxidative stress response pathway is a gene of HtrA serine protease family.

Embodiment A8: The modified bacterium of any one of the preceding embodiments A, wherein the functional expression product of the gene involved in or regulating the endogenous anti-oxidative stress response pathway is an HtrA serine protease family related protein.

Embodiment A9: The modified bacterium of any one of the preceding embodiments A, wherein the bacterium is deficient in the activity of HtrA serine protease.

Embodiment A10: The modified bacterium of any one of the preceding embodiments A, wherein the gene involved in or regulating the endogenous anti-oxidative stress response pathway is htrA.

Embodiment A11: The modified bacterium of any one of the preceding embodiments A, wherein the bacterium is deficient in htrA.

Embodiment A12: The modified bacterium of any one of embodiments A1-A11, wherein the essential gene is a gene naturally occurring in the bacterial chromosome, and the natural promoter of the essential gene is functionally replaced by the strictly hypoxia inducible promoter, whereby the expression of the essential gene in the bacterium is completely under the control of the strictly hypoxia inducible promoter.

Embodiment A13: The modified bacterium of any one of embodiments A1-A11, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment A14: The modified bacterium of embodiment A13, wherein the exogenous essential gene expression cassette is integrated into the chromosome of the bacterium, or the expression cassette of embodiment 18 or the expression cassette comprising the polynucleotide of embodiment 17 controlled by a strictly hypoxia inducible promoter is integrated into the chromosome of the bacterium.

Embodiment A15: The modified bacterium of embodiment A13, wherein the exogenous essential gene expression cassette is located outside the chromosome of the bacterium, or the expression cassette of embodiment 18 or an expression cassette comprising a polynucleotide of embodiment 17 controlled by a strictly hypoxia inducible promoter is located outside the chromosome of the bacterium.

Embodiment A16: The modified bacterium of embodiment A15, wherein the exogenous essential gene expression cassette is present in a plasmid carried by the bacterium, or the expression cassette of embodiment 18 or an expression cassette comprising a polynucleotide of embodiment 17 controlled by a strictly hypoxia inducible promoter is present in a plasmid carried by the bacterium.

Embodiment A17: The modified bacterium of any one of the preceding embodiments A, which further comprises a pH regulated expression cassette, wherein the pH regulated expression cassette comprises a gene encoding a bacteria-derived hemolysin protein under the control of a promoter that is active under acid pH condition.

Embodiment A18: The modified bacterium of embodiments A17, wherein the bacteria-derived hemolysin protein is a gram-negative bacterial hemolysin protein.

Embodiment A19: The modified bacterium of embodiments A18, wherein the gene encoding the gram-negative bacterial hemolysin protein is hlyA or hlyE.

Embodiment A20: The modified bacterium of embodiments A19, wherein the hlyA or hlyE is derived from Listeria monocytogenes, Vibrio cholerae, or Escherichia coli.

Embodiment A21: The modified bacterium of any one of embodiments A17-A20, wherein the promoter that is active under acid pH condition is active at a pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment A22: The modified bacterium of any one of embodiments A17-A21, wherein the promoter that is active under acid pH condition is selected from the group consisting of sseA, ssrA, ssaB, ssaG, ssaM, and ssaR.

Embodiment A23: The modified bacterium of any one of embodiments A17-A21, wherein the promoter that is active under acid pH condition is sseA.

Embodiment A24: The modified bacterium of any one of the preceding embodiments A, wherein the unmodified starting strain is a facultative anaerobic bacterium.

Embodiment A25: The modified bacterium of any one of the preceding embodiments A, wherein the bacterium is a bacterium of Enterobacteriaceae.

Embodiment A26: The modified bacterium of any one of the preceding embodiments A, wherein the bacterium is a bacteirum of *Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp., or Pantoea sp.*

Embodiment A27: The modified bacterium of any one of the previous Embodiments A, wherein the bacterium is selected from the group consisting of E. coli such as the Nissle strain and the BL21 (DE3) strain, *Enterobacter blattae* (*E. blattae*), *Enterobacter fergusonii* (*E. fergusonii*)*, Enterobacter hermannii* (*E. hermannii*), *Enterobacter vulneris* (*E. vulneris*), *Salmonella enterica* (*S. enterica*), *Salmonella bongori* (*S. bongori*), *Salmonella typhi* (*S. typhi*), *Salmonella choleraesuis* (*S. choleraesuis*), *Salmonella typhimurium* (*S. typhimurium*), *Shigella dysenteriae* (*S. dysenteriae*), *Shigella flexneri* (*S. flexneri*), *Shigella boydii* (*S. boydii*), *Shigella sonnei* (*S. sonnei*), *Klebsiella pneumoniae* (*K. peumoniae*), *Klebsiella oxytoca* (*K. oxytoca*), *Yersinia pestis* (*Y. pestis*), *Yersinia enterocolitica* (*Y. enterocolitica*), *Yersinia pseudotuberculosis* (*Y. pseudotuberculosis*), *Yersinia aldouae* (*Y. aldouae*), *Yersinia bercovieri* (*Y. bercovieri*), *Yersinia frederiksenii* (*Y. frederiksenii*)*, Yersinia intermedia* (*Y. intermedia*), *Yersinia kristersenii* (*Y. kristersenii*), *Yersinia mollaretti* (*Y. mollaretti*), *Yersinia rohdei* (*Y. rohdei), Yersinia ruckeri* (*Y. ruckeri*), *Citrobacter freundii* (*C. freundii*)*, Citrobacter koseri* (*C. kaseri*), *Citrobacter braakii* (*C. braakii*), *Enterobacter aerogenes* (*E. aerogenes*), *Enterobacter cloacae* (*E. cloacae*), *Enterobacter gergoviae (E. gergoviac), Enterobacter sakazakii* (*E. sakazakii*), *Enterobacter taylorae* (*E. tavlorae*), *Enterobacter amnigenus* (*E. aminigenus*), *Enterobacter intermedius* (*E. intermedius*), *Enterobacter asburiae* (*E. asburiac*), *Enterobacter cancerogenus* (*E. cancerogenus), Enterobacter dissolvens* (*E. dissolvens), Enterobacter nimipressuralis* (*E. nimipressuralis*), *Serratia marcescens* (*S. marcescens), Serratia entomophila* (*S. entomophila*), *Serratia ficaria* (*S. ficaria*), *Serratia fonticola* (*S*. *fonticola*)*, Serratia grimesii* (*S. grimesii), Serratia liquefaciens* (*S. liquefaciens*)*, Serratia odorifera* (*S. odorifera*), *Serratia plymuthica* (*S. plymuthica*), *Serratia proteamaculans* (*S. proteamaculans), Serratia rubidaea* (*S. rubidaea*), *Serratia ureilytica* (*S. ureilytica*), *Proteus mirabilis* (*P. mirabilis), Proteus vulgaris* (*P. vulgaris*), *Proteus myxofaciens* (*P. myxofaciens*)*, Proteus penneri* (*P. penneri), Proteus hauseri* (*P. hauseri*)), *Morganella morganii* (*M. morganii*), *Providencia alcalifaciens* (*P. alcalifaciens*), *Providenciarustigianii* (*P. rustigianii*), *Providencia stuartii* (*P. stuartii*), *Providencia rettgeri* (*P. rettgeri*), *Providencia heimbachae* (*P. heimbochae*), *Hafnia alvei* (*H. alvei*),and *Pantoea agglomerans* (*P. agglomerans*).

Embodiment A28: The modified bacterium of any one of the preceding embodiments A, wherein the bacterium is *Salmonella typhimurium.*

Embodiment A29: The modified bacterium of embodiment A28, wherein the starting strain is *Salmonella typhimurium* SL7207.

Embodiment A30: The modified bacterium of any one of the preceding embodiments A, wherein the bacterium expresses a wild-type lipopolysaccharide (LPS).

Embodiment A31: The modified bacterium of any one of the preceding embodiments A, wherein, when administrated to a subject with a tumor, the bacterium is capable of surviving and proliferating in tumor tissue and is rapidly cleared in normal tissue.

Embodiment A32: The modified bacterium of any one of the preceding embodiments A, wherein, when administrated to a subject with a malignant tumor, the bacterium is capable of inhibiting the growth of the malignant tumor.

Embodiment A33: The modified bacterium of any one of the preceding embodiments A, wherein, when administrated to a subject with a malignant tumor, the bacterium is capable of inducing an anti-tumor specific immune response.

Embodiment A34: The modified bacterium of any one of the preceding embodiments A, wherein, when administrated to a subject with a malignant tumor, the bacterium is capable of inducing anti-tumor immune memory.

Embodiment A35: The modified bacterium of any one of the preceding embodiments A, which does not express a wild-type flagellin.

Embodiment A36: The modified bacterium of any one of the preceding embodiments A, which is deficient in the fliC gene.

Embodiment A37: The modified bacterium of any one of the preceding embodiments A, wherein the survival level of the bacterium in macrophages ranges from about 50% to about 30% of the survival level of the unmodified starting strain.

Embodiment A38: The modified bacterium of any one of embodiments A1-A36, wherein the survival level of the bacterium in macrophages ranges from about 30% to about 10% of the survival level of the unmodified starting strain.

Embodiment A39: The modified bacterium of any one of embodiments A1-A36, wherein the survival level of the bacterium in macrophages ranges from about 10% to about 1% of the survival level of the unmodified starting strain.

### Embodiment B

Embodiment B1: A modified bacterium, wherein the bacterium comprises, as compared to the unmodified starting strain,
i) the expression cassette of embodiment 18, a hypoxia regulated essential gene expression cassette and a pH regulated expression cassette, said essential gene expression cassette comprises an essential gene of said bacterium controlled by a strictly hypoxia inducible promoter, or
ii) an expression cassette comprising the polynucleotide of Embodiment 17 and/or an essential gene controlled by a strictly hypoxia inducible promoter and a pH regulated expression cassettes,
wherein the pH regulated expression cassette comprises a gene encoding a bacteria-derived hemolysin protein controlled by a promoter active under acidic pH conditions, and the bacterium is deficient in at least one gene involved in or regulating an endogenous anti-oxidative stress response pathway or a functional expression product thereof.

Embodiment B2: The modified bacterium of embodiment B1, wherein the strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment B3: The modified bacterium of embodiment B1, wherein the strictly hypoxia inducible promoter is ssbp1 promoter.

Embodiment B4: The modified bacterium of any one of the preceding embodiments B, wherein the expression product of the essential gene is responsible for synthesis of 2,6-diaminopimelic acid (DAP) within the bacterium, and when cultured under aerobic conditions, the growth of the bacterium is dependent on the additional DAP or an analog thereof added to the medium.

Embodiment B5: The modified bacterium of embodiment B4, wherein the essential gene is selected from dapA, dapB, dapD, dapE, argD, dapF, and any combination thereof.

Embodiment B6: The modified bacterium of embodiment B4, wherein the essential gene is selected from dapA and dapE.

Embodiment B7: The modified bacterium of any one of the preceding embodiments B, wherein the gene involved in or regulating the endogenous anti-oxidative stress response pathway is a gene of HtrA serine protease family.

Embodiment B8: The modified bacterium of any one of the preceding embodiments B, wherein the functional expression product of the gene involved in or regulating the endogenous anti-oxidative stress response pathway is an HtrA serine protease family related protein.

Embodiment B9: The modified bacterium of any one of the preceding embodiments B, wherein the bacterium is deficient in the activity of HtrA serine protease.

Embodiment B10: The modified bacterium of any one of the preceding embodiments B, wherein the gene involved in or regulating the endogenous anti-oxidative stress response pathway is htrA.

Embodiment B11: The modified bacterium of any one of the preceding embodiments B, wherein the bacterium is deficient in htrA.

Embodiment B12: The modified bacterium of any one of embodiments B1-B11, wherein the essential gene is a gene naturally occurring in the bacterial chromosome, and the natural promoter of the essential gene is functionally replaced by the strictly hypoxia inducible promoter, whereby the expression of the essential gene in the bacterium is completely under the control of the strictly hypoxia inducible promoter.

Embodiment B13: The modified bacterium of any one of embodiments B1-B11, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment B14: The modified bacterium of embodiment B13, wherein the exogenous essential gene expression cassette is integrated into the chromosome of the bacterium, or the expression cassette of embodiment 18 or the expression cassette comprising the polynucleotide of embodiment 17 controlled by a strictly hypoxia inducible promoter is integrated into the chromosome of the bacterium.

Embodiment B15: The modified bacterium of embodiment B13, wherein the exogenous essential gene expression cassette is located outside the chromosome of the bacterium, or the expression cassette of embodiment 18 or an expression cassette comprising a polynucleotide of embodiment 17 controlled by a strictly hypoxia inducible promoter is located outside the chromosome of the bacterium.

Embodiment B16: The modified bacterium of embodiment B15, wherein the exogenous essential gene expression cassette is present in a plasmid carried by the bacterium, or the expression cassette of embodiment 18 or an expression cassette comprising the polynucleotide of embodiment 17 controlled by a strictly hypoxia inducible promoter is present in a plasmid carried by the bacterium.

Embodiment B17: The modified bacterium of embodiments any one of the preceding embodiments B, wherein the bacteria-derived hemolysin protein is a gram-negative bacterial hemolysin protein.

Embodiment B18: The modified bacterium of embodiments B17, wherein the gene encoding the gram-negative bacterial hemolysin protein is hlyA or hlyE.

Embodiment B19: The modified bacterium of embodiments B18, wherein the hlyA or hlyE is derived from Listeria monocytogenes, Vibrio cholerae, or Escherichia coli.

Embodiment B20: The modified bacterium of any one of the preceding embodiments B, wherein the promoter that is active under acid pH condition is active at a pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment B21: The modified bacterium of any one of the preceding embodiments B, wherein the promoter that is active under acid pH condition is selected from the group consisting of sseA, ssrA, ssaB, ssaG, ssaM, and ssaR.

Embodiment B22: The modified bacterium of any one of the preceding embodiments B, wherein the promoter that is active under acid pH condition is sseA.

Embodiment B23: The modified bacterium of any one of the preceding embodiments B, wherein the unmodified starting strain is a facultative anaerobic bacterium.

Embodiment B24: The modified bacterium of any one of the preceding embodiments B, wherein the bacterium is a bacterium of Enterobacteriaceae.

Embodiment B25: The modified bacterium of any one of embodiments B, wherein the bacterium is a bacteirum of *Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp., and Pantoea sp.*

Embodiment B26: The modified bacterium of any one of the preceding embodiments B, wherein the bacterium is selected from the group consisting of E. coli such as the Nissle strain and the BL21 (DE3) strain, *Enterobacter blattae (E. blattae), Enterobacter fergusonii (E. fergusonii*)*, Enterobacter hermannii (E. hermannii), Enterobacter vulneris (E. vulneris), Salmonella enterica (S. enterica), Salmonella bongori (S. bongori), Salmonella typhi (S. typhi), Salmonella choleraesuis (S. choleraesuis), Salmonella typhimurium (S. typhimurium), Shigella dysenteriae (S. dysenteriae), Shigella flexneri (S. flexneri), Shigella boydii (S. boydii), Shigella sonnei (S. sonnei), Klebsiella pneumoniae (K. peumoniae), Klebsiella oxytoca (K. oxytoca), Yersinia pestis (Y. pestis), Yersinia enterocolitica (Y. enterocolitica), Yersinia pseudotuberculosis (Y. pseudotuberculosis), Yersinia aldouae (Y. aldouae), Yersinia bercovieri* (*Y. bercovieri*), *Yersinia frederiksenii (Y*. *frederiksenii*)*, Yersinia intermedia* (*Y. intermedia*), *Yersinia kristersenii* (*Y. kristersenii*), *Yersinia mollaretti* (*Y. mollaretti*), *Yersinia rohdei* (*Y. rohdei*), *Yersinia ruckeri* (*Y. ruckeri*), *Citrobacter freundii* (*C. freundii*)*, Citrobacter koseri* (*C. kaseri*), *Citrobacter braakii* (*C. braakii*), *Enterobacter aerogenes* (*E. aerogenes*), *Enterobacter cloacae* (*E. cloacae*), *Enterobacter gergoviae* (*E. gergoviac*), *Enterobacter sakazakii* (*E. sakazakii*), *Enterobacter taylorae* (*E. tavlorae*), *Enterobacter amnigenus* (*E. aminigenus*), *Enterobacter intermedius* (*E. intermedius*), *Enterobacter asburiae* (*E. asburiac*), *Enterobacter cancerogenus* (*E. cancerogenus*), *Enterobacter dissolvens* (*E. dissolvens*), *Enterobacter nimipressuralis* (*E. nimipressuralis*), *Serratia marcescens* (*S. marcescens*), *Serratia entomophila* (*S. entomophila*), *Serratia ficaria* (*S. ficaria*), *Serratia fonticola* (*S. fonticola*)*, Serratia grimesii* (*S. grimesii*), *Serratia liquefaciens* (*S. liquefaciens*)*, Serratia odorifera* (*S. odorifera*), *Serratia plymuthica* (*S. plymuthica*), *Serratia proteamaculans* (*S. proteamaculans*), *Serratia rubidaea* (*S. rubidaea*), *Serratia ureilytica* (*S. ureilytica*), *Proteus mirabilis* (*P. mirabilis*), *Proteus vulgaris* (*P. vulgaris*), *Proteus myxofaciens* (*P. myxofaciens*)*, Proteus penneri* (*P. penneri*), *Proteus hauseri* (*P. hauseri*)), *Morganella morganii (M. morganii*), *Providencia alcalifaciens* (*P. alcalifaciens*), *Providenciarustigianii* (*P. rustigianii*), *Providencia stuartii* (*P. stuartii*), *Providencia rettgeri* (*P. rettgeri*), *Providencia heimbachae* (*P. heimbochae*), *Hafnia alvei* (*H. alvei*),and *Pantoea agglomerans* (*P. agglomerans*).

Embodiment B27: The modified bacterium of any one of the preceding embodiments B, wherein the bacterium is *Salmonella typhimurium.*

Embodiment B28: The modified bacterium of embodiment B27, wherein the starting strain is *Salmonella typhimurium* SL7207.

Embodiment B29: The modified bacterium of any one of the preceding embodiments B, wherein the bacterium expresses a wild-type lipopolysaccharide (LPS).

Embodiment B30: The modified bacterium of any one of the preceding embodiments B, wherein, when administrated to a subject with a tumor, the bacterium is capable of surviving and proliferating in tumor tissue and is rapidly cleared in normal tissue.

Embodiment B31: The modified bacterium of any one of the preceding embodiments B, wherein, when administrated to a subject with a malignant tumor, the bacterium is capable of inhibiting the growth of the malignant tumor.

Embodiment B32: The modified bacterium of any one of the preceding embodiments B, wherein, when administrated to a subject with a malignant tumor, the bacterium is capable of inducing an anti-tumor specific immune response.

Embodiment B33: The modified bacterium of any one of the preceding embodiments B, wherein, when administrated to a subject with a malignant tumor, the bacterium is capable of inducing anti-tumor immune memory.

Embodiment B34: The modified bacterium of any one of the preceding embodiments B, which does not express a wild-type flagellin.

Embodiment B35: The modified bacterium of any one of the preceding embodiments B, which is deficient in the fliC gene.

Embodiment B36: The modified bacterium of any one of the preceding embodiments B, wherein the survival level of the bacterium in macrophages ranges from about 50% to about 30% of the survival level of the unmodified starting strain.

Embodiment B37: The modified bacterium of any one of embodiments B1-B35, wherein the survival level of the bacterium in macrophages ranges from about 30% to about 10% of the survival level of the unmodified starting strain.

Embodiment B38: The modified bacterium of any one of embodiments B1-B35, wherein the survival level of the bacterium in macrophages ranges from about 10% to about 1% of the survival level of the unmodified starting strain.

### Embodiment C

Embodiment C1: A modified *Salmonella typhimurium* bacterium, wherein the bacterium comprises, as compared to the unmodified starting strain,
i) the expression cassette of embodiment 18 and a hypoxia regulated essential gene expression cassette, said essential gene expression cassette comprises an essential gene of said bacterium controlled by a strictly hypoxia inducible promoter; or
ii) an expression cassette comprising the polynucleotide of Embodiment 17 and/or an essential gene controlled by a strictly hypoxia inducible promoter,
wherein the bacterium is deficient in at least one gene or functional expression product thereof involved in or regulating an endogenous anti-oxidative stress response pathway.

Embodiment C2: The modified bacterium of embodiment C1, wherein the strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment C3: The modified bacterium of embodiment C1, wherein the strictly hypoxia inducible promoter is ssbp1 promoter.

Embodiment C4: The modified bacterium of any one of the preceding embodiments C, wherein the expression product of the essential gene is responsible for synthesis of 2,6-diaminopimelic acid (DAP) within the bacterium, and when cultured under aerobic conditions, the growth of the bacterium is dependent on the additional DAP or an analog thereof added to the medium.

Embodiment C5: The modified bacterium of embodiment C4, wherein the essential gene is selected from dapA, dapB, dapD, dapE, argD, dapF, and any combination thereof.

Embodiment C6: The modified bacterium of embodiment C4, wherein the essential gene is selected from dapA and dapE.

Embodiment C7: The modified bacterium of any one of the preceding embodiments C, wherein the gene involved in or regulating the endogenous anti-oxidative stress response pathway is a gene of HtrA serine protease family.

Embodiment C8: The modified bacterium of any one of the preceding embodiments C, wherein the functional expression product of the gene involved in or regulating the endogenous anti-oxidative stress response pathway is an HtrA serine protease family related protein.

Embodiment C9: The modified bacterium of any one of the preceding embodiments C, wherein the bacterium is deficient in the activity of HtrA serine protease.

Embodiment C10: The modified bacterium of any one of the preceding embodiments C, wherein the gene involved in or regulating the endogenous anti-oxidative stress response pathway is htrA.

Embodiment C11: The modified bacterium of any one of the preceding embodiments C, wherein the bacterium is deficient in htrA.

Embodiment C12: The modified bacterium of any one of embodiments C1-C11, wherein the essential gene is a gene naturally occurring in the bacterial chromosome, and the natural promoter of the essential gene is functionally replaced by the strictly hypoxia inducible promoter, whereby the expression of the essential gene in the bacterium is completely under the control of the strictly hypoxia inducible promoter.

Embodiment C13: The modified bacterium of any one of embodiments C1-C11, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment C14: The modified bacterium of embodiment C13, wherein the exogenous essential gene expression cassette is integrated into the chromosome of the bacterium, or the expression cassette of embodiment 18 or an expression cassette comprising a polynucleotide of embodiment 17 controlled by a strictly hypoxia inducible promoter is integrated into the chromosome of the bacterium.

Embodiment C15: The modified bacterium of embodiment C13, wherein the exogenous essential gene expression cassette is located outside the chromosome of the bacterium, or the expression cassette of embodiment 18 or an expression cassette comprising a polynucleotide of embodiment 17 controlled by a strictly hypoxia inducible promoter is located outside the chromosome of the bacterium.

Embodiment C16: The modified bacterium of embodiment C15, wherein the exogenous essential gene expression cassette is present in a plasmid carried by the bacterium, or the expression cassette of embodiment 18 or an expression cassette comprising a polynucleotide of embodiment 17 controlled by a strictly hypoxia inducible promoter is located in a plasmid carried by the bacterium.

Embodiment C17: The modified bacterium of embodiments any one of the preceding embodiments C, wherein the bacteria-derived hemolysin protein is a gram-negative bacterial hemolysin protein.

Embodiment C18: The modified bacterium of embodiment C17, wherein the gene encoding the gram-negative bacterial hemolysin protein is hlyA or hlyE.

Embodiment C19: The modified bacterium of embodiment C18, wherein the hlyA or hlyE is derived from Listeria monocytogenes, Vibrio cholerae, or Escherichia coli.

Embodiment C20: The modified bacterium of any one of the preceding embodiments C, wherein the promoter that is active under acid pH condition is active at a pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment C21: The modified bacterium of any one of the preceding embodiments C, wherein the promoter that is active under acid pH condition is selected from the group consisting of sseA, ssrA, ssaB, ssaG, ssaM, and ssaR.

Embodiment C22: The modified bacterium of any one of the preceding embodiments C, wherein the promoter that is active under acid pH condition is sseA.

Embodiment C23: The modified bacterium of any one of the preceding embodiments C, wherein the unmodified starting strain is a facultative anaerobic bacterium.

Embodiment C24: The modified bacterium of any one of the preceding embodiments C, wherein the starting strain is Salmonella typhimurium SL7207.

Embodiment C25: The modified bacterium of any one of embodiments C1-C24, wherein the bacterium expresses a wild-type lipopolysaccharide (LPS).

Embodiment C26: The modified bacterium of any one of the preceding embodiments C, wherein, when administrated to a subject with a tumor, the bacterium is capable of surviving and proliferating in tumor tissue but is rapidly cleared in normal tissue.

Embodiment C27: The modified bacterium of any one of the preceding embodiments C, wherein, when administrated to a subject with a malignant tumor, the bacterium is capable of inhibiting the growth of the malignant tumor.

Embodiment C28: The modified bacterium of any one of the preceding embodiments C, wherein, when administrated to a subject with a malignant tumor, the bacterium is capable of inducing an anti-tumor specific immune response.

Embodiment C29: The modified bacterium of any one of the preceding embodiments C, wherein, when administrated to a subject with a malignant tumor, the bacterium is capable of inducing anti-tumor immune memory.

Embodiment C30: The modified bacterium of any one of the preceding embodiments C, which does not express a wild-type flagellin.

Embodiment C31: The modified bacterium of any one of the preceding embodiments C, which is deficient in the fliC gene.

Embodiment C32: The modified bacterium of any one of the preceding embodiments C, wherein the survival level of the bacterium in macrophages ranges from about 50% to about 30% of the survival level of the unmodified starting strain.

Embodiment C33: The modified bacterium of any one of embodiments C1-C31, wherein the survival level of the bacterium in macrophages ranges from about 30% to about 10% of the survival level of the unmodified starting strain.

Embodiment C34: The modified bacterium of any one of embodiments C1-C31, wherein the survival level of the bacterium in macrophages ranges from about 10% to about 1% of the survival level of the unmodified starting strain.

### Embodiment D

Embodiment D1: A modified bacterium, wherein the bacterium comprises, as compared to the unmodified starting strain,
i) the expression cassette of embodiment 18 and a hypoxia regulated essential gene expression cassette, said essential gene expression cassette comprises an essential gene of said bacterium controlled by a strictly hypoxia inducible promoter; or
ii) an expression cassette comprising the polynucleotide of Embodiment 17 and/or an essential gene controlled by a strictly hypoxia inducible promoter,
wherein the bacterium is deficient in at least one gene required for survival in macrophages or the functional expression product thereof.

Embodiment D2: The modified bacterium of embodiment D1, wherein the strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment D3: The modified bacterium of embodiment D1, wherein the strictly hypoxia inducible promoter is ssbp1 promoter.

Embodiment D4: The modified bacterium of any one of the preceding embodiments D, wherein the expression product of the essential gene is responsible for synthesis of 2,6-diaminopimelic acid (DAP) within the bacterium, and when cultured under aerobic conditions, the growth of the bacterium is dependent on the additional DAP or an analog thereof added to the medium.

Embodiment D5: The modified bacterium of embodiment D4, wherein the essential gene is selected from dapA, dapB, dapD, dapE, argD, dapF, and any combination thereof.

Embodiment D6: The modified bacterium of embodiment D4, wherein the essential gene is selected from dapA and dapE.

Embodiment D7: The modified bacterium of any one of the preceding embodiments D, wherein the gene required for survival in macrophages is STM3120, STM3119, slyA, sifA, SPI-2, phoP, or htrA gene.

Embodiment D8: The modified bacterium of any one of the preceding embodiments D, wherein the functional expression product of the gene required for survival in macrophages is an HtrA serine protease family related protein.

Embodiment D9: The modified bacterium of any one of the preceding embodiments D, wherein the bacterium is deficient in the activity of HtrA serine protease.

Embodiment D10: The modified bacterium of any one of the preceding embodiments D, wherein the gene required for survival in macrophages is htrA.

Embodiment D11: The modified bacterium of any one of the preceding embodiments D, wherein the bacterium is deficient in htrA.

Embodiment D12: The modified bacterium of any one of embodiments D1-D11, wherein the essential gene is a gene naturally occurring in the bacterial chromosome, and the natural promoter of the essential gene is functionally replaced by the strictly hypoxia inducible promoter, whereby the expression of the essential gene in the bacterium is completely under the control of the strictly hypoxia inducible promoter.

Embodiment D13: The modified bacterium of any one of embodiments D1-D11, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment D14: The modified bacterium of embodiment D13, wherein the exogenous essential gene expression cassette is integrated into the chromosome of the bacterium, or the expression cassette of embodiment 18 or the expression cassette comprising the polynucleotide of embodiment 17 controlled by a strictly hypoxia inducible promoter is integrated into the chromosome of the bacterium.

Embodiment D15: The modified bacterium of embodiment D13, wherein the exogenous essential gene expression cassette is located outside the chromosome of the bacterium, or the expression cassette of embodiment 18 or an expression cassette comprising a polynucleotide of embodiment 17 controlled by a strictly hypoxia inducible promoter is located outside the chromosome of the bacterium.

Embodiment D16: The modified bacterium of Embodiment D15, wherein the exogenous essential gene expression cassette is present in a plasmid carried by the bacterium, or the expression cassette of Embodiment 18 or an expression cassette comprising a polynucleotide of Embodiment 17 controlled by a strictly hypoxia inducible promoter is present in a plasmid carried by the bacterium.

Embodiment D17: The modified bacterium of any one of the preceding embodiments D, which further comprises a pH regulated expression cassette, wherein the pH regulated expression cassette comprises a gene encoding a bacteria-derived hemolysin protein under the control of a promoter that is active under acid pH condition.

Embodiment D18: The modified bacterium of embodiments D17, wherein the bacteria-derived hemolysin protein is a gram-negative bacterial hemolysin protein.

Embodiment D19: The modified bacterium of embodiments D18, wherein the gene encoding the gram-negative bacterial hemolysin protein is hlyA or hlyE.

Embodiment D20: The modified bacterium of embodiments D19, wherein the hlyA or hlyE is derived from Listeria monocytogenes, Vibrio cholerae, or Escherichia coli.

Embodiment D21: The modified bacterium of any one of embodiments D17-D20, wherein the promoter that is active under acid pH condition is active at a pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment D22: The modified bacterium of any one of embodiments D17-D21, wherein the promoter that is active under acid pH condition is selected from the group consisting of sseA, ssrA, ssaB, ssaG, ssaM, and ssaR.

Embodiment D23: The modified bacterium of any one of embodiments D17-D21, wherein the promoter that is active under acid pH condition is sseA.

Embodiment D24: The modified bacterium of any one of the preceding embodiments D, wherein the unmodified starting strain is a facultative anaerobic bacterium.

Embodiment D25: The modified bacterium of any one of the preceding embodiments D, wherein the bacterium is a bacterium of Enterobacteriaceae.

Embodiment D26: The modified bacterium of any one of the preceding embodiments D, wherein the bacterium is a bacteirum of *Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp., and Pantoea sp.*

Embodiment D27: The modified bacterium of any one of the preceding embodiments D, wherein the bacterium is selected from the group consisting of E. coli such as the Nissle strain and the BL21 (DE3) strain, *Enterobacter blattae* (*E. blattae*), *Enterobacter fergusonii* (*E. fergusonii*)*, Enterobacter hermannii* (*E. hermannii*), *Enterobacter vulneris* (*E. vulneris*), *Salmonella enterica* (*S. enterica*), *Salmonella bongori* (*S. bongori*), *Salmonella typhi* (*S. typhi*), *Salmonella choleraesuis* (*S. choleraesuis*), *Salmonella typhimurium* (*S. typhimurium*), *Shigella dysenteriae* (*S. dysenteriae*), *Shigella flexneri* (*S. flexneri*), *Shigella boydii* (*S. boydii*), *Shigella sonnei* (*S. sonnei*), *Klebsiella pneumoniae* (*K. peumoniae*), *Klebsiella oxytoca* (*K. oxytoca*), *Yersinia pestis* (*Y. pestis*), *Yersinia enterocolitica* (*Y. enterocolitica*), *Yersinia pseudotuberculosis* (*Y. pseudotuberculosis*), *Yersinia aldouae* (*Y. aldouae*), *Yersinia bercovieri* (*Y. bercovieri*), *Yersinia frederiksenii* (*Y. frederiksenii*)*, Yersinia intermedia* (*Y. intermedia*), *Yersinia kristersenii* (*Y. kristersenii*), *Yersinia mollaretti* (*Y. mollaretti*), *Yersinia rohdei* (*Y. rohdei*), *Yersinia ruckeri* (*Y. ruckeri*), *Citrobacter freundii* (*C. freundii*)*, Citrobacter koseri* (*C. kaseri*), *Citrobacter braakii* (*C. braakii*), *Enterobacter aerogenes* (*E. aerogenes*), *Enterobacter cloacae* (*E. cloacae*), *Enterobacter gergoviae* (*E. gergoviac*), *Enterobacter sakazakii* (*E. sakazakii*), *Enterobacter taylorae* (*E. tavlorae*), *Enterobacter amnigenus* (*E. aminigenus*), *Enterobacter intermedius* (*E. intermedius*), *Enterobacter asburiae* (*E. asburiac*), *Enterobacter cancerogenus* (*E. cancerogenus*), *Enterobacter dissolvens* (*E. dissolvens*), *Enterobacter nimipressuralis* (*E. nimipressuralis*), *Serratia marcescens* (*S. marcescens*), *Serratia entomophila* (*S. entomophila*), *Serratia ficaria* (*S. ficaria*), *Serratia fonticola* (*S. fonticola*)*, Serratia grimesii* (*S. grimesii*), *Serratia liquefaciens* (*S. liquefaciens*)*, Serratia odorifera* (*S. odorifera*), *Serratia plymuthica* (*S. plymuthica*), *Serratia proteamaculans* (*S. proteamaculans*), *Serratia rubidaea* (*S. rubidaea*), *Serratia ureilytica* (*S. ureilytica*), *Proteus mirabilis* (*P. mirabilis*), *Proteus vulgaris* (*P. vulgaris*), *Proteus myxofaciens* (*P. myxofaciens*)*, Proteus penneri* (*P. penneri*), *Proteus hauseri* (*P. hauseri*)), *Morganella morganii* (*M. morganii*), *Providencia alcalifaciens* (*P. alcalifaciens*), *Providenciarustigianii* (*P. rustigianii*), *Providencia stuartii* (*P. stuartii*), *Providencia rettgeri* (*P. rettgeri*), *Providencia heimbachae* (*P. heimbochae*), *Hafnia alvei* (*H. alvei*),*and Pantoea agglomerans* (*P. agglomerans*).

Embodiment D28: The modified bacterium of any one of the preceding embodiments D, wherein the bacterium is *Salmonella typhimurium.*

Embodiment D29: The modified bacterium of embodiment D28, wherein the starting strain is *Salmonella typhimurium* SL7207.

Embodiment D30: The modified bacterium of any one of the preceding embodiments D, wherein the bacterium expresses a wild-type lipopolysaccharide (LPS).

Embodiment D31: The modified bacterium of any one of the preceding embodiments D, wherein, when administrated to a subject with a tumor, the bacterium is capable of surviving and proliferating in tumor tissue and is rapidly cleared in normal tissue.

Embodiment D32: The modified bacterium of any one of the preceding embodiments D, wherein, when administrated to a subject with a malignant tumor, the bacterium is capable of inhibiting the growth of the malignant tumor.

Embodiment D33: The modified bacterium of any one of the preceding embodiments D, wherein, when administrated to a subject with a malignant tumor, the bacterium is capable of inducing an anti-tumor specific immune response.

Embodiment D34: The modified bacterium of any one of the preceding embodiments D, wherein, when administrated to a subject with a malignant tumor, the bacterium is capable of inducing anti-tumor immune memory.

Embodiment D35: The modified bacterium of any one of the preceding embodiments D, which does not express a wild-type flagellin.

Embodiment D36: The modified bacterium of any one of the preceding embodiments D, which is deficient in the fliC gene.

Embodiment D37: The modified bacterium of any one of the preceding embodiments D, wherein the survival level of the bacterium in macrophages ranges from about 50% to about 30% of the survival level of the unmodified starting strain.

Embodiment D38: The modified bacterium of any one of embodiments D1-D36, wherein the survival level of the bacterium in macrophages ranges from about 30% to about 10% of the survival level of the unmodified starting strain.

Embodiment D39: The modified bacterium of any one of embodiments D1-D36, wherein the survival level of the bacterium in macrophages ranges from about 10% to about 1% of the survival level of the unmodified starting strain.

### Embodiment E

Embodiment E1: A modified bacterium, wherein the bacterium comprises, as compared to the unmodified starting strain,
i) the expression cassette of embodiment 18, a hypoxia regulated essential gene expression cassette and a pH regulated expression cassette, said essential gene expression cassette comprises an essential gene of said bacterium controlled by a strictly hypoxia inducible promoter; or
ii) an expression cassette comprising the polynucleotide of Embodiment 17 and/or an essential gene controlled by a strictly hypoxia inducible promoter and a pH regulated expression cassette,
wherein the pH regulated expression cassette comprises a gene encoding a bacteria-derived hemolysin protein controlled by a promoter active under acidic pH conditions, wherein the bacterium expresses wild-type lipopolysaccharide (LPS) and the bacterium is deficient in at least one gene or functional expression product thereof required for survival in macrophages.

Embodiment E2: The modified bacterium of embodiment E1, wherein the strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment E3: The modified bacterium of embodiment E1, wherein the strictly hypoxia inducible promoter is ssbp1 promoter.

Embodiment E4: The modified bacterium of any one of the preceding embodiments E, wherein the expression product of the essential gene is responsible for synthesis of 2,6-diaminopimelic acid (DAP) within the bacterium, and when cultured under aerobic conditions, the growth of the bacterium is dependent on the additional DAP or an analog thereof added to the medium.

Embodiment E5: The modified bacterium of embodiment E4, wherein the essential gene is selected from dapA, dapB, dapD, dapE, argD, dapF, and any combination thereof.

Embodiment E6: The modified bacterium of embodiment E4, wherein the essential gene is selected from dapA and dapE.

Embodiment E7: The modified bacterium of any one of the preceding embodiments E, wherein the gene required for survival in macrophages is STM3120, STM3119, slyA, sifA, SPI-2, phoP, or htrA gene.

Embodiment E8: The modified bacterium of any one of the preceding embodiments E, wherein the functional expression product of the gene required for survival in macrophages is an HtrA serine protease family related protein.

Embodiment E9: The modified bacterium of any one of the preceding embodiments E, wherein the bacterium is deficient in the activity of HtrA serine protease.

Embodiment E10: The modified bacterium of any one of the preceding embodiments E, wherein the gene required for survival in macrophages is htrA.

Embodiment E11: The modified bacterium of any one of the preceding embodiments E, wherein the bacterium is deficient in htrA.

Embodiment E12: The modified bacterium of any one of embodiments E1-E11, wherein the essential gene is a gene naturally occurring in the bacterial chromosome, and the natural promoter of the essential gene is functionally replaced by the strictly hypoxia inducible promoter, whereby the expression of the essential gene in the bacterium is completely under the control of the strictly hypoxia inducible promoter.

Embodiment E13: The modified bacterium of any one of embodiments E1-E11, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment E14: The modified bacterium of embodiment E13, wherein the exogenous essential gene expression cassette is integrated into the chromosome of the bacterium, or the expression cassette of embodiment 18 or the expression cassette comprising the polynucleotide of embodiment 17 controlled by a strictly hypoxia inducible promoter is integrated into the chromosome of the bacterium.

Embodiment E15: The modified bacterium of embodiment E13, wherein the exogenous essential gene expression cassette is located outside the chromosome of the bacterium, or the expression cassette of embodiment 18 or an expression cassette comprising a polynucleotide of embodiment 17 controlled by a strictly hypoxia inducible promoter is located outside the chromosome of the bacterium.

Embodiment E16: The modified bacterium of embodiment E15, wherein the exogenous essential gene expression cassette is present in a plasmid carried by the bacterium, or the expression cassette of embodiment 18 or an expression cassette comprising a polynucleotide of embodiment 17 controlled by a strictly hypoxia inducible promoter is present in a plasmid carried by the bacterium.

Embodiment E17: The modified bacterium of any one of the preceding embodiments E, wherein the bacteria-derived hemolysin protein is a gram-negative bacterial hemolysin protein.

Embodiment E18: The modified bacterium of embodiment E17, wherein the gene encoding the gram-negative bacterial hemolysin protein is hlyA or hlyE.

Embodiment E19: The modified bacterium of embodiment E18, wherein the hlyA or hlyE is derived from Listeria monocytogenes, Vibrio cholerae, or Escherichia coli.

Embodiment E20: The modified bacterium of any one of preceding embodiments E, wherein the promoter that is active under acid pH condition is active at a pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment E21: The modified bacterium of any one of embodiments E, wherein the promoter that is active under acid pH condition is selected from the group consisting of sseA, ssrA, ssaB, ssaG, ssaM, and ssaR.

Embodiment E22: The modified bacterium of any one of embodiments E, wherein the promoter that is active under acid pH condition is sseA.

Embodiment E23: The modified bacterium of any one of embodiments E, wherein the unmodified starting strain is a facultative anaerobic bacterium.

Embodiment E24: The modified bacterium of any one of embodiments E, wherein the bacterium is a bacterium of Enterobacteriaceae.

Embodiment E25: The modified bacterium of any one of embodiments E, wherein the bacterium is a bacteirum of *Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp., and Pantoea sp.*

Embodiment E26: The modified bacterium of any one of the preceding embodiments E, wherein the bacterium is selected from the group consisting of E. coli such as the Nissle strain and the BL21 (DE3) strain, *Enterobacter blattae* (*E. blattae*), *Enterobacter fergusonii* (*E. fergusonii*)*, Enterobacter hermannii* (*E. hermannii*), *Enterobacter vulneris* (*E. vulneris*), *Salmonella enterica* (*S. enterica*), *Salmonella bongori* (*S. bongori*), *Salmonella typhi* (*S. typhi*), *Salmonella choleraesuis* (*S. choleraesuis*), *Salmonella typhimurium* (*S. typhimurium*), *Shigella dysenteriae* (*S. dysenteriae*), *Shigella flexneri* (*S. flexneri*), *Shigella boydii* (*S. boydii*), *Shigella sonnei* (*S. sonnei*), *Klebsiella pneumoniae* (*K. peumoniae*), *Klebsiella oxytoca* (*K. oxytoca*), *Yersinia pestis* (*Y. pestis*), *Yersinia enterocolitica* (*Y. enterocolitica*), *Yersinia pseudotuberculosis* (*Y. pseudotuberculosis*), *Yersinia aldouae* (*Y. aldouae*), *Yersinia bercovieri* (*Y. bercovieri), Yersinia frederiksenii* (*Y. frederiksenii*)*, Yersinia intermedia* (*Y. intermedia*), *Yersinia kristersenii* (*Y. kristersenii), Yersinia mollaretti* (*Y. mollaretti*), *Yersinia rohdei* (*Y. rohdei*), *Yersinia ruckeri* (*Y. ruckeri*), *Citrobacter freundii* (*C. freundii*)*, Citrobacter koseri* (*C. kaseri*), *Citrobacter braakii* (*C. braakii*), *Enterobacter aerogenes* (*E. aerogenes*), *Enterobacter cloacae* (*E. cloacae*), *Enterobacter gergoviae* (*E. gergoviac*), *Enterobacter sakazakii* (*E. sakazakii*), *Enterobacter taylorae* (*E. tavlorae*), *Enterobacter amnigenus* (*E. aminigenus*), *Enterobacter intermedius* (*E. intermedius*), *Enterobacter asburiae* (*E. asburiac*), *Enterobacter cancerogenus* (*E. cancerogenus*), *Enterobacter dissolvens* (*E. dissolvens*), *Enterobacter nimipressuralis* (*E. nimipressuralis*), *Serratia marcescens* (*S. marcescens*), *Serratia entomophila* (*S. entomophila*), *Serratia ficaria* (*S. ficaria*), *Serratia fonticola* (*S. fonticola*)*, Serratia grimesii* (*S. grimesii*), *Serratia liquefaciens* (*S. liquefaciens*)*, Serratia odorifera* (*S. odorifera*), *Serratia plymuthica* (*S. plymuthica*), *Serratia proteamaculans* (*S. proteamaculans*), *Serratia rubidaea* (*S. rubidaea*), *Serratia ureilytica* (*S. ureilytica*), *Proteus mirabilis* (*P. mirabilis*), *Proteus vulgaris* (*P. vulgaris), Proteus myxofaciens* (*P. myxofaciens*)*, Proteus penneri* (*P. penneri*), *Proteus hauseri* (*P. hauseri*)), *Morganella morganii* (*M. morganii*), *Providencia alcalifaciens* (*P. alcalifaciens*), *Providenciarustigianii* (*P. rustigianii*), *Providencia stuartii* (*P. stuartii*), *Providencia rettgeri* (*P. rettgeri*), *Providencia heimbachae* (*P. heimbochae*), *Hafnia alvei* (*H. alvei*),and *Pantoea agglomerans* (*P. agglomerans*).

Embodiment E27: The modified bacterium of any one of the preceding embodiments E, wherein the bacterium is *Salmonella typhimurium.*

Embodiment E28: The modified bacterium of embodiment E27, wherein the starting strain is *Salmonella typhimurium* SL7207.

Embodiment E29: The modified bacterium of any one of the preceding embodiments E, wherein, when administrated to a subject with a tumor, the bacterium is capable of surviving and proliferating in tumor tissue and is rapidly cleared in normal tissue.

Embodiment E30: The modified bacterium of any one of the preceding embodiments E, wherein, when administrated to a subject with a malignant tumor, the bacterium is capable of inhibiting the growth of the malignant tumor.

Embodiment E31: The modified bacterium of any one of the preceding embodiments E, wherein, when administrated to a subject with a malignant tumor, the bacterium is capable of inducing an anti-tumor specific immune response.

Embodiment E32: The modified bacterium of any one of the preceding embodiments E, wherein, when administrated to a subject with a malignant tumor, the bacterium is capable of inducing anti-tumor immune memory.

Embodiment E33: The modified bacterium of any one of the preceding embodiments E, which does not express a wild-type flagellin.

Embodiment E34: The modified bacterium of any one of the preceding embodiments E, which is deficient in the fliC gene.

Embodiment E35: The modified bacterium of any one of the preceding embodiments E, wherein the survival level of the bacterium in macrophages ranges from about 50% to about 30% of the survival level of the unmodified starting strain.

Embodiment E36: The modified bacterium of any one of embodiments E1-E34, wherein the survival level of the bacterium in macrophages ranges from about 30% to about 10% of the survival level of the unmodified starting strain.

Embodiment E37: The modified bacterium of any one of embodiments E1-E34, wherein the survival level of the bacterium in macrophages ranges from about 10% to about 1% of the survival level of the unmodified starting strain.

### Embodiment F

Embodiment F1: A modified *Salmonella typhimurium* bacterium, wherein the bacterium comprises, as compared to the unmodified starting strain,
i) the expression cassette of embodiment 18, a hypoxia regulated essential gene expression cassette and a pH regulated expression cassette, said essential gene expression cassette comprises an essential gene of said bacterium controlled by a strictly hypoxia inducible promoter; or
ii) an expression cassette comprising the polynucleotide of Embodiment 17 and/or an essential gene controlled by a strictly hypoxia inducible promoter and a pH regulated expression cassette,
wherein the pH regulated expression cassette comprises a gene encoding a bacteria-derived hemolysin protein controlled by a promoter active under acidic pH conditions, wherein the bacterium expresses wild-type lipopolysaccharide (LPS) and the bacterium is deficient in at least one gene or functional expression product thereof required for survival in macrophages.

Embodiment F2: The modified bacterium of embodiment F1, wherein the strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment F3: The modified bacterium of embodiment F1, wherein the strictly hypoxia inducible promoter is ssbp1 promoter.

Embodiment F4: The modified bacterium of any one of the preceding embodiments F, wherein the expression product of the essential gene is responsible for synthesis of 2,6-diaminopimelic acid (DAP) within the bacterium, and when cultured under aerobic conditions, the growth of the bacterium is dependent on the additional DAP or an analog thereof added to the medium.

Embodiment F5: The modified bacterium of embodiment F4, wherein the essential gene is selected from dapA, dapB, dapD, dapE, argD, dapF, and any combination thereof.

Embodiment F6: The modified bacterium of embodiment F4, wherein the essential gene is selected from dapA and dapE.

Embodiment F7: The modified bacterium of any one of the preceding embodiments F, wherein the gene required for survival in macrophages is STM3120, STM3119, slyA, sifA, SPI-2, phoP, or htrA gene.

Embodiment F8: The modified bacterium of any one of the preceding embodiments F, wherein the gene required for survival in macrophages is the gene involved in or regulating the endogenous anti-oxidative stress response pathway.

Embodiment F9: The modified bacterium of embodiment F8, wherein the gene involved in or regulating the endogenous anti-oxidative stress response pathway is a gene of HtrA serine protease family.

Embodiment F10: The modified bacterium of embodiment F9, wherein the functional expression product of the gene involved in or regulating the endogenous anti-oxidative stress response pathway is an HtrA serine protease family related protein.

Embodiment F11: The modified bacterium of embodiment F9, wherein the bacterium is deficient in the activity of HtrA serine protease.

Embodiment F12: The modified bacterium of embodiment F9, wherein the gene involved in or regulating the endogenous anti-oxidative stress response pathway is htrA.

Embodiment F13: The modified bacterium of embodiment F9, wherein the bacterium is deficient in htrA.

Embodiment F14: The modified bacterium of any one of embodiments F1-F13, wherein the essential gene is a gene naturally occurring in the bacterial chromosome, and the natural promoter of the essential gene is functionally replaced by the strictly hypoxia inducible promoter, whereby the expression of the essential gene in the bacterium is completely under the control of the strictly hypoxia inducible promoter.

Embodiment F15: The modified bacterium of any one of embodiments F1-F13, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment F16: The modified bacterium of Embodiment F15, wherein the exogenous essential gene expression cassette is integrated into the chromosome of the bacterium, or the expression cassette of Embodiment 18 or the expression cassette comprising the polynucleotide of Embodiment 17 controlled by a strictly hypoxia inducible promoter is integrated into the chromosome of the bacterium.

Embodiment F17: The modified bacterium of embodiment F15, wherein the exogenous essential gene expression cassette is located outside the chromosome of the bacterium, or the expression cassette of embodiment 18 or the expression cassette comprising the polynucleotide of embodiment 17 controlled by a strictly hypoxia inducible promoter is located outside the chromosome of the bacterium.

Embodiment F18: The modified bacterium of Embodiment F17, wherein the exogenous essential gene expression cassette is present in a plasmid carried by the bacterium, or the expression cassette of Embodiment 18 or an expression cassette comprising a polynucleotide of Embodiment 17 controlled by a strictly hypoxia inducible promoter is present in a plasmid carried by the bacterium.

Embodiment F19: The modified bacterium of any one of the preceding embodiments F, wherein the bacteria-derived hemolysin protein is a gram-negative bacterial hemolysin protein.

Embodiment F20: The modified bacterium of embodiment F19, wherein the gene encoding the gram-negative bacterial hemolysin protein is hlyA or hlyE.

Embodiment F21: The modified bacterium of embodiment F20, wherein the hlyA or hlyE is derived from Listeria monocytogenes, Vibrio cholerae, or Escherichia coli.

Embodiment F22: The modified bacterium of any one of the preceding embodiments F, wherein the promoter that is active under acid pH condition is active at a pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment F23: The modified bacterium of any one of the preceding embodiments F, wherein the promoter that is active under acid pH condition is selected from the group consisting of sseA, ssrA, ssaB, ssaG, ssaM, and ssaR.

Embodiment F24: The modified bacterium of any one of the preceding embodiments F, wherein the promoter that is active under acid pH condition is sseA.

Embodiment F25: The modified bacterium of any one of the preceding embodiments F, wherein the starting strain is Salmonella typhimurium SL7207.

Embodiment F26: The modified bacterium of any one of the preceding embodiments F, wherein, when administrated to a subject with a tumor, the bacterium is capable of surviving and proliferating in tumor tissue and is rapidly cleared in normal tissue.

Embodiment F27: The modified bacterium of any one of the preceding embodiments F, wherein, when administrated to a subject with a malignant tumor, the bacterium is capable of inhibiting the growth of the malignant tumor.

Embodiment F28: The modified bacterium of any one of the preceding embodiments F, wherein, when administrated to a subject with a malignant tumor, the bacterium is capable of inducing an anti-tumor specific immune response.

Embodiment F29: The modified bacterium of any one of the preceding embodiments F, wherein, when administrated to a subject with a malignant tumor, the bacterium is capable of inducing anti-tumor immune memory.

Embodiment F30: The modified bacterium of any one of the preceding embodiments F, which does not express a wild-type flagellin.

Embodiment F31: The modified bacterium of any one of the preceding embodiments F, which is deficient in the fliC gene.

Embodiment F32: The modified bacterium of any one of the preceding embodiments F, wherein the survival level of the bacterium in macrophages ranges from about 50% to about 30% of the survival level of the unmodified starting strain.

Embodiment F33: The modified bacterium of any one of embodiments F1-F31, wherein the survival level of the bacterium in macrophages ranges from about 30% to about 10% of the survival level of the unmodified starting strain.

Embodiment F34: The modified bacterium of any one of embodiments F1-F31, wherein the survival level of the bacterium in macrophages ranges from about 10% to about 1% of the survival level of the unmodified starting strain.

### Embodiment K

Embodiment K1: a pharmaceutical composition comprising an effective amount of the modified bacterium of any one of the preceding embodiments A, embodiments B, embodiments C, embodiments D, embodiment E, or embodiment F.

Embodiment K2: The pharmaceutical composition of embodiment K1, which is used for treating a malignant tumor.

Embodiment K3: The pharmaceutical composition of embodiment K1, which is used for inducing an anti-tumor specific immune response in a subject with a malignant tumor.

Embodiment K4: The pharmaceutical composition of embodiment K1, which is used for inducing anti-tumor immune memory in a subject with a malignant tumor.

Embodiment K5: The pharmaceutical composition of embodiment K1, which is used for preventing or treating metastasis or recurrence of a malignant tumor.

Embodiment K6: The pharmaceutical composition of embodiment K1, which is used for treating a malignant tumor that is resistant to, or has failed to, a prior anti-tumor therapy.

Embodiment K7: The pharmaceutical composition of embodiments K1-K6, wherein the modified bacterium is a live bacterium.

### Embodiment L

Embodiment L1: a method for treating a malignant tumor, comprising administering to a subject suffering the malignant tumor an effective amount of the modified bacterium of any one of the preceding embodiments A, embodiments B, embodiments C, embodiments D, embodiments E, or embodiments F, or the pharmaceutical composition of any one of the preceding embodiments K.

Embodiment L2: a method for inducing an anti-tumor specific immune response in a subject with a malignant tumor, comprising administering to the subject an effective amount of the modified bacterium of any one of the preceding embodiments A, embodiments B, embodiments C, embodiments D, embodiments E, or embodiments F, or the pharmaceutical composition of any one of the preceding embodiments K.

Embodiment L3: a method for inducing anti-tumor immune memory in a subject with a malignant tumor, comprising administering to the subject an effective amount of the modified bacterium of any one of the preceding embodiments A, embodiments B, embodiments C, embodiments D, embodiments E, or embodiments F, or the pharmaceutical composition of any one of the preceding embodiments K.

Embodiment L4: a method for preventing or treating metastasis or recurrence of a malignant tumor, comprising administering to a subject with the malignant tumor an effective amount of the modified bacterium of any one of the preceding embodiments A, embodiments B, embodiments C, embodiments D, embodiments E, or embodiments F, or the pharmaceutical composition of any one of the preceding embodiments K.

Embodiment L5: a method for preventing or treating metastasis or recurrence of a malignant tumor, comprising administering to a subject with or at a high risk of metastasis or recurrence of the malignant tumor an effective amount of the modified bacterium of any one of the preceding embodiments A, embodiments B, embodiments C, embodiments D, embodiments E, or embodiments F, or the pharmaceutical composition of any one of the preceding embodiments K.

Embodiment L6: a method for treating a malignant tumor that is resistant to, or has failed to, a prior anti-tumor therapy, comprising administering to a subject with the malignant tumor that is resistant to, or has failed to, the prior anti-tumor therapy, an effective amount of the modified bacterium of any one of the preceding embodiments A, embodiments B, embodiments C, embodiments D, embodiments E, or embodiments F, or the pharmaceutical composition of any one of the preceding embodiments K.

Embodiment L7: The method of embodiments L1-L6, wherein the modified bacterium is a live bacterium.

### Embodiment M

Embodiment M1: use of the modified bacterium of any one of the preceding embodiments A, embodiments B, embodiments C, embodiments D, embodiments E, or embodiments F in the manufacture of a medicament for treating a malignant tumor.

Embodiment M2: use of the modified bacterium of any one of the preceding embodiments A, embodiments B, embodiments C, embodiments D, embodiments E, or embodiments F in the manufacture of a medicament for inducing an anti-tumor specific immune response in a subject with a malignant tumor.

Embodiment M3: use of the modified bacterium of any one of the preceding embodiments A, embodiments B, embodiments C, embodiments D, embodiments E, or embodiments F in the manufacture of a medicament for inducing anti-tumor immune memory in a subject with a malignant tumor.

Embodiment M4: use of the modified bacterium of any one of the preceding embodiments A, embodiments B, embodiments C, embodiments D, embodiments E, or embodiments F in the manufacture of a medicament for preventing or treating metastasis or recurrence of a malignant tumor.

Embodiment M5: use of the modified bacterium of any one of the preceding embodiments A, embodiments B, embodiments C, embodiments D, embodiments E, or embodiments F in the manufacture of a medicament for treating a malignant tumor that is resistant to, or has failed to, a prior anti-tumor therapy.

Embodiment M6: The use of embodiments M1-M5, wherein the modified bacterium is a live bacterium.

### Embodiment N

Embodiment N1: The pharmaceutical composition of any one of embodiments K, the method of any one of embodiments L, or the use of any one of embodiments M, wherein the malignant tumor is a malignant tumor from the nervous system, respiratory system, digestive system, urinary system, reproductive system, hematopoietic system, lymphatic system, endocrine system, or skin mucosa.

Embodiment N2: The pharmaceutical composition of any one of embodiments K, the method of any one of embodiments L, or the use of any one of embodiments M, wherein the malignant tumor is a sarcoma or carcinoma.

Embodiment N3: The pharmaceutical composition of any one of embodiments K, the method of any one of embodiments L, or the use of any one of embodiments M, wherein the malignant tumor is a solid tumor.

Embodiment N4: The pharmaceutical composition of any one of embodiments K, the method of any one of embodiments L, or the use of any one of embodiments M, wherein malignant tumor is selected from the group consisting of glioma, neuroblastoma, retinoblastoma, nasopharyngeal carcinoma, oral cavity carcinoma, tongue carcinoma, larynx carcinoma, head and neck carcinoma, melanoma, bronchus carcinoma, lung carcinoma, pleural carcinoma, esophagus carcinoma, gastric carcinoma, hepatocellular carcinoma, pancreatic carcinoma, cholangiocarcinoma, colorectal carcinoma, rectal carcinoma, renal cell carcinoma, bladder carcinoma, prostate carcinoma, suprarenoma, thyroid carcinoma, parathyroid gland carcinoma, Pituitary tumor, Hodgkin's disease, non-Hodgkin's lymphoma, multiple myeloma, cervical cancer, ovarian cancer, endometrial cancer, breast cancer, bone cancer, and osteosarcoma.

Embodiment N5: The pharmaceutical composition of any one of embodiments K, the method of any one of embodiments L, or the use of any one of embodiments M, wherein the bacterium, medicament, or pharmaceutical composition of the present invention is administered by intravenous, intratumoral, intramuscular, subcutaneous, intraperitoneal, transcerebral, gastrointestinal, body surface, oral mucosal, transnasal, transrectal, or transvaginal routes.

### DEFINITIONS

"Polypeptide", "peptide", and "protein" are used interchangeably in the present invention to refer to a polymer of amino acid residues. The terms apply to a polymer of amino acids, in which one or more amino acid residues are artificial chemical analogues of corresponding naturally occurring amino acid(s), as well as to a naturally occurring amino acid polymer. The terms "polypeptide", "peptide", "amino acid sequence", and "protein" may also include modified forms including, but not limited to, glycosylation, lipid ligation, sulfation, γcarboxylation of glutamic acid residues, and ADP-ribosylation. The modification also includes the modification to the polypeptide sequence, including, but not limited to, the substitution, deletion, insertion and/or addition of one or more amino acids.

The term "amino acid" includes both naturally occurring amino acids and non-natural amino acids in a protein. Theconventional nomenclature (one-letter and three-letter) of the amino acidsnaturally occurred in proteins is employed, which can be seen in Sambrook.

| Amino acid | One-letter | Three-letter |
|---|---|---|
| Alanine | A | Ala |
| Arginine | R | Arg |
| Asparagine | N | Asn |
| Aspartic acid | D | Asp |
| Cysteine | C | Cys |
| Glutamine | Q | Gln |
| Glutamic acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tryosine | Y | Tyr |
| Valine | V | Val |

For the present invention, in order to determine the percentage identity betweentwo amino acid sequences or two nuleic acid sequences, the sequences arealigned for the purpose of optimal comparison (e.g., a gap can be introduced intothe first amino acid or nucleic acid sequence for the optimal alignment withthe second amino acid or nucleic acid sequence). The amino acid residues or nucleotides at the corresponding amino acid positions or nucleotide positions are then compared. Whena position in the first sequence is occupied by the same amino acid residue ornucleotide at the corresponding position in the second sequence, thesemolecules are identical at this position. Thepercentage identity between two sequences is a function of the number ofidentical positions shared by the sequences (i.e., percentage identity=numberof identical positions/total number of positions (i.e., the overlappingpositions)× 100). Preferably, the two sequences are idnetical in length.

A person skilled in the art knows that various computer programs can be used todetermine the identity between two sequences.

"Amino acid identity percentage" or "amino acid sequence identity percentage" refersto the comparison between the amino acids of two polypeptides, and whenoptimally aligned, the two polypeptides have approximately the specifiedpercentage of identical amino acids. Forexample, "95% amino acid identity" refers to the comparison between the aminoacids of two polypeptides, and when optimally aligned, 95% of the amino acidsof the two polypeptides are identical.

The term "conserved substitution" is also referred toas substitution by "homologous" amino acid residue, meaning a substitution inwhich an amino acid is replaced by an amino acid residue with a similar sidechain, e.g., amino acids with a basic side chain (e.g. lysine, arginine andhistidine), amino acids with a acidic side chain (e.g. aspartic acid, glutamicacid), amino acids with a non-charged polar side chain (e.g. glycine,asparagine, glutamine, serine, threonine, tyrosine, cysteine), amino acids witha non-polar side chain (e.g. alanine, valine, leucine, isoleucine, proline,phenylalanine, methionine, tryptophan), amino acids with a beta-branched sidechain (e.g. threonine, valine, isoleucine) and amino acids with an aromaticside chain (e.g. tyrosine, phenylalanine, tryptophan, histidine).

"Hypoxia regulated essential gene expression cassette" herein means a stretch of DNA capable of promoting the expression of an essential gene under hypoxic conditions, which contains an essential gene under the control of a hypoxia inducible promoter and, if necessary, may further contain other regulated elements required for the expression of the essential gene.

As used herein, "essential gene" refers to a gene that is decisive for the growth and/or survival of a bacterium, in the absence of which or its functional expression product the bacterium fails to survive, divide and/or grow normally. Typical examples of the deletion of an essential gene or a functional expression product thereof are nutritionally deficient strains that cannot survive, divide and/or grow normally under in vitro culture conditions or in vivo environment in the absence of specific exogenous supplements. Essential genes usually appear as a single copy on the bacterial chromosome.

As used herein, "strictly hypoxia inducible promoter" refers to a promoter that is capable of initiating the transcription of a specific gene under anaerobic conditions but is virtually impossible to activate and produce transcripts under aerobic conditions.

"Strictly hypoxia inducible promoters" useful in the present invention can be identified by the following experiments:
An expression strain containing an essential gene (e.g., dapA/dapE) controlled by a promoter to be determined is constructed, and subjected to Luria-Bertani (abbreviated as LB) plate streaking in an anaerobic incubator and anaerobic culture at 37°C for 24 h. Under anaerobic conditions, single clones are picked and resuspended in 20 µL of LB broth. The 20 µl resuspension of a single clone is equally added to the liquid in the following four tubes (A, B, C and D, 5 µl per tube):
A. 2 ml LB broth,
B. 2 ml LB broth,
C. 2 ml LB broth+DAP,
D. 2 ml LB broth+DAP.

The tubes A and C are incubated at 37°C in an anaerobic incubator for 24 h, and the tubes B and D are incubated at 37°C in an aerobic shaker for 24 h. If the bacteria in the tubes A, C and D can grow, and the bacteria in the tube B cannot grow (the detected OD600 value is less than 0.05), the promoter is considered as a strictly hypoxia inducible promoter.

Exemplary strictly hypoxia inducible promoters that can be used in the present invention as identified by the experiments above are shown in Table 1.

**Table 1**

| Promoter name | Sequence | SEQ ID NO: |
|---|---|---|
| pepTp | GCAGGGGTAAAAGTGACCTGACGCAATATTTGTCT TTTCTTGCTTCTTAATAATGTTGTCACAAAAAGTG AGGGTGACTACATGG | 25 |
| fnrSp | CGCCGCAAAGTTTGAGCGAAGTCAATAAACTCTCT ACCCATTCAGGGCAATATCTCTCTTGCAGGTGAAT GCAACGTCAAGCGAT | 26 |
| ysgAp | TCAGAAGAAGCAAAAAGACACTTTACCGAAGGGTT TAACATTTTTTCGTGATACTCATCACCATGACGCA AATGCGTTGCATAAA | 27 |
| ssbp1 | AACCGAGGTCACAACATAGTAAAAGCGCTATTGGT AATGGTACAATCGCGCGTTTACACTTATTCAGAAC GATTTTTTTCAGGAG | 28 |
| Hip1 | GGATAAAAGTGACCTGACGCAATATTTGTCTTTTC TTGCTTAATAATGTTGTCA | 29 |
| BBa_I14018 | TGTAAGTTTATACATAGGCGAGTACTCTGTTATGG | 30 |
| BBa_R1074 | TTAAATTTCCTCTCGTCAGGCCGGAATAACTCCCT ATAATGCGCCACCACACTGATAGTGCTAGTGTAGA TCAC | 31 |
| Ptet-Fnr | AAAATTGATCTGAATCAATATTTTGACAAAAATTG ATCTGAATCAATATTTACTGAGC | 32 |
| Ptet-arcA | GTTAATAAAATGTTATTGACAGTTAATAAAATGTT ATACTGAGC | 33 |
| sseA | TTAGCACGTTAATTATCTATCGTGTATATG | 34 |

As used herein, "gene involved in or regulating the endogenous anti-oxidative stress response pathway" refers to a gene involved in the resistance of bacteria (e.g., intracellular bacteria, such as Salmonella) to the killing by reactiveoxygen species (ROS) in vivo or in vitro.

As used herein, "gene required for survival in macrophages" refers to a gene involved in maintaining or enhancing the viability of intracellular bacteria in macrophages. The functions of the products of these genes are associated to the resistance to the killing effect of macrophages on microorganisms, and thus deletion of these genes or their functional expression products can reduce the viability of bacteria (e.g., intracellular bacteria, such as Salmonella) in macrophages.

As used herein, the "functional expression product" includes RNA, proteins, and the like.

As used herein, "facultative anaerobic bacteria" refers to bacteria that can survive under both aerobic and anaerobic conditions.

As used herein, the "pH regulated expression cassette" refers to a group of gene expression elements in which the expression of the corresponding gene (s) is regulated by environmental pH.

As used herein, "a promoter that is active under acid pH condition" refers to a promoter that can be activated under acid pH condition and regulate the expression of the corresponding gene. In some embodiments, the promoter that is active under acid pH condition of the present invention may be activated or still have promoter activity at a pH less than 7.0, 6.9, 6.8, 6.7, 6.6, 6.5, 6.4, 6.3, 6.2, 6.1, 6.0, 5.9, 5.8, 5.7, 5.6, or 5.5. In some embodiments, the promoter that is active under acid pH condition also has promoter activity at neutral pH (e.g., pH 7.0 to 7.4).

### Examples

### Example 1: Construction of IPTG Inducible Plasmid Expressing Human Interleukin 10 Protein

1.1. The target fragments were amplified by PCR with plasmids pET22b-pelB-Amp (SEQ ID NO: 1) and pSC101-huil10-Amp (SEQ ID NO: 2) as the template, respectively.

Fragment 1 (pET22b-pelB-Amp) was amplified from pET22b-pelB-Amp using the following primer sequences:
Forward primer CACCACCACCACCACCACTGAGATCCG (SEQ ID NO: 35); and
Reverse primer ATCATCATCATCATCGGCCATCGCCGGCTGGGC (SEQ ID NO: 36).

Fragment 2 (D5-linker-huil10) was amplified from pSC101-huil10-Amp using the following primer sequences:
Forward primer TGGCCGATGATGATGATGATAGCCCGGGTCAGGGTACGCA (SEQ ID NO: 37); and
Reverse primer

TCAGTGGTGGTGGTGGTGGTGATTACGGATTTTCATGGTCATATATGC (SEQ ID
NO: 38).

Control fragment 3 (huil10) was amplified from pSC101-hull10-Amp using the following primer sequences:
Forward primer TGGCCAGCCCGGGTCAGGGTACGCA (SEQ ID NO: 39); and
Reverse Primer 
TCAGTGGTGGTGGTGGTGGTGATTACGGATTTTCATGGTCATATATGC (SEQ ID
NO: 40)
PCR amplification was performed with PrimeSTAR Max DNA Polymerase (Takara # R054A) in the following system.

### PCR System

| Reagents | Amount | Final Concentration |
|---|---|---|
| PrimeSTAR Max Premix | 25 µL | 1× |
| Primer 1 | 10-15 pmol | 0.2-0.3 µM |
| Primer 2 | 10-15 pmol | 0.2-0.3 µM |
| Template | < 200 ng | |
| Sterilized Distilled Water | to a total volume of 50 µL | |

The condition for PCR was as follows: denaturation at 98°C for 10 sec, annealing at 55°C for 10 sec, extension at 72°C for 5 sec/kb, 30 cycles of denaturation-annealing-extension in total.

1.2. Fragment 1 and Fragment 2, as well as Fragment 1 and Fragment 3, were recombined by ClonExpress II One Step Cloning Kit (Vazyme # C112) according to the instructions, and chemically transformed into clone competent cells (DH5α Competent E. coli Strain, Vazyme # C502). The recombinant plasmids were designated as plasmid-pET22b-pelB-D5-hil10-Amp (SEQ ID NO: 3) and plasmid-pET22b-pelB- hil10-Amp (SEQ ID NO: 4).

The gene sequence of interleukin-10 fused to the prokaryotic signal peptide pelB and the polar negatively charged aspartic acid Asp linker peptide at the N-terminus is shown in the Appendix (SEQ ID NO: 5), and the linker peptide is shown in bold.

1.3. The clones of competent cells DH5α carrying the recombinant plasmid-pET22b-pelB-D5-hil10-Amp and plasmid-pET22b-pelB-hil10-Amp were identified by PCR with the following identification primers: the forward identification primer 1F with the sequence of CTCGACGCTCTCCCTTATG (SEQ ID NO: 41), and the reverse identification primer 1R with the sequence of CTGACATGGCTTTATAGATACCT (SEQ ID NO: 42).

Positive clones were cultured in LB broth containing penicillin (Amp, 100 mg/L) and plasmids were extracted (Plasmid Mini Kit I OMEGA # D6943-03).

### Example 2: Construction of E. coli Strain BL21 (DE3) Expressing Human Interleukin 10 Protein with the Induction by IPTG

The recombinant plasmid was transformed into the strain BL21 (DE3) for the expression of recombinant protein (BL21(DE3) Competent *E.coli* Strain, Vazyme #C504-03) by chemical transformation. Specifically, the competent cells were thawed on ice, 100 ng of recombinant plasmid was mixed with the competent cells, and placed on ice for 30 min. After a heat shock in a 42 °C water bath for 45 sec, it was immediately placed on ice for 3 min. 900 µL of LB medium (antibiotic-free) was added, the bacteria were incubated in a shaker at 37°C for 1 h (200-250 rpm) and centrifuged at 5,000 rpm for 5 min, 900 µL of supernatant was discarded, the bacteria were resuspended with the remaining amount of medium, and gently spread with a sterile spreader on an LB plate containing penicillin (Amp, 100 mg/L). The plates were inverted in a 37°C incubator overnight.

The successful construction of BL21 (DE3)-plasmid-pET22b-pelB-D5-huil10-Amp strain and BL21 (DE3)-plasmid-pET22b-pelB-huil10-Amp strain was identified by PCR and electrophoresis. The identification primers are the identification primers 1F/1R in 1.3.

### Example 3: Expression and Secretion of Interleukin-10 by Strain BL21 (DE3)-plasmid-pET22b-pelB-D5-huil10-Amp

3.1. The cryopreserved strain (the strain constructed in Example 2) was taken out from -80°C, placed on ice until thaw, and isolated by streaking on an LB plate containing penicillin (Amp, 100 mg/L); the plate was placed in an incubator at 37°C overnight. Single colonies grown vigorously and individually were picked the next day, inoculated in sterile culture tubes with 4 mL LB medium containing penicillin (Amp, 100 mg/L), and incubated at 37°C. When the culture had an OD=0.4-0.5, IPTG was added to a final concentration of 0.1 mM for an overnight induction in a shaker at 16°C (induction for 14-16 h, 200-250 rpm), and the bacterial culture was taken out and kept on ice.

3.2. The culture was centrifuged at 4,200 rpm and 4°C for 10 min, and the bacterial pellet and the supernatant of LB medium were separately collected. The LB medium supernatant was placed in an ultrafiltration tube (Amicon Ultra, #UFC901096) and centrifuged at 2,500 rpm for 2 h at 4°C, and the concentrated LB medium supernatant was collected, in which the secreted IL-10 was present.

3.3. The lysing solution (Qiagen, Qproteome Bacterial Protein Prep Kit #37900, performed by reference to the experimental manual, Qproteome Bacterial Protein Preparation Handbook, www.qiagen.com) was added to the bacterial pellet, the mixture was placed on ice for 30 min, and then centrifuged at 4°C at 14,000 rpm for 30 min, and the bacterial debris pellet and the supernatant of the lysate were separately collected. The bacterial debris precipitate contains functional protein in inclusion body, and the lysate supernatant contains the expressed and unsecreted soluble proteins.

### 3.4. Characterization of Expressed and Secreted Interleukin-10

3.4.1. 5×SDS loading buffer (Beyotime, # P0015) was added to the above collected samples (concentrated LB medium supernatant, bacterial debris precipitate and lysate supernatant), followed by the metal bath at 100°C for 5 min; then SDS-PAGE was performed: 80 V for 20 min, and increasing the voltage to 120 V after the samples were transferred to the interface between the separation gel and the concentrating gel, until the dye completely left the gel.

3.4.2 According to the instructions, a Pyxis corollary PVDF membrane and transferring instrument were used for membrane transfer. After the membrane transfer, the membrane was washed sequentially with TBS buffer and TBST buffer; the PVDF membrane was blocked with 5% skim milk (dissolved in TBST buffer) at room temperature for 1 h; and incubated overnight at 4°C with the primary antibody (6*his, his-tag monoclonal antibody, Proteintech, # 66005-1-lg) which was diluted in 5% skim milk at a ratio of 1:10000; the PVDF membrane incubated overnight was washed for several times with TBST buffer to remove the antibody on the surface of the membrane, and incubated at room temperature for 1 h with the secondary antibody (HRP-conjugated Affinipure Goat Anti-Rabbit IgG(H+L), Proteintech, # SA00001-2-100UL) which was diluted in 5% skim milk at a ratio of 1:5000; the incubated PVDF membrane was washed for several times with TBST buffer to remove the unbound antibody on the membrane surface, and finally the membrane was soaked in TBS. The prepared developing solutions A and B (Thermo Fisher, SuperSignal^{™} West Pico PLUS Chemiluminecent Substrate) were mixed in a ratio of 1:1, the membrane was placed in a tray, and the mixed solution was poured into the tray, followed by the exposure for 5 min and the imaging with a Jena multifunctional imager.

As shown in FIG. 1, in the presence of the D5 peptide linker in the fusion polypeptide, E. coli can express and secrete IL-10 upon IPTG induction, while in the absence of the D5 peptide linker, E. coli can express IL-10 upon IPTG induction, but the expressed IL-10 cannot be secreted out of the cell.

### Example 4: Construction of Plasmid for the Constitutive Expression of Human Interleukin 10 Protein

4.1. The desired fragments were amplified by PCR with plasmid-pET22b-pelB-D5-huil10-Amp (SEQ ID NO: 3) and pSC101-ptac-sfGFP-KanR (SEQ ID NO: 6) plasmids as templates, respectively.

Fragment 4 (pSC101-ptac-KanR) was amplified from pSC101-ptac-sfGFP-KanR (SEQ ID NO: 6) using the following primer sequences:
Forward primer CTGCTAACAAAGCCCGAAAG (SEQ ID NO: 43); and
Reverse primer CTTTCCTGTGTGAATTATTTCTAGAGG (SEQ ID NO: 44).

Fragment 5 (pelB-D5linker-huil10) was amplified from plasmid-pET22b-pelB-D5-huil10-Amp (SEQ ID NO: 3) using the following primer sequences:
Forward Primer
   AAATAATTCACACAGGAAAGtataCACATCATGAAATACCTGCTGCCG (SEQ ID NO: 45); and
Reverse Primer
   CTTTCGGGCTTTGTTAGCAGCCTTTCGGGCTTTGTTAGCA (SEQ ID NO: 46), with an annealing temperature of 55°C.

Fragment 6 (pelB-huil10) was amplified from plasmid-pET22b-pelB-huil10-Amp (SEQ ID NO: 4) using the following primer sequences:
Forward Primer
   AAATAATTCACACAGGAAAGtataCACATCATGAAATACCTGCTGCCG (SEQ ID NO: 45); and
Reverse Primer
   CTTTCGGGCTTTGTTAGCAGCCTTTCGGGCTTTGTTAGCA (SEQ ID NO: 46), with an annealing temperature of 55°C.

4.2. The fragment 4 and the fragment 5, as well as the fragment 4 and the fragment 6, were recombined by ClonExpress II One Step Cloning Kit (Vazyme # C112), respectively, and chemically transformed into the clone competent cells (DH5α Competent E. coli Strain, Vazyme # C502), and the recombinant plasmids were designated as plasmid-pSC101-ptac-pelB-D5-huil10-KanR (SEQ ID NO: 7) and plasmid-pSC101-ptac-pelB-huil10-KanR (SEQ ID NO: 8).

4.3. The cloned competent cells DH5α carrying the recombinant plasmids plasmid-pSC101-ptac-pelB-D5-huil10-KanR and plasmid-pSC101-ptac-pelB-huil10-KanR were identified by PCR, using the following identification primers: forward identification primer 2F with a sequence of CCGTCTTACTGTCAAGAGGAC (SEQ ID NO: 47), reverse identification primer 2R with a sequence of CTGACATGGCTTTATAGATACCT (SEQ ID NO: 48).

Positive clones were cultured in LB broth containing kanamycin (50 mg/L) and plasmids were extracted (Plasmid Mini Kit I OMEGA # D6943-03).

### Example 5: Construction of Escherichia coli Strain BL21 (DE3) for the Constitutive Expression of Human Interleukin 10 Protein

The plasmids plasmid-pSC101-ptac-pelB-D5-huil10-KanR and plasmid-pSC101-ptac-pelB-huil10-KanR were chemically transformed into strain BL21 (DE3) the expression of recombinant protein, respectively, according to a method similar to that of Example 2 (except the use of an LB plate containing 50 mg/L kanamycin) (Sangon Biotech (Shanghai) Co., Ltd., B528419, referring to the product instructions for the chemical transformation method, https://store.sangon.com/product).

The successful construction of BL21 (DE3)-plasmid-pSC101-ptac-pelB-D5-huil10-KanR strain and the BL21 (DE3)-plasmid-pSC101-ptac-pelB-huil10-KanR strain was identified by PCR and electrophoresis. The identification primers are the above identification primers 2F/2R.

### Example 6: Expression and Secretion of Interleukin-10 by Strain BL21 (DE3)-plasmid-pSC101-ptac-pelB-D5-huil10-KanR

6.1. The cryopreserved strain (the strain constructed in Example 5) was taken out from -80°C, placed on ice until thaw, and isolated by streaking on an LB plate containing 50 mg/L kanamycin; the plate was placed in an incubator at 37°C for overnight culture. The next day, a vigorous single colony was picked, inoculated into a sterile culture tube with 4 mL LB broth containing 50 mg/L kanamycin and cultured overnight, and the bacterial solution was taken out and kept on ice.

6.2. The culture was centrifuged at 4,200 rpm for 10 min, and the bacterial pellet and the supernatant of LB medium were separately collected. The LB medium supernatant was placed in an ultrafiltration tube (Amicon Ultra, # UFC901096) and centrifuged at 2,500 rpm for 2 h at 4°C, and the concentrated LB medium supernatant was collected, in which the secreted IL-10 was present.

6.3. The lysis solution (Qiagen, # 37900) was added to the bacterial pellet, the mixture was placed on ice for 30 min, and then centrifuged at 4°C at 14,000 rpm for 30 min, and the bacterial debris pellet and the supernatant of the lysate were separately collected. The bacterial debris pellete contains functional protein in inclusion body, and the lysate supernatant contains expressed and unsecreted soluble proteins.

### 6.4. Western Blot Analysis of Expressed and Secreted IL-10

IL-10 in each sample was analyzed by Western Blot as described in Example 3.4. As shown in FIG. 2, in the presence of the D5 peptide linker in the fusion polypeptide, E. coli can express IL-10 by the ptac constitutive promoter and secrete the same, while in the absence of the D5 peptide linker, IL-10 expressed by E. coli cannot be secreted out of the cell.

### Example 7: Effect of Peptide Linker on IL-10 Secretion

The purpose of this example was to identify the effect of different peptide linker on the expression in bacterium and secretion from bacterium of IL-10.

To this end, the inventors constructed the peptide linkers as shown in Table 2 according to amino acid types, basic and polar uncharged amino acid and amino acid numbers.

### Peptide Linkers and the Coding Nucleotide Sequences thereof

| Amino Acid Sequence | Nucleotide Sequence |
|---|---|
| E5 | GAAGAAGAAGAAGAA |
| N5 | AACAACAACAACAAC |
| Q5 | CAGCAGCAGCAGCAG |
| K5 | AAGAAGAAGAAGAAG |
| R5 | CGGCGGCGGCGGCGG |
| D1 | GAT |
| D2 | GATGAT |
| D3 | GATGATGAT |
| D4 | GATGATGATGAT |
| D5 | GATGATGATGATGAT |
| D6 | GATGATGATGATGATGAT |
| D7 | GATGATGATGATGATGATGAT |
| D8 | GATGATGATGATGATGATGATGAT |
| D9 | GATGATGATGATGATGATGATGATGAT |
| D10 | GATGATGATGATGATGATGATGATGATGAT |
| D3E2 | GATGATGATGAAGAA |
| D3E3 | GATGATGATGAAGAAGAA |
| DEDED | GATGAAGATGAAGAT |
| DDEDD | GATGATGAAGATGAT |
| DDEDDE | GATGATGAAGATGATGAA |
| D3N2 | GAAGAAGAAAACAAC |
| D3N3 | GAAGAAGAAAACAACAAC |

The nucleotide sequence encoding a peptide linker in Table 2 were inserted between the nucleotide sequence encoding the signal peptide in the plasmid plasmid-pET22b-pelB-huil10-Amp (SEQ ID NO: 4) and the nucleotide sequence encoding the huIL10, to obtain plasmid plasmid-pET22b-pelB-peptide linker-huil10-Amp encoding different pelB+peptide linker+huil10 fusion polypeptides, in which plasmid-pET22b-pelB-D5-huil10-Amp was prepared as shown in Example 1.

The prepared plasmid was transformed into E. coli and the expression of the fusion polypeptide was detected as described in Examples 2 and 3. As shown in FIG. 3, the fusion polypeptides comprising peptide linker D4 or D5 can be expressed and secreted from E. coli into the culture supernatant, while the fusion polypeptide comprising another peptide linker can be expressed, but the secretion from E. coli into the culture supernatant was not detected.

### Example 8

The purpose of this example is to construct a plasmid for the constitutive high expression and secretion of human/murine interleukin-10, and to test the secretion of the fusion polypeptide containing peptide linker D5 (pelB-D5-huil10) with different bacterial chassis and plasmids with different copy numbers.

### 8.1. Construction of Plasmid for Constitutive High Expression and Secretion of Human Interleukin-10

The pelB-D5-huil10 DNA fragment was amplified by PCR with the plasmid plasmid-pET22b-pelB-D5-huil10-Amp (SEQ ID NO: 3) as the template; and the linear fragment was amplified by PCR with the plasmid pUC-ptac-bba0032-KanR (SEQ ID NO: 50) as the template.
pelB-D5-huil10 was amplified with the forward primer of
   AAATAATTCACACAGGAAAGtataCACATCATGAAATACCTGCTGCCG (SEQ ID NO:45); and
the reverse primer of
   CTTTCGGGCTTTGTTAGCAGCCTTTCGGGCTTTGTTAGCA (SEQ ID NO: 46), with an annealing temperature of 55°C.
pUC-ptac-bba0032-KanR was amplified with the forward primer of
   CTTTCCTGTGTGAattatttctagagg (SEQ ID NO:44); and
the reverse primer of cacgcttagcaataactag (SEQ ID NO: 49), with an annealing temperature of 55°C.

The fragments were recombined by ClonExpress II One Step Cloning Kit (Vazyme # C112) and chemically transformed into clonal competent cells (DH5α Competent *E. coli* Strain, Vazyme # C502), respectively, and the recombinant plasmid was designated as plasmid-pUC-ptac-pelB-D5-huil10-KanR (SEQ ID NO: 51).

The transformed clones were identified using the following primers:
iden-bb-F: ctgattctgtggACAAGAGG; and
iden-huil10-R: CAGGATCCTGATTTTCTGCC.

### 8.2. Construction of Plasmid for the Constitutive High Expression and Secretion of Murine Interleukin-10

The plasmid plasmid-pET22b-pelB-D5-mil10-Amp containing the nucleotide sequence encoding murine interleukin 10 was synthesized as a template, and the pelB-D5-mil10 was amplified as described above and recombined with the pUC-ptac-bba0032-KanR plasmid backbone through the Vazyme one-step cloning kit to obtain a recombinant plasmid for the constitutive expression of murine interleukin 10, which was designated as plasmid-pUC-ptac-pelB-D5-mil10-KanR (SEQ ID NO: 52).

The primers for amplifying pelB-D5-mil10 were the same as those for constructing the plasmid for human interleukin above,
the forward primer of
   AAATAATTCACACAGGAAAGtataCACATCATGAAATACCTGCTGCCG (SEQ ID NO:45); and
the reverse primer of
   CTTTCGGGCTTTGTTAGCAGCCTTTCGGGCTTTGTTAGCA (SEQ ID NO: 46), with an annealing temperature of 55°C.

The forward primer for amplifying pUC-ptac-bba0032-KanR was CTTTCCTGTGTGAatatttctagg (SEQ ID NO: 44); the reverse primer was cacgctagcaataactag (SEQ ID NO: 49), with an annealing temperature of 55°C.

The fragments were recombined by ClonExpress II One Step Cloning Kit (Vazyme # C112), respectively, and chemically transformed into cloning competent cells (DH5α Competent E. coli Strain, Vazyme # C502), the recombinant plasmid was designated as plasmid-pUC-ptac-pelB-D5-mil10-KanR (SEQ ID NO: 52).

The transformed clones were identified using the following primers:
iden-bb-F: ctgattctgtggACAAGAGG; and
iden-huil10-R: CAGGATCCTGATTTTCTGCC.

### Example 9: Construction of Variant Strain of Salmonella typhimurium SL7207 Strain

9.1. Desired fragments were obtained by PCR amplification using PsseA-hlyA-loxp-KnaR-loxp plasmid (SEQ ID NO: 9) and Pssbp1-dapE-Cm plasmid (SEQ ID NO: 10) as templates. For PsseA-hlyA-loxp-KnaR-loxp, the following primer sequences were used: the forward primer TTCACAGAAAAGTGTTGCCCCCTTCCATGGCGGAAGGGGGACAAA-GGTGAATTTGTCCTACTCAGGAGAGAGCGTTCA (SEQ ID NO: 11) and the reverse primer TCTGACGTACACAGCAATTTTGCGTTACCTGTTAATCGAGATTGAAACACC-GAAGAAAGGCCCACCCG (SEQ ID NO: 12); for Pssbp1-dapE-Cm, the following primer sequences were used: the forward primer TTGGTTTCAGTGAATCCCGTTATCAGCAGTT-TTTTGATGAGGTGTAGTCTATTTGTCCTACTCAGGAGAGCG (SEQ ID NO: 13) and the reverse primer CAATATTTTGCCAGCCAGTCCATGCTTATTTCCTCTTACCGGAACGC-TCACGAAGAAAGGCCCACCCG (SEQ ID NO: 14), and the annealing temperature is 55 °C. Desired fragment 1 was PsseA-hlyA-loxp-KnaR-loxp (SEQ ID NO: 1) and desired fragment 2 was Pssbp1-dapE-Cm (SEQ ID NO: 2).

9.2. The pSim6 plasmid (BioVector NTCC Inc.: 3574840) containing the λ phage Red recombinase was introduced into a facultative anaerobic Salmonella strain SL7207 (NCBI: ASM1320710v1) to prepare a pSim6 plasmid-containing SL7207 strain electrocompetent cells SL7207 (pSim6).

9.3. Desired fragment 1 was introduced into SL7207 (pSim6) competent cells by electroporation (E = 18KV/cm). The dapE gene (which regulates synthesis of diaminopimelic acid essential for cell wall synthesis) in the SL7207 genome was replaced with desired fragment 1 using λ-red homologous recombination. Specifically, the pSim6 plasmid-containing target strain was cultured at 30 °C; the expression of recombinase was conducted at 42 °C for 15 min; the homologous recombination process was completed by adding homologous arms containing 50 bp away from each upstream and downstream of the target gene locus to the PCR primers under the effect of a homologous recombinase to obtain the strain SL7207 (ΔdapE:: PsseA-hlyA-KnaR).

9.4. The Cre plasmid (Gene Bridges: A112) containing the P1 phage Cre recombinase was introduced into the strain SL7207 (ΔdapE:: PsseA-hlyA-KnaR) for recombination to remove kanamycin resistance, thereby obtaining the strain SL7207 (ΔdapE:: PsseA-hlyA).

9.5. The pSim6 plasmid was introduced into the strain SL7207 (ΔdapE:: PsseA-hlyA) to prepare SL7207 (ΔdapE:: PsseA-hlyA) (pSim6) electrocompetent cells.

9.6. Desired fragment 2 was introduced into SL7207 (ΔdapE:: PsseA-hlyA) (pSim6) electrocompetent cells by electroporation (E = 18KV / cm). The htrA gene (encoding a serine protease) in the SL7207 genome (ΔdapE:: PsseA-hlyA) was replaced with desired fragment 2 using λ-red homologous recombination to obtain the strain DB-ZW1, which was an SL7207 strain (ΔdapE:: PsseA-hlyA; ΔhtrA:: Pssbp1-dapE-Cm) deficient in htrA gene, containing an hlyA gene under the control of PsseA and a dapE gene under the control of a strictly hypoxia inducible promoter Pssbp1.

The strain construction scheme was shown in FIG.4.

### Example 10: Anti-tumor effects on multiple tumor models by DB-ZW1

### 10.1. Construction of tumor model

C57BL/6 mice (purchased from Beijing Vital River Laboratory Animal Technology Co., Ltd., with a body weight of about 18g, kept in a SPF environment) were subcutaneously inoculated with 1 × 10⁶ MB49 mouse bladder cancer cells (Merck SCC148)/ B16 melanoma cells (ATCC: CRL-6475) to establish a subcutaneous tumor model for mouse bladder cancer / melanoma. Experiments were carried out 14-18 days after the inoculation when the tumor volume was about 100 mm ³. In situ colon cancer model was induced by DSS/AOM: 1 week after a single intraperitoneal injection of 10 mg/kg AOM, the mice was fed with water containing 2.5% DSS for 7 days, and then normal drinking water for 14 days, which was one cycle of DSS treatment. The DSS treatment was repeated for another 3 cycles to obtain a mouse model with colorectal cancer.

10.2. Changes in the distribution of DB-ZW1 in tumors and different organs

1 × 10 ⁷ CFU of DB-ZW1 (125 µL) was injected via tail vein into MB49 bladder tumor-bearing mice, with 3 mice in each group. Approximately 90% of DB-ZW1 in mice was observed to reside in tumors 1 day post injection (1 dpi). The concentration of DB-ZW1 in tumors was further elevated by 100-fold (to 10⁸ CFU/g) within the following 2 days and maintained at a high level for the following two weeks. In contrast, the DB-ZW1 concentration in normal organs steadily decreased to < 10² CFU/g within two weeks (FIG. 5A).

To further determine whether DB-ZW1 preferentially proliferated in tumors, the number of bacteria distributed in different organs was determined 24 hours post injection of 1 × 10⁷ CFU of DB-ZW1 bacteria with a volume of 125 µL into MB49 bladder tumor-bearing mice via tail vein, with 3 mice in each group (FIG. 7B). The results showed that DB-ZW1 spread rapidly in mice within 30 minutes post injection, and most of DB-ZW1 cells (about 99.9%) were distributed in the liver, spleen and blood. And the number of DB-ZW1 cells was significantly reduced within 4 hours post injection, and the total number of DB-ZW1 cells was reduced by about 90%. Subsequently, DB-ZW1 in tumors proliferated exponentially at a growth rate of about 0.7 h⁻¹, and saturated within 3 days. In contrast, in normal organs, the number of DB-ZW1 cells were all steadily reduced. This specific tumor growth profile of DB-ZW1 was key to ensuring the validity of the following findings.

### 10.3. Anti-tumor effects of DB-ZW1 on bladder cancer, melanoma and colon cancer

DB-ZW1 was used to treat subcutaneous bladder cancer, in situ melanoma and subcutaneous and in situ colon cancer mice, and 1 × 10⁷ CFU of DB-ZW1 with a volume of 125 µL was injected into tumor-bearing mice via tail vein, with 5 mice in each group. As shown in FIGs. 8A-C, DB-ZW1 significantly reduced the tumor volume. In addition, the treatment of mice with in situ colon cancer using DB-ZW1 also significantly reduced the number of tumors in the colon of mice (FIG.6D).

### Example 11: Construction and Characterization of Bacteria Expressing and Secreting IL-10

The purpose of this example was to detect the effects of constitutive expression and secretion of the fusion polypeptide of the present invention in different bacteria.

The plasmids obtained in Example 8 were electrotransformed into E. coli Nissle and Salmonella typhimurium DB-ZW1 (see Example 9) to obtain strains Nissle-pelB-D5-huIL10, Nissle-pelB-D5-mIL10, DB-ZW1-pelB-D5-huIL10 and DB-ZW1 (Salmonella)-pelB-D5-mIL10.

Bacteria were cultured to express the fusion polypeptides (pelB-D5-huil10 and pelB-D5-mil10) which were subjected to Western Blot analysis as described in Example 6. Proteins were quantified by ImageJ analysis of Western Blot images.

As shown in FIG. 7, the fusion polypeptide of the present invention can also be expressed in E. coli Nissle strain and anti-tumor Salmonella typhimurium DB-ZW1, and can be efficiently secreted out of bacterial cells. Specifically, the efficiency of secretion of the fusion polypeptide of the present invention from E. coli Nissle and Salmonella typhimurium is about 80% and 70%, respectively.

As shown in FIG. 8, the fusion polypeptide of the present invention can also be expressed in E. coli Nissle strain and anti-tumor Salmonella typhimurium DB-ZW1, and efficiently secreted out of bacterial cells.

### Example 12: Anti-Tumor Effect of Interleukin 10 Secreting Bacteria

The purpose of this example is to verify the anti-tumor effect of anti-tumor bacteria expressing and secreting interleukin 10 through in vivo experiments.

A subcutaneous tumor model for mouse MB49 bladder cancer was constructed as described in Example 10.1.

12.1. E. coli strain Nissle -pelB-D5-IL10, E. coli strain Nissle (control) and PBS (blank control) were administered to tumor-bearing mice (n = 6) (1 × 10 ⁷CFU by tail vein injection, with a volume of 125 µL), respectively. On the day of injecting bacteria to tumor-bearing mice and on the Days 0, 2, 4, 6, 8, 10, 12 and 14 post injection, the growth of tumor in mouse was recorded with a Vernier micrometer.

The longest diameter (a) and the perpendicular largest transverse diameter (b) of the tumors in mice were accurately measured with a Vernier micrometer. The tumor volume (V) was calculated according to the formula, V = a × b ² (in mm³). When the tumor volume is greater than 2000 mm ³, exceeding the ethical value, the mouse was considered to be dead. In addition, on Days 0, 2, 4, 6, 8, 10, 12 and 14 post injection of bacteria, the changes in body weight of tumor-bearing mice were weighed to evaluate the biological safety of bacteria.

As shown in FIG. 9, the tumor size in mice injected with Nissle-pelB-D5-IL10 was significantly reduced as compared with mice injected with Nissle and PBS (P < 0.005), demonstrating that interleukin 10 expressed and secreted by bacteria had biological function and therapeutic effect on tumor.

As shown in FIG. 10, the body weight of mice injected with Nissle-pelB-D5-IL10 and Nissle control slowly recovered to normal body weight levels as the days post injection of bacteria extended. On Day 14 post injection of bacteria, the body weight of mice was not statistically different from that of the PBS group, demonstrating that interleukin 10 expressed and secreted by bacteria is biologically safe.

12.2. DB-ZW1-pelB-D5-IL10, DB-ZW1 (control) and PBS (blank control) were respectively administered to tumor-bearing mice (n = 6) (tail vein injection of 1 × 10⁷ CFU, with a volume of 125 µL). On the day of injecting bacteria to tumor-bearing mice and Days 2, 4, 6, and 8 post injection, the growth of tumor in mice was recorded with a Vernier micrometer, and the body weight was measured.

The longest diameter (a) and the perpendicular largest transverse diameter (b) of the tumors in mice were accurately measured with a Vernier micrometer. The tumor volume (V) was calculated according to the formula, V = a × b² (in mm³). When the tumor volume was greater than 2000 mm³, exceeding the ethical value, the mouse was considered to be dead.

### Example 13: Determination of Expression by Interleukin-10 Secreting Bacteria in Tumor-Bearing Mice

The purpose of this example was to verify that the anti-tumor bacteria were successfully expressed in mice and secreted interleukin 10 by detecting the expression amount of interleukin 10 in the liver, spleen and tumor of tumor-bearing mice, and produced a positive anti-tumor effect.

A subcutaneous tumor model for mouse MB49 bladder cancer was constructed as described in Example 10.1, and the mice were injected with E. coli strain Nissle-pelB-D5-IL10, E. coli strain Nissle (control) and PBS (blank control) as described in Example 12.1.

Mice were dissected on the Day 3 post injection, and the spleen, liver and tumor tissues were weighed. RIPA lysis solution (moderate) (Beyotime # P0013C) was sele cted to fully lyse tissue samples on ice according to the product instructions (see, http s://m.beyotime.com/mobilegoods.do?method=code&code=P0013C).

Interleukin-10 ELISA detection kit (Biolegend ELISA MAX ^{™} Deluxe Set IL-10 # 430604\ # 431414) was used to quantitatively detect the concentration of the expressed Interleukin-10 in each tissue sample according to the product instructions (see https://www.biolegend.com/en-us/products).

### Sequence Listing

SEQ ID NO: 1: pET22b-pelB-Amp 
TGGCGAATGGGACGCGCCCTGTAGCGGCGCATTAAGCGCGGCGGGTGTGGTGGTTACGCGCAGCGTGACCGCTACACTTGCCA
GCGCCCTAGCGCCCGCTCCTTTCGCTTTCTTCCCTTCCTTTCTCGCCACGTTCGCCGGCTTTCCCCGTCAAGCTCTAAATCGG
GGGCTCCCTTTAGGGTTCCGATTTAGTGCTTTACGGCACCTCGACCCCAAAAAACTTGATTAGGGTGATGGTTCACGTAGTGG
GCCATCGCCCTGATAGACGGTTTTTCGCCCTTTGACGTTGGAGTCCACGTTCTTTAATAGTGGACTCTTGTTCCAAACTGGAA
CAACACTCAACCCTATCTCGGTCTATTCTTTTGATTTATAAGGGATTTTGCCGATTTCGGCCTATTGGTTAAAAAATGAGCTG
ATTTAACAAAAATTTAACGCGAATTTTAACAAAATATTAACGTTTACAATTTCAGGTGGCACTTTTCGGGGAAATGTGCGCGG
AACCCCTATTTGTTTATTTTTCTAAATACATTCAAATATGTATCCGCTCATGAGACAATAACCCTGATAAATGCTTCAATAAT
ATTGAAAAAGGAAGAGTATGAGTATTCAACATTTCCGTGTCGCCCTTATTCCCTTTTTTGCGGCATTTTGCCTTCCTGTTTTT
GCTCACCCAGAAACGCTGGTGAAAGTAAAAGATGCTGAAGATCAGTTGGGTGCACGAGTGGGTTACATCGAACTGGATCTCAA
CAGCGGTAAGATCCTTGAGAGTTTTCGCCCCGAAGAACGTTTTCCAATGATGAGCACTTTTAAAGTTCTGCTATGTGGCGCGG
TATTATCCCGTATTGACGCCGGGCAAGAGCAACTCGGTCGCCGCATACACTATTCTCAGAATGACTTGGTTGAGTACTCACCA
GTCACAGAAAAGCATCTTACGGATGGCATGACAGTAAGAGAATTATGCAGTGCTGCCATAACCATGAGTGATAACACTGCGGC
CAACTTACTTCTGACAACGATCGGAGGACCGAAGGAGCTAACCGCTTTTTTGCACAACATGGGGGATCATGTAACTCGCCTTG
ATCGTTGGGAACCGGAGCTGAATGAAGCCATACCAAACGACGAGCGTGACACCACGATGCCTGCAGCAATGGCAACAACGTTG
CGCAAACTATTAACTGGCGAACTACTTACTCTAGCTTCCCGGCAACAATTAATAGACTGGATGGAGGCGGATAAAGTTGCAGG
ACCACTTCTGCGCTCGGCCCTTCCGGCTGGCTGGTTTATTGCTGATAAATCTGGAGCCGGTGAGCGTGGGTCTCGCGGTATCA
TTGCAGCACTGGGGCCAGATGGTAAGCCCTCCCGTATCGTAGTTATCTACACGACGGGGAGTCAGGCAACTATGGATGAACGA
AATAGACAGATCGCTGAGATAGGTGCCTCACTGATTAAGCATTGGTAACTGTCAGACCAAGTTTACTCATATATACTTTAGAT
TGATTTAAAACTTCATTTTTAATTTAAAAGGATCTAGGTGAAGATCCTTTTTGATAATCTCATGACCAAAATCCCTTAACGTG
AGTTTTCGTTCCACTGAGCGTCAGACCCCGTAGAAAAGATCAAAGGATCTTCTTGAGATCCTTTTTTTCTGCGCGTAATCTGC
TGCTTGCAAACAAAAAAACCACCGCTACCAGCGGTGGTTTGTTTGCCGGATCAAGAGCTACCAACTCTTTTTCCGAAGGTAAC
TGGCTTCAGCAGAGCGCAGATACCAAATACTGTCCTTCTAGTGTAGCCGTAGTTAGGCCACCACTTCAAGAACTCTGTAGCAC
CGCCTACATACCTCGCTCTGCTAATCCTGTTACCAGTGGCTGCTGCCAGTGGCGATAAGTCGTGTCTTACCGGGTTGGACTCA
AGACGATAGTTACCGGATAAGGCGCAGCGGTCGGGCTGAACGGGGGGTTCGTGCACACAGCCCAGCTTGGAGCGAACGACCTA
CACCGAACTGAGATACCTACAGCGTGAGCTATGAGAAAGCGCCACGCTTCCCGAAGGGAGAAAGGCGGACAGGTATCCGGTAA
GCGGCAGGGTCGGAACAGGAGAGCGCACGAGGGAGCTTCCAGGGGGAAACGCCTGGTATCTTTATAGTCCTGTCGGGTTTCGC
CACCTCTGACTTGAGCGTCGATTTTTGTGATGCTCGTCAGGGGGGCGGAGCCTATGGAAAAACGCCAGCAACGCGGCCTTTTT
ACGGTTCCTGGCCTTTTGCTGGCCTTTTGCTCACATGTTCTTTCCTGCGTTATCCCCTGATTCTGTGGATAACCGTATTACCG
CCTTTGAGTGAGCTGATACCGCTCGCCGCAGCCGAACGACCGAGCGCAGCGAGTCAGTGAGCGAGGAAGCGGAAGAGCGCCTG
ATGCGGTATTTTCTCCTTACGCATCTGTGCGGTATTTCACACCGCATATATGGTGCACTCTCAGTACAATCTGCTCTGATGCC
GCATAGTTAAGCCAGTATACACTCCGCTATCGCTACGTGACTGGGTCATGGCTGCGCCCCGACACCCGCCAACACCCGCTGAC
GCGCCCTGACGGGCTTGTCTGCTCCCGGCATCCGCTTACAGACAAGCTGTGACCGTCTCCGGGAGCTGCATGTGTCAGAGGTT
TTCACCGTCATCACCGAAACGCGCGAGGCAGCTGCGGTAAAGCTCATCAGCGTGGTCGTGAAGCGATTCACAGATGTCTGCCT
GTTCATCCGCGTCCAGCTCGTTGAGTTTCTCCAGAAGCGTTAATGTCTGGCTTCTGATAAAGCGGGCCATGTTAAGGGCGGTT
TTTTCCTGTTTGGTCACTGATGCCTCCGTGTAAGGGGGATTTCTGTTCATGGGGGTAATGATACCGATGAAACGAGAGAGGAT
GCTCACGATACGGGTTACTGATGATGAACATGCCCGGTTACTGGAACGTTGTGAGGGTAAACAACTGGCGGTATGGATGCGGC
GGGACCAGAGAAAAATCACTCAGGGTCAATGCCAGCGCTTCGTTAATACAGATGTAGGTGTTCCACAGGGTAGCCAGCAGCAT
CCTGCGATGCAGATCCGGAACATAATGGTGCAGGGCGCTGACTTCCGCGTTTCCAGACTTTACGAAACACGGAAACCGAAGAC
CATTCATGTTGTTGCTCAGGTCGCAGACGTTTTGCAGCAGCAGTCGCTTCACGTTCGCTCGCGTATCGGTGATTCATTCTGCT
AACCAGTAAGGCAACCCCGCCAGCCTAGCCGGGTCCTCAACGACAGGAGCACGATCATGCGCACCCGTGGGGCCGCCATGCCG
GCGATAATGGCCTGCTTCTCGCCGAAACGTTTGGTGGCGGGACCAGTGACGAAGGCTTGAGCGAGGGCGTGCAAGATTCCGAA
TACCGCAAGCGACAGGCCGATCATCGTCGCGCTCCAGCGAAAGCGGTCCTCGCCGAAAATGACCCAGAGCGCTGCCGGCACCT
GTCCTACGAGTTGCATGATAAAGAAGACAGTCATAAGTGCGGCGACGATAGTCATGCCCCGCGCCCACCGGAAGGAGCTGACT
GGGTTGAAGGCTCTCAAGGGCATCGGTCGAGATCCCGGTGCCTAATGAGTGAGCTAACTTACATTAATTGCGTTGCGCTCACT
GCCCGCTTTCCAGTCGGGAAACCTGTCGTGCCAGCTGCATTAATGAATCGGCCAACGCGCGGGGAGAGGCGGTTTGCGTATTG
GGCGCCAGGGTGGTTTTTCTTTTCACCAGTGAGACGGGCAACAGCTGATTGCCCTTCACCGCCTGGCCCTGAGAGAGTTGCAG
CAAGCGGTCCACGCTGGTTTGCCCCAGCAGGCGAAAATCCTGTTTGATGGTGGTTAACGGCGGGATATAACATGAGCTGTCTT
CGGTATCGTCGTATCCCACTACCGAGATATCCGCACCAACGCGCAGCCCGGACTCGGTAATGGCGCGCATTGCGCCCAGCGCC
ATCTGATCGTTGGCAACCAGCATCGCAGTGGGAACGATGCCCTCATTCAGCATTTGCATGGTTTGTTGAAAACCGGACATGGC
ACTCCAGTCGCCTTCCCGTTCCGCTATCGGCTGAATTTGATTGCGAGTGAGATATTTATGCCAGCCAGCCAGACGCAGACGCG
CCGAGACAGAACTTAATGGGCCCGCTAACAGCGCGATTTGCTGGTGACCCAATGCGACCAGATGCTCCACGCCCAGTCGCGTA
CCGTCTTCATGGGAGAAAATAATACTGTTGATGGGTGTCTGGTCAGAGACATCAAGAAATAACGCCGGAACATTAGTGCAGGC
AGCTTCCACAGCAATGGCATCCTGGTCATCCAGCGGATAGTTAATGATCAGCCCACTGACGCGTTGCGCGAGAAGATTGTGCA
CCGCCGCTTTACAGGCTTCGACGCCGCTTCGTTCTACCATCGACACCACCACGCTGGCACCCAGTTGATCGGCGCGAGATTTA
ATCGCCGCGACAATTTGCGACGGCGCGTGCAGGGCCAGACTGGAGGTGGCAACGCCAATCAGCAACGACTGTTTGCCCGCCAG
TTGTTGTGCCACGCGGTTGGGAATGTAATTCAGCTCCGCCATCGCCGCTTCCACTTTTTCCCGCGTTTTCGCAGAAACGTGGC
TGGCCTGGTTCACCACGCGGGAAACGGTCTGATAAGAGACACCGGCATACTCTGCGACATCGTATAACGTTACTGGTTTCACA
TTCACCACCCTGAATTGACTCTCTTCCGGGCGCTATCATGCCATACCGCGAAAGGTTTTGCGCCATTCGATGGTGTCCGGGAT
CTCGACGCTCTCCCTTATGCGACTCCTGCATTAGGAAGCAGCCCAGTAGTAGGTTGAGGCCGTTGAGCACCGCCGCCGCAAGG
AATGGTGCATGCAAGGAGATGGCGCCCAACAGTCCCCCGGCCACGGGGCCTGCCACCATACCCACGCCGAAACAAGCGCTCAT
GAGCCCGAAGTGGCGAGCCCGATCTTCCCCATCGGTGATGTCGGCGATATAGGCGCCAGCAACCGCACCTGTGGCGCCGGTGA
TGCCGGCCACGATGCGTCCGGCGTAGAGGATCGAGATCTCGATCCCGCGAAATTAATACGACTCACTATAGGGGAATTGTGAG
CGGATAACAATTCCCCTCTAGAAATAATTTTGTTTAACTTTAAGAAGGAGATATACATATGAAATACCTGCTGCCGACCGCTG
CTGCTGGTCTGCTGCTCCTCGCTGCCCAGCCGGCGATGGCCGATGATGATGATGATAGCCGCGGCCAGTACAGCCGCGAGGAC
AATAACTGCACCCATTTTCCGGTGGGCCAGAGCCATATGCTGCTGGAACTGCGCACCGCGTTTAGCCAAGTTAAGACGTTCTT
CCAGACCAAAGACCAGCTGGATAACATTTTACTGACCGACTCTTTAATGCAAGATTTTAAGGGCTATCTGGGCTGCCAAGCTT
TAAGCGAGATGATCCAGTTCTATTTAGTGGAAGTGATGCCGCAAGCTGAAAAACACGGCCCGGAGATCAAAGAACATTTAAAC
 
TCTTTAGGCGAGAAACTGAAGACTTTACGCATGCGTTTACGTCGTTGCCATCGCTTTCTGCCGTGCGAGAACAAAAGCAAAGC
GGTGGAGCAAGTTAAGAGCGACTTCAACAAACTGCAAGATCAAGGTGTGTATAAGGCGATGAACGAATTCGATATCTTTATCA
ACTGCATCGAAGCGTATATGATGATTAAAATGAAAAGCCACCACCACCACCACCACTGAGATCCGGCTGCTAACAAAGCCCGA
AAGGAAGCTGAGTTGGCTGCTGCCACCGCTGAGCAATAACTAGCATAACCCCTTGGGGCCTCTAAACGGGTCTTGAGGGGTTT
TTTGCTGAAAGGAGGAACTATATCCGGAT
SEQ ID NO: 2: pSC101-huil10-Amp 
ctaaattgtaagcgttaatattttgttaaaattcgcgttaaatttttgttaaatcagctcattttttaaccaataggccgaaa
tcggcaaaatcccttataaatcaaaagaatagaccgagatagggttgagtgttgttccagtttggaacaagagtccactatta
aagaacgtggactccaacgtcaaagggcgaaaaaccgtctatcagggcgatggcccactacgtgaaccatcaccctaatcaag
ttttttggggtcgaggtgccgtaaagcactaaatcggaaccctaaagggagcccccgatttagagcttgacggggaaagccgg
cgaacgtggcgagaaaggaagggaagaaagcgaaaggagcgggcgctagggcgctggcaagtgtagcggtcacgctgcgcgta
accaccacacccgccgcgcttaatgcgccgctacagggcgcgtcccattcgccattcaggctgcgcaactgttgggaagggcg
atcggtgcgggcctcttcgctattacgccagctggcgaaagggggatgtgctgcaaggcgattaagttgggtaacgccagggt
tttcccagtcacgacgttgtaaaacgacggccagtgagcgcgcgtaatacgactcactataggcctctagaaataattttgtt
taactttaagaaggagatataCAatgaaaaaacgctggttttggtttgtgctggcgctgattgtgctgtttccgtttcagagc
agctttgcgaaccagAGCCCGGGTCAGGGTACGCAGAGCGAAAACAGCTGTACGCATTTTCCGGGTAACCTGCCGAATATGCT
GCGTGATCTGCGTGATGCATTTAGCCGTGTGAAAACATTTTTTCAGATGAAAGATCAGCTGGATAATCTGCTGCTGAAAGAAA
GCCTGCTGGAAGATTTTAAAGGTTATCTGGGTTGTCAGGCCCTGAGCGAAATGATTCAGTTTTATCTGGAAGAAGTTATGCCG
CAGGCAGAAAATCAGGATCCTGATATTAAAGCACATGTTAATAGCCTGGGTGAAAATCTGAAAACACTGCGTCTGCGTCTGCG
CCGTTGCCATCGCTTTCTGCCGTGTGAAAATggcggtggcagcggcggcAAAAGCAAAGCAGTGGAACAGGTTAAAAACGCAT
TTAATAAACTGCAGGAAAAAGGTATCTATAAAGCCATGTCAGAATTTGATATCTTTATCAATTATATCGAAGCATATATGACC
ATGAAAATCCGTAATCACCACCACCATCACCACTAACTCGAGcaccaccaccaccaccactgagatccggctgctaacaaagc
ccgaaaggaagctgagttggctgctgccaccgctgagcaataactagcataaccccttggggcctctaaacgggtcttgaggg
gttttttgctgaaaggaggaactatatccggattggcgaatggggcgaattgggtaccgggccccccctcgagccgatcatat
tcaataacccttaatataacttcgtataatgtatgctatacgaagttattagGTCTGAAGAGGAGTTTACGTCCCATATGGAG
TCCCGCccaatgcttaatcagtgaggcacctatctcagcgatctgtctatttcgttcatccatagttgcctgactccccgtcg
tgtagataactacgatacgggagggcttaccatctggccccagtgctgcaatgataccgcgagacccacgctcaccggctcca
gatttatcagcaataaaccagccagccggaagggccgagcgcagaagtggtcctgcaactttatccgcctccatccagtctat
taattgttgccgggaagctagagtaagtagttcgccagttaatagtttgcgcaacgttgttgccattgctacaggcatcgtgg
tgtcacgctcgtcgtttggtatggcttcattcagctccggttcccaacgatcaaggcgagttacatgatcccccatgttgtgc
aaaaaagcggttagctccttcggtcctccgatcgttgtcagaagtaagttggccgcagtgttatcactcatggttatggcagc
actgcataattctcttactgtcatgccatccgtaagatgcttttctgtgactggtgagtactcaaccaagtcattctgagaat
agtgtatgcggcgaccgagttgctcttgcccggcgtcaatacgggataataccgcgccacatagcagaactttaaaagtgctc
atcattggaaaacgttcttcggggcgaaaactctcaaggatcttaccgctgttgagatccagttcgatgtaacccactcgtgc
acccaactgatcttcagcatcttttactttcaccagcgtttctgggtgagcaaaaacaggaaggcaaaatgccgcaaaaaagg
gaataagggcgacacggaaatgttgaatactcatgcgaaacgatcctcatcctgtctcttgatcagatcCtgatcccctgcgc
catcagatccttggcggcaagaaagccatccagtttactttgcagggcttcccaaccttaccagagggcgccccagctggcaa
ttccgACGTCATTGCGTTGCTAGCCAAGCTTAGGATCCGGAACccttaatataacttcgtataatgtatgctatacgaagtta
ttaggtccctcgaagaggttcactagtcctcgaggtcgacggtatcgataagcttgatatcgaattcctgcagcccgggggat
ccactagttctagagcggccgccaccgcggtggagctccagcttttgttccctttagtgagggttaattgcgcgcttggcgta
atcatggtcatagctgtttcctgtgtgaaattgttatccgctcacaattccacacaacatacgagccggaagcataaagtgta
aagcctggggtgcctaatgagtgagctaactcacattaattgcgttgcgctcactgcccgctttccagtcgggaaacctgtcg
tgccagctgcattaatgaatcggccaacgcgcggggagaggcggtttgcgtattgggcgctcttccgcttcctcgctcactga
ctcgctgcgctcggtcgttcggctgcggcgagcggtatcagctcactcaaaggcggtaatacggttatccacagaatcagggg
ataacgcaggaaagaacatgtgagcaaaaggccagcaaaaggccaggaaccgtaaaaaggccgcgttgctggcgtttttccat
aggctccgcccccctgacgagcatcacaaaaatcgacgctcaagtcagaggtggcgaaacccgacaggactataaagatacca
ggcgtttccccctggaagctccctcgtgcgctctcctgttccgaccctgccgcttaccggatacctgtccgcctttctccctt
cgggaagcgtggcgctttctcatagctcacgctgtaggtatctcagttcggtgtaggtcgttcgctccaagctgggctgtgtg
cacgaaccccccgttcagcccgaccgctgcgccttatccggtaactatcgtcttgagtccaacccggtaagacacgacttatc
gccactggcagcagccactggtaacaggattagcagagcgaggtatgtaggcggtgctacagagttcttgaagtggtggccta
actacggctacactagaaggacagtatttggtatctgcgctctgctgaagccagttaccttcggaaaaagagttggtagctct
tgatccggcaaacaaaccaccgctggtagcggtggtttttttgtttgcaagcagcagattacgcgcagaaaaaaaggatctca
agaagatcctttgatcttttctacggggtctgacgctcagtggaacgaaaactcacgttaagggattttggtcatgagattat
caaaaaggatcttcacctagatccttttaaattaaaaatgaagttttaaatcaatctaaagtatatatgagtaaacttggtct
gacagttaccaatgcttaatcagtgaggcacctatctcagcgatctgtctatttcgttcatccatagttgcctgactccccgt
cgtgtagataactacgatacgggagggcttaccatctggccccagtgctgcaatgataccgcgagacccacgctcaccggctc
cagatttatcagcaataaaccagccagccggaagggccgagcgcagaagtggtcctgcaactttatccgcctccatccagtct
attaattgttgccgggaagctagagtaagtagttcgccagttaatagtttgcgcaacgttgttgccattgctacaggcatcgt
ggtgtcacgctcgtcgtttggtatggcttcattcagctccggttcccaacgatcaaggcgagttacatgatcccccatgttgt
gcaaaaaagcggttagctccttcggtcctccgatcgttgtcagaagtaagttggccgcagtgttatcactcatggttatggca
gcactgcataattctcttactgtcatgccatccgtaagatgcttttctgtgactggtgagtactcaaccaagtcattctgaga
atagtgtatgcggcgaccgagttgctcttgcccggcgtcaatacgggataataccgcgccacatagcagaactttaaaagtgc
tcatcattggaaaacgttcttcggggcgaaaactctcaaggatcttaccgctgttgagatccagttcgatgtaacccactcgt
gcacccaactgatcttcagcatcttttactttcaccagcgtttctgggtgagcaaaaacaggaaggcaaaatgccgcaaaaaa
gggaataagggcgacacggaaatgttgaatactcatactcttcctttttcaatattattgaagcatttatcagggttattgtc
tcatgagcggatacatatttgaatgtatttagaaaaataaacaaataggggttccgcgcacatttccccgaaaagtgccac
SEQ ID NO: 3: plasmid-pET22b-pelB-D5-huil10-Amp 
TGGCGAATGGGACGCGCCCTGTAGCGGCGCATTAAGCGCGGCGGGTGTGGTGGTTACGCGCAGCGTGACCGCTACACTTGCCA
GCGCCCTAGCGCCCGCTCCTTTCGCTTTCTTCCCTTCCTTTCTCGCCACGTTCGCCGGCTTTCCCCGTCAAGCTCTAAATCGG
 
GGGCTCCCTTTAGGGTTCCGATTTAGTGCTTTACGGCACCTCGACCCCAAAAAACTTGATTAGGGTGATGGTTCACGTAGTGG
GCCATCGCCCTGATAGACGGTTTTTCGCCCTTTGACGTTGGAGTCCACGTTCTTTAATAGTGGACTCTTGTTCCAAACTGGAA
CAACACTCAACCCTATCTCGGTCTATTCTTTTGATTTATAAGGGATTTTGCCGATTTCGGCCTATTGGTTAAAAAATGAGCTG
ATTTAACAAAAATTTAACGCGAATTTTAACAAAATATTAACGTTTACAATTTCAGGTGGCACTTTTCGGGGAAATGTGCGCGG
AACCCCTATTTGTTTATTTTTCTAAATACATTCAAATATGTATCCGCTCATGAGACAATAACCCTGATAAATGCTTCAATAAT
ATTGAAAAAGGAAGAGTATGAGTATTCAACATTTCCGTGTCGCCCTTATTCCCTTTTTTGCGGCATTTTGCCTTCCTGTTTTT
GCTCACCCAGAAACGCTGGTGAAAGTAAAAGATGCTGAAGATCAGTTGGGTGCACGAGTGGGTTACATCGAACTGGATCTCAA
CAGCGGTAAGATCCTTGAGAGTTTTCGCCCCGAAGAACGTTTTCCAATGATGAGCACTTTTAAAGTTCTGCTATGTGGCGCGG
TATTATCCCGTATTGACGCCGGGCAAGAGCAACTCGGTCGCCGCATACACTATTCTCAGAATGACTTGGTTGAGTACTCACCA
GTCACAGAAAAGCATCTTACGGATGGCATGACAGTAAGAGAATTATGCAGTGCTGCCATAACCATGAGTGATAACACTGCGGC
CAACTTACTTCTGACAACGATCGGAGGACCGAAGGAGCTAACCGCTTTTTTGCACAACATGGGGGATCATGTAACTCGCCTTG
ATCGTTGGGAACCGGAGCTGAATGAAGCCATACCAAACGACGAGCGTGACACCACGATGCCTGCAGCAATGGCAACAACGTTG
CGCAAACTATTAACTGGCGAACTACTTACTCTAGCTTCCCGGCAACAATTAATAGACTGGATGGAGGCGGATAAAGTTGCAGG
ACCACTTCTGCGCTCGGCCCTTCCGGCTGGCTGGTTTATTGCTGATAAATCTGGAGCCGGTGAGCGTGGGTCTCGCGGTATCA
TTGCAGCACTGGGGCCAGATGGTAAGCCCTCCCGTATCGTAGTTATCTACACGACGGGGAGTCAGGCAACTATGGATGAACGA
AATAGACAGATCGCTGAGATAGGTGCCTCACTGATTAAGCATTGGTAACTGTCAGACCAAGTTTACTCATATATACTTTAGAT
TGATTTAAAACTTCATTTTTAATTTAAAAGGATCTAGGTGAAGATCCTTTTTGATAATCTCATGACCAAAATCCCTTAACGTG
AGTTTTCGTTCCACTGAGCGTCAGACCCCGTAGAAAAGATCAAAGGATCTTCTTGAGATCCTTTTTTTCTGCGCGTAATCTGC
TGCTTGCAAACAAAAAAACCACCGCTACCAGCGGTGGTTTGTTTGCCGGATCAAGAGCTACCAACTCTTTTTCCGAAGGTAAC
TGGCTTCAGCAGAGCGCAGATACCAAATACTGTCCTTCTAGTGTAGCCGTAGTTAGGCCACCACTTCAAGAACTCTGTAGCAC
CGCCTACATACCTCGCTCTGCTAATCCTGTTACCAGTGGCTGCTGCCAGTGGCGATAAGTCGTGTCTTACCGGGTTGGACTCA
AGACGATAGTTACCGGATAAGGCGCAGCGGTCGGGCTGAACGGGGGGTTCGTGCACACAGCCCAGCTTGGAGCGAACGACCTA
CACCGAACTGAGATACCTACAGCGTGAGCTATGAGAAAGCGCCACGCTTCCCGAAGGGAGAAAGGCGGACAGGTATCCGGTAA
GCGGCAGGGTCGGAACAGGAGAGCGCACGAGGGAGCTTCCAGGGGGAAACGCCTGGTATCTTTATAGTCCTGTCGGGTTTCGC
CACCTCTGACTTGAGCGTCGATTTTTGTGATGCTCGTCAGGGGGGCGGAGCCTATGGAAAAACGCCAGCAACGCGGCCTTTTT
ACGGTTCCTGGCCTTTTGCTGGCCTTTTGCTCACATGTTCTTTCCTGCGTTATCCCCTGATTCTGTGGATAACCGTATTACCG
CCTTTGAGTGAGCTGATACCGCTCGCCGCAGCCGAACGACCGAGCGCAGCGAGTCAGTGAGCGAGGAAGCGGAAGAGCGCCTG
ATGCGGTATTTTCTCCTTACGCATCTGTGCGGTATTTCACACCGCATATATGGTGCACTCTCAGTACAATCTGCTCTGATGCC
GCATAGTTAAGCCAGTATACACTCCGCTATCGCTACGTGACTGGGTCATGGCTGCGCCCCGACACCCGCCAACACCCGCTGAC
GCGCCCTGACGGGCTTGTCTGCTCCCGGCATCCGCTTACAGACAAGCTGTGACCGTCTCCGGGAGCTGCATGTGTCAGAGGTT
TTCACCGTCATCACCGAAACGCGCGAGGCAGCTGCGGTAAAGCTCATCAGCGTGGTCGTGAAGCGATTCACAGATGTCTGCCT
GTTCATCCGCGTCCAGCTCGTTGAGTTTCTCCAGAAGCGTTAATGTCTGGCTTCTGATAAAGCGGGCCATGTTAAGGGCGGTT
TTTTCCTGTTTGGTCACTGATGCCTCCGTGTAAGGGGGATTTCTGTTCATGGGGGTAATGATACCGATGAAACGAGAGAGGAT
GCTCACGATACGGGTTACTGATGATGAACATGCCCGGTTACTGGAACGTTGTGAGGGTAAACAACTGGCGGTATGGATGCGGC
GGGACCAGAGAAAAATCACTCAGGGTCAATGCCAGCGCTTCGTTAATACAGATGTAGGTGTTCCACAGGGTAGCCAGCAGCAT
CCTGCGATGCAGATCCGGAACATAATGGTGCAGGGCGCTGACTTCCGCGTTTCCAGACTTTACGAAACACGGAAACCGAAGAC
CATTCATGTTGTTGCTCAGGTCGCAGACGTTTTGCAGCAGCAGTCGCTTCACGTTCGCTCGCGTATCGGTGATTCATTCTGCT
AACCAGTAAGGCAACCCCGCCAGCCTAGCCGGGTCCTCAACGACAGGAGCACGATCATGCGCACCCGTGGGGCCGCCATGCCG
GCGATAATGGCCTGCTTCTCGCCGAAACGTTTGGTGGCGGGACCAGTGACGAAGGCTTGAGCGAGGGCGTGCAAGATTCCGAA
TACCGCAAGCGACAGGCCGATCATCGTCGCGCTCCAGCGAAAGCGGTCCTCGCCGAAAATGACCCAGAGCGCTGCCGGCACCT
GTCCTACGAGTTGCATGATAAAGAAGACAGTCATAAGTGCGGCGACGATAGTCATGCCCCGCGCCCACCGGAAGGAGCTGACT
GGGTTGAAGGCTCTCAAGGGCATCGGTCGAGATCCCGGTGCCTAATGAGTGAGCTAACTTACATTAATTGCGTTGCGCTCACT
GCCCGCTTTCCAGTCGGGAAACCTGTCGTGCCAGCTGCATTAATGAATCGGCCAACGCGCGGGGAGAGGCGGTTTGCGTATTG
GGCGCCAGGGTGGTTTTTCTTTTCACCAGTGAGACGGGCAACAGCTGATTGCCCTTCACCGCCTGGCCCTGAGAGAGTTGCAG
CAAGCGGTCCACGCTGGTTTGCCCCAGCAGGCGAAAATCCTGTTTGATGGTGGTTAACGGCGGGATATAACATGAGCTGTCTT
CGGTATCGTCGTATCCCACTACCGAGATATCCGCACCAACGCGCAGCCCGGACTCGGTAATGGCGCGCATTGCGCCCAGCGCC
ATCTGATCGTTGGCAACCAGCATCGCAGTGGGAACGATGCCCTCATTCAGCATTTGCATGGTTTGTTGAAAACCGGACATGGC
ACTCCAGTCGCCTTCCCGTTCCGCTATCGGCTGAATTTGATTGCGAGTGAGATATTTATGCCAGCCAGCCAGACGCAGACGCG
CCGAGACAGAACTTAATGGGCCCGCTAACAGCGCGATTTGCTGGTGACCCAATGCGACCAGATGCTCCACGCCCAGTCGCGTA
CCGTCTTCATGGGAGAAAATAATACTGTTGATGGGTGTCTGGTCAGAGACATCAAGAAATAACGCCGGAACATTAGTGCAGGC
AGCTTCCACAGCAATGGCATCCTGGTCATCCAGCGGATAGTTAATGATCAGCCCACTGACGCGTTGCGCGAGAAGATTGTGCA
CCGCCGCTTTACAGGCTTCGACGCCGCTTCGTTCTACCATCGACACCACCACGCTGGCACCCAGTTGATCGGCGCGAGATTTA
ATCGCCGCGACAATTTGCGACGGCGCGTGCAGGGCCAGACTGGAGGTGGCAACGCCAATCAGCAACGACTGTTTGCCCGCCAG
TTGTTGTGCCACGCGGTTGGGAATGTAATTCAGCTCCGCCATCGCCGCTTCCACTTTTTCCCGCGTTTTCGCAGAAACGTGGC
TGGCCTGGTTCACCACGCGGGAAACGGTCTGATAAGAGACACCGGCATACTCTGCGACATCGTATAACGTTACTGGTTTCACA
TTCACCACCCTGAATTGACTCTCTTCCGGGCGCTATCATGCCATACCGCGAAAGGTTTTGCGCCATTCGATGGTGTCCGGGAT
CTCGACGCTCTCCCTTATGCGACTCCTGCATTAGGAAGCAGCCCAGTAGTAGGTTGAGGCCGTTGAGCACCGCCGCCGCAAGG
AATGGTGCATGCAAGGAGATGGCGCCCAACAGTCCCCCGGCCACGGGGCCTGCCACCATACCCACGCCGAAACAAGCGCTCAT
GAGCCCGAAGTGGCGAGCCCGATCTTCCCCATCGGTGATGTCGGCGATATAGGCGCCAGCAACCGCACCTGTGGCGCCGGTGA
TGCCGGCCACGATGCGTCCGGCGTAGAGGATCGAGATCTCGATCCCGCGAAATTAATACGACTCACTATAGGGGAATTGTGAG
CGGATAACAATTCCCCTCTAGAAATAATTTTGTTTAACTTTAAGAAGGAGATATACATATGAAATACCTGCTGCCGACCGCTG
CTGCTGGTCTGCTGCTCCTCGCTGCCCAGCCGGCGATGGCCGATGATGATGATGATAGCCCGGGTCAGGGTACGCAGAGCGAA
AACAGCTGTACGCATTTTCCGGGTAACCTGCCGAATATGCTGCGTGATCTGCGTGATGCATTTAGCCGTGTGAAAACATTTTT
TCAGATGAAAGATCAGCTGGATAATCTGCTGCTGAAAGAAAGCCTGCTGGAAGATTTTAAAGGTTATCTGGGTTGTCAGGCCC
TGAGCGAAATGATTCAGTTTTATCTGGAAGAAGTTATGCCGCAGGCAGAAAATCAGGATCCTGATATTAAAGCACATGTTAAT
AGCCTGGGTGAAAATCTGAAAACACTGCGTCTGCGTCTGCGCCGTTGCCATCGCTTTCTGCCGTGTGAAAATAAAAGCAAAGC
AGTGGAACAGGTTAAAAACGCATTTAATAAACTGCAGGAAAAAGGTATCTATAAAGCCATGTCAGAATTTGATATCTTTATCA
ATTATATCGAAGCATATATGACCATGAAAATCCGTAATCACCACCACCACCACCACTGAGATCCGGCTGCTAACAAAGCCCGA
AAGGAAGCTGAGTTGGCTGCTGCCACCGCTGAGCAATAACTAGCATAACCCCTTGGGGCCTCTAAACGGGTCTTGAGGGGTTT
TTTGCTGAAAGGAGGAACTATATCCGGAT
SEQ ID NO: 4: plasmid-pET22b-pelB-huil10-Amp 
TGGCGAATGGGACGCGCCCTGTAGCGGCGCATTAAGCGCGGCGGGTGTGGTGGTTACGCGCAGCGTGACCGCTACACTTGCCA
GCGCCCTAGCGCCCGCTCCTTTCGCTTTCTTCCCTTCCTTTCTCGCCACGTTCGCCGGCTTTCCCCGTCAAGCTCTAAATCGG
GGGCTCCCTTTAGGGTTCCGATTTAGTGCTTTACGGCACCTCGACCCCAAAAAACTTGATTAGGGTGATGGTTCACGTAGTGG
GCCATCGCCCTGATAGACGGTTTTTCGCCCTTTGACGTTGGAGTCCACGTTCTTTAATAGTGGACTCTTGTTCCAAACTGGAA
CAACACTCAACCCTATCTCGGTCTATTCTTTTGATTTATAAGGGATTTTGCCGATTTCGGCCTATTGGTTAAAAAATGAGCTG
ATTTAACAAAAATTTAACGCGAATTTTAACAAAATATTAACGTTTACAATTTCAGGTGGCACTTTTCGGGGAAATGTGCGCGG
AACCCCTATTTGTTTATTTTTCTAAATACATTCAAATATGTATCCGCTCATGAGACAATAACCCTGATAAATGCTTCAATAAT
ATTGAAAAAGGAAGAGTATGAGTATTCAACATTTCCGTGTCGCCCTTATTCCCTTTTTTGCGGCATTTTGCCTTCCTGTTTTT
GCTCACCCAGAAACGCTGGTGAAAGTAAAAGATGCTGAAGATCAGTTGGGTGCACGAGTGGGTTACATCGAACTGGATCTCAA
CAGCGGTAAGATCCTTGAGAGTTTTCGCCCCGAAGAACGTTTTCCAATGATGAGCACTTTTAAAGTTCTGCTATGTGGCGCGG
TATTATCCCGTATTGACGCCGGGCAAGAGCAACTCGGTCGCCGCATACACTATTCTCAGAATGACTTGGTTGAGTACTCACCA
GTCACAGAAAAGCATCTTACGGATGGCATGACAGTAAGAGAATTATGCAGTGCTGCCATAACCATGAGTGATAACACTGCGGC
CAACTTACTTCTGACAACGATCGGAGGACCGAAGGAGCTAACCGCTTTTTTGCACAACATGGGGGATCATGTAACTCGCCTTG
ATCGTTGGGAACCGGAGCTGAATGAAGCCATACCAAACGACGAGCGTGACACCACGATGCCTGCAGCAATGGCAACAACGTTG
CGCAAACTATTAACTGGCGAACTACTTACTCTAGCTTCCCGGCAACAATTAATAGACTGGATGGAGGCGGATAAAGTTGCAGG
ACCACTTCTGCGCTCGGCCCTTCCGGCTGGCTGGTTTATTGCTGATAAATCTGGAGCCGGTGAGCGTGGGTCTCGCGGTATCA
TTGCAGCACTGGGGCCAGATGGTAAGCCCTCCCGTATCGTAGTTATCTACACGACGGGGAGTCAGGCAACTATGGATGAACGA
AATAGACAGATCGCTGAGATAGGTGCCTCACTGATTAAGCATTGGTAACTGTCAGACCAAGTTTACTCATATATACTTTAGAT
TGATTTAAAACTTCATTTTTAATTTAAAAGGATCTAGGTGAAGATCCTTTTTGATAATCTCATGACCAAAATCCCTTAACGTG
AGTTTTCGTTCCACTGAGCGTCAGACCCCGTAGAAAAGATCAAAGGATCTTCTTGAGATCCTTTTTTTCTGCGCGTAATCTGC
TGCTTGCAAACAAAAAAACCACCGCTACCAGCGGTGGTTTGTTTGCCGGATCAAGAGCTACCAACTCTTTTTCCGAAGGTAAC
TGGCTTCAGCAGAGCGCAGATACCAAATACTGTCCTTCTAGTGTAGCCGTAGTTAGGCCACCACTTCAAGAACTCTGTAGCAC
CGCCTACATACCTCGCTCTGCTAATCCTGTTACCAGTGGCTGCTGCCAGTGGCGATAAGTCGTGTCTTACCGGGTTGGACTCA
AGACGATAGTTACCGGATAAGGCGCAGCGGTCGGGCTGAACGGGGGGTTCGTGCACACAGCCCAGCTTGGAGCGAACGACCTA
CACCGAACTGAGATACCTACAGCGTGAGCTATGAGAAAGCGCCACGCTTCCCGAAGGGAGAAAGGCGGACAGGTATCCGGTAA
GCGGCAGGGTCGGAACAGGAGAGCGCACGAGGGAGCTTCCAGGGGGAAACGCCTGGTATCTTTATAGTCCTGTCGGGTTTCGC
CACCTCTGACTTGAGCGTCGATTTTTGTGATGCTCGTCAGGGGGGCGGAGCCTATGGAAAAACGCCAGCAACGCGGCCTTTTT
ACGGTTCCTGGCCTTTTGCTGGCCTTTTGCTCACATGTTCTTTCCTGCGTTATCCCCTGATTCTGTGGATAACCGTATTACCG
CCTTTGAGTGAGCTGATACCGCTCGCCGCAGCCGAACGACCGAGCGCAGCGAGTCAGTGAGCGAGGAAGCGGAAGAGCGCCTG
ATGCGGTATTTTCTCCTTACGCATCTGTGCGGTATTTCACACCGCATATATGGTGCACTCTCAGTACAATCTGCTCTGATGCC
GCATAGTTAAGCCAGTATACACTCCGCTATCGCTACGTGACTGGGTCATGGCTGCGCCCCGACACCCGCCAACACCCGCTGAC
GCGCCCTGACGGGCTTGTCTGCTCCCGGCATCCGCTTACAGACAAGCTGTGACCGTCTCCGGGAGCTGCATGTGTCAGAGGTT
TTCACCGTCATCACCGAAACGCGCGAGGCAGCTGCGGTAAAGCTCATCAGCGTGGTCGTGAAGCGATTCACAGATGTCTGCCT
GTTCATCCGCGTCCAGCTCGTTGAGTTTCTCCAGAAGCGTTAATGTCTGGCTTCTGATAAAGCGGGCCATGTTAAGGGCGGTT
TTTTCCTGTTTGGTCACTGATGCCTCCGTGTAAGGGGGATTTCTGTTCATGGGGGTAATGATACCGATGAAACGAGAGAGGAT
GCTCACGATACGGGTTACTGATGATGAACATGCCCGGTTACTGGAACGTTGTGAGGGTAAACAACTGGCGGTATGGATGCGGC
GGGACCAGAGAAAAATCACTCAGGGTCAATGCCAGCGCTTCGTTAATACAGATGTAGGTGTTCCACAGGGTAGCCAGCAGCAT
CCTGCGATGCAGATCCGGAACATAATGGTGCAGGGCGCTGACTTCCGCGTTTCCAGACTTTACGAAACACGGAAACCGAAGAC
CATTCATGTTGTTGCTCAGGTCGCAGACGTTTTGCAGCAGCAGTCGCTTCACGTTCGCTCGCGTATCGGTGATTCATTCTGCT
AACCAGTAAGGCAACCCCGCCAGCCTAGCCGGGTCCTCAACGACAGGAGCACGATCATGCGCACCCGTGGGGCCGCCATGCCG
GCGATAATGGCCTGCTTCTCGCCGAAACGTTTGGTGGCGGGACCAGTGACGAAGGCTTGAGCGAGGGCGTGCAAGATTCCGAA
TACCGCAAGCGACAGGCCGATCATCGTCGCGCTCCAGCGAAAGCGGTCCTCGCCGAAAATGACCCAGAGCGCTGCCGGCACCT
GTCCTACGAGTTGCATGATAAAGAAGACAGTCATAAGTGCGGCGACGATAGTCATGCCCCGCGCCCACCGGAAGGAGCTGACT
GGGTTGAAGGCTCTCAAGGGCATCGGTCGAGATCCCGGTGCCTAATGAGTGAGCTAACTTACATTAATTGCGTTGCGCTCACT
GCCCGCTTTCCAGTCGGGAAACCTGTCGTGCCAGCTGCATTAATGAATCGGCCAACGCGCGGGGAGAGGCGGTTTGCGTATTG
GGCGCCAGGGTGGTTTTTCTTTTCACCAGTGAGACGGGCAACAGCTGATTGCCCTTCACCGCCTGGCCCTGAGAGAGTTGCAG
CAAGCGGTCCACGCTGGTTTGCCCCAGCAGGCGAAAATCCTGTTTGATGGTGGTTAACGGCGGGATATAACATGAGCTGTCTT
CGGTATCGTCGTATCCCACTACCGAGATATCCGCACCAACGCGCAGCCCGGACTCGGTAATGGCGCGCATTGCGCCCAGCGCC
ATCTGATCGTTGGCAACCAGCATCGCAGTGGGAACGATGCCCTCATTCAGCATTTGCATGGTTTGTTGAAAACCGGACATGGC
ACTCCAGTCGCCTTCCCGTTCCGCTATCGGCTGAATTTGATTGCGAGTGAGATATTTATGCCAGCCAGCCAGACGCAGACGCG
CCGAGACAGAACTTAATGGGCCCGCTAACAGCGCGATTTGCTGGTGACCCAATGCGACCAGATGCTCCACGCCCAGTCGCGTA
CCGTCTTCATGGGAGAAAATAATACTGTTGATGGGTGTCTGGTCAGAGACATCAAGAAATAACGCCGGAACATTAGTGCAGGC
AGCTTCCACAGCAATGGCATCCTGGTCATCCAGCGGATAGTTAATGATCAGCCCACTGACGCGTTGCGCGAGAAGATTGTGCA
CCGCCGCTTTACAGGCTTCGACGCCGCTTCGTTCTACCATCGACACCACCACGCTGGCACCCAGTTGATCGGCGCGAGATTTA
ATCGCCGCGACAATTTGCGACGGCGCGTGCAGGGCCAGACTGGAGGTGGCAACGCCAATCAGCAACGACTGTTTGCCCGCCAG
TTGTTGTGCCACGCGGTTGGGAATGTAATTCAGCTCCGCCATCGCCGCTTCCACTTTTTCCCGCGTTTTCGCAGAAACGTGGC
TGGCCTGGTTCACCACGCGGGAAACGGTCTGATAAGAGACACCGGCATACTCTGCGACATCGTATAACGTTACTGGTTTCACA
TTCACCACCCTGAATTGACTCTCTTCCGGGCGCTATCATGCCATACCGCGAAAGGTTTTGCGCCATTCGATGGTGTCCGGGAT
CTCGACGCTCTCCCTTATGCGACTCCTGCATTAGGAAGCAGCCCAGTAGTAGGTTGAGGCCGTTGAGCACCGCCGCCGCAAGG
AATGGTGCATGCAAGGAGATGGCGCCCAACAGTCCCCCGGCCACGGGGCCTGCCACCATACCCACGCCGAAACAAGCGCTCAT
GAGCCCGAAGTGGCGAGCCCGATCTTCCCCATCGGTGATGTCGGCGATATAGGCGCCAGCAACCGCACCTGTGGCGCCGGTGA
TGCCGGCCACGATGCGTCCGGCGTAGAGGATCGAGATCTCGATCCCGCGAAATTAATACGACTCACTATAGGGGAATTGTGAG
CGGATAACAATTCCCCTCTAGAAATAATTTTGTTTAACTTTAAGAAGGAGATATACATATGAAATACCTGCTGCCGACCGCTG
CTGCTGGTCTGCTGCTCCTCGCTGCCCAGCCGGCGATGGCCAGCCCGGGTCAGGGTACGCAGAGCGAAAACAGCTGTACGCAT
TTTCCGGGTAACCTGCCGAATATGCTGCGTGATCTGCGTGATGCATTTAGCCGTGTGAAAACATTTTTTCAGATGAAAGATCA
GCTGGATAATCTGCTGCTGAAAGAAAGCCTGCTGGAAGATTTTAAAGGTTATCTGGGTTGTCAGGCCCTGAGCGAAATGATTC
AGTTTTATCTGGAAGAAGTTATGCCGCAGGCAGAAAATCAGGATCCTGATATTAAAGCACATGTTAATAGCCTGGGTGAAAAT
CTGAAAACACTGCGTCTGCGTCTGCGCCGTTGCCATCGCTTTCTGCCGTGTGAAAATAAAAGCAAAGCAGTGGAACAGGTTAA
AAACGCATTTAATAAACTGCAGGAAAAAGGTATCTATAAAGCCATGTCAGAATTTGATATCTTTATCAATTATATCGAAGCAT
ATATGACCATGAAAATCCGTAATCACCACCACCACCACCACTGAGATCCGGCTGCTAACAAAGCCCGAAAGGAAGCTGAGTTG
 
GCTGCTGCCACCGCTGAGCAATAACTAGCATAACCCCTTGGGGCCTCTAAACGGGTCTTGAGGGGTTTTTTGCTGAAAGGAGG
AACTATATCCGGAT
SEQ ID NO: 5: pelB-D5-huil10 
ATGAAATACCTGCTGCCGACCGCTGCTGCTGGTCTGCTGCTCCTCGCTGCCCAGCCGGCGATGGCCGATGATGATGATGATAG
CCCGGGTCAGGGTACGCAGAGCGAAAACAGCTGTACGCATTTTCCGGGTAACCTGCCGAATATGCTGCGTGATCTGCGTGATG
CATTTAGCCGTGTGAAAACATTTTTTCAGATGAAAGATCAGCTGGATAATCTGCTGCTGAAAGAAAGCCTGCTGGAAGATTTT
AAAGGTTATCTGGGTTGTCAGGCCCTGAGCGAAATGATTCAGTTTTATCTGGAAGAAGTTATGCCGCAGGCAGAAAATCAGGA
TCCTGATATTAAAGCACATGTTAATAGCCTGGGTGAAAATCTGAAAACACTGCGTCTGCGTCTGCGCCGTTGCCATCGCTTTC
TGCCGTGTGAAAATAAAAGCAAAGCAGTGGAACAGGTTAAAAACGCATTTAATAAACTGCAGGAAAAAGGTATCTATAAAGCC
ATGTCAGAATTTGATATCTTTATCAATTATATCGAAGCATATATGACCATGAAAATCCGTAATCACCACCACCACCACCACTG
A
SEQ ID NO: 6: pSC101-ptac-sfGFP-KanR 
gtcttgaggggttttttgctgaaaggaggaactatatccggattggcgaatggggcgaattgggtaccgggccccccctcgag
ccgatcatattcaataacccttaatataacttcgtataatgtatgctatacgaagttattagGTCTGAAGAGGAGTTTACGTC
CCATATGGAGTCCCGCttaatcagcttgcttacgcaggaatgctgggatatccagataatccggctctttcgcagtttgcggc
gcattgtcattcacgactttagcaaccggcttctgctcctgggtcagcggagccatcccgtgctgctggtagcgatccatcac
tggctgctgaacctgcttattagtcaccagagtgatttcaggacgtttgtccatgccgatacctgtcgcaacaacagttacgc
gcagctcgtcattcatatccgggtcaagagaagtaccgataaccacggtcgcgttgtcggaagcaaatgcacggatggtgtta
cctaccgtttcgaactcatccagacgcaggtcgaagcccgctgtgatgtttaccagtacgccgcgtgcgccagacaggtcgat
atcttccagcagcggagaagagatagccatttcagcagcttcttccgcacgatcttcaccgctcgccacaccagaacccatca
ttgcgtagcccatttcagacattacggtgcgtacgtctgcaaagtccacgttcatcaaacccggacgagtaatcagttcggcg
ataccttgcacagcgccttttagtacatcgttcgctgcgccaaacgcatccagcagggagataccacggcccagaactttcag
cagtttgtcgttcgggatagtgatcagagagtccacatgcttggacagttcagtgatcccctgctccgcgaatgccatacgct
tcttgccttcaaagttgaaaggcttagtgacgacagcaacggtcaggatacccaaatcttttgccacttcagcgacgactggt
gctgcacctgtaccggtaccacccatacccgcagcaataaagaccatgtctgcaccttccagcgccgcacgcagtgcatc
gcgatcctcatcagccgcattgcggccaacttctggattagcgccagcgcccagtcctttggtgataccgctaccgatttgaa
tcgtctgtccaaccgctgttttacgcagcgcttgtgcatcggtatttaccgcgaagaattcaacaccttcaatgcgctcacgc
accatgtgttcaacagcattaccgccgccgccgacgccgatgactttaatcaccgcgtcattggtaagttccattggttc
aaacatAGATCATCGACCCCTCATTTTATTGCCAGATCAGAAGAACTcgtcaagaaggcgatagaaggcgatgcgctgcgaat
cgggagcggcgataccgtaaagcacgaggaagcggtcagcccattcgccgccaagctcttcagcaatatcacgggtagccaac
gctatgtcctgatagcggtccgccacacccagccggccacagtcgatgaatccagaaaagcggccattttccaccatgatatt
cggcaagcaggcatcgccatgggtcacgacgagatcctcgccgtcgggcatgcgcgccttgagcctggcgaacagttcggcCg
gcgcgagcccctgatgctcttcgtccagatcatcctgatcgacaagaccggcttccatccgagtacgtgctcgctcgatgcga
tgtttcgcttggtggtcgaatgggcaggtagccggatcaagcgtatgcagccgccgcattgcatcagccatgatggatacttt
ctcggcaggagcaaggtgagatgacaggagatcctgccccggcacttcgcccaatagcagccagtcccttcccgcttcagtga
caacgtcgagcacagctgcgcaaggaacgcccgtcgtggccagccacgatagccgcgctgcctcgtcctgcagttcattcagg
gcaccggacaggtcggtcttgacaaaaagaaccgggcgcccctgcgctgacagccggaacacggcggcatcagagcagccgat
tgtctgttgtgcccagtcatagccgaatagcctctccacccaagcggccggagaacctgcgtgcaatccatcttgttcaatca
tgcgaaacgatcctcatcctgtctcttgatcagatcCtgatcccctgcgccatcagatccttggcggcaagaaagccatccag
tttactttgcagggcttcccaaccttaccagagggcgccccagctggcaattccactagAGGACTTCGATTAGGCCAAATAGG
CCGTTCAGATCCTTCCGTATTTAGCCAGTATGTTCTCTAGTGTGGTTCGTTGTTTTTGCGTGAGCCATGAGAACGAACCATTG
AGATCATGCTTACTTTGCATGTCACTCAAAAATTTTGCCTCAAAACTGGTGAGCTGAATTTTTGCAGTTAAAGCATCGTGTAG
TGTTTTTCTTAGTCCGTTACGTAGGTAGGAATCTGATGTAATGGTTGTTGGTATTTTGTCACCATTCATTTTTATCTGGTTGT
TCTCAAGTTCGGTTACGAGATCCATTTGTCTATCTAGTTCAACTTGGAAAATCAACGTATCAGTCGGGCGGCCTCGCTTATCA
ACCACCAATTTCATATTGCTGTAAGTGTTTAAATCTTTACTTATTGGTTTCAAAACCCATTGGTTAAGCCTTTTAAACTCATG
GTAGTTATTTTCAAGCATTAACATGAACTTAAATTCATCAAGGCTAATCTCTATATTTGCCTTGTGAGTTTTCTTTTGTGTTA
GTTCTTTTAATAACCACTCATAAATCCTCATAGAGTATTTGTTTTCAAAAGACTTAACATGTTCCAGATTATATTTTATGAAT
TTTTTTAACTGGAAAAGATAAGGCAATATCTCTTCACTAAAAACTAATTCTAATTTTTCGCTTGAGAACTTGGCATAGTTTGT
CCACTGGAAAATCTCAAAGCCTTTAACCAAAGGATTCCTGATTTCCACAGTTCTCGTCATCAGCTCTCTGGTTGCTTTAGCTA
ATACACCATAAGCATTTTCCCTACTGATGTTCATCATCTGAGCGTATTGGTTATAAGTGAACGATACCGTCCGTTCTTTCCTT
GTAGGGTTTTCAATCGTGGGGTTGAGTAGTGCCACACAGCATAAAATTAGCTTGGTTTCATGCTCCGTTAAGTCATAGCGACT
AATCGCTAGTTCATTTGCTTTGAAAACAACTAATTCAGACATACATCTCAATTGGTCTAGGTGATTTTAATCACTATACCAAT
TGAGATGGGCTAGTCAATGATAATTACTAGTCCTTTTCCTTTGAGTTGTGGGTATCTGTAAATTCTGCTAGACCTTTGCTGGA
AAACTTGTAAATTCTGCTAGACCCTCTGTAAATTCCGCTAGACCTTTGTGTGTTTTTTTTGTTTATATTCAAGTGGTTATAAT
TTATAGAATAAAGAAAGAATAAAAAAAGATAAAAAGAATAGATCCCAGCCCTGTGTATAACTCACTACTTTAGTCAGTTCCGC
AGTATTACAAAAGGATGTCGCAAACGCTGTTTGCTCCTCTACAAAACAGACCTTAAAACCCTAAAGGCTTAAGTAGCACCCTC
GCAAGCTCGGGCAAATCGCTGAATATTCCTTTTGTCTCCGACCATCAGGCACCTGAGTCGCTGTCTTTTTCGTGACATTCAGT
TCGCTGCGCTCACGGCTCTGGCAGTGAATGGGGGTAAATGGCACTACAGGCGCCTTTTATGGATTCATGCAAGGAAACTACCC
ATAATACAAGAAAAGCCCGTCACGGGCTTCTCAGGGCGTTTTATGGCGGGTCTGCTATGTGGTGCTATCTGACTTTTTGCTGT
TCAGCAGTTCCTGCCCTCTGATTTTCCAGTCTGACCACTTCGGATTATCCCGTGACAGGTCATTCAGACTGGCTAATGCACCC
AGTAAGGCAGCGGTATCATCAACAGGCTTACCCGTCTTACTGTCAAGAGGACATCCGGTCAtgagcgcgcgTTGACAATTAAT
CATCGGCTCGTATAATGcctctagaaataatTCACACAGGAAAGtataCAATGAGCAAAGGAGAAGAACTTTTCACtggagtt
gtcccaattcttgttgaattagatggtgatgttaatgggcacaaattttctgtccgtggagagggtgaaggtgatgctacaaa
cggaaaactcacccttaaatttatttgcactactggaaaactacctgttccgtggccaacacttgtcactactctgacctatg
gtgttcaatgcttttcccgttatccggatcacatgaaacggcatgactttttcaagagtgccatgcccgaaggttatgtacag
gaacgcactatatctttcaaagatgacgggacctacaagacgcgtgctgaagtcaagtttgaaggtgatacccttgttaatcg
tatcgagttaaagggtattgattttaaagaagatggaaacattcttggacacaaactcgagtacaactttaactcacacaatg
tatacatcacggcagacaaacaaaagaatggaatcaaagctaacttcaaaattcgccacaacgttgaagatggttccgttcaa
ctagcagaccattatcaacaaaatactccaattggcgatggccctgtccttttaccagacaaccattacctgtcgacacaatc
 
tgtcctttcgaaagatcccaacgaaaagcgtgaccacatggtccttcttgagtttgtaactgctgctgggattacacatGGCA
TGGATCACCTCTACAAATAAgatccggctgctaacaaagcccgaaaggaagctgagttggctgctgccaccgctgagcaataa
ctagcataaccccttggggcctctaaacgg
SEQ ID NO: 7: plasmid-pSC101-ptac-pelB-D5-huil10-KanR 
gtcttgaggggttttttgctgaaaggaggaactatatccggattggcgaatggggcgaattgggtaccgggccccccctcgag
ccgatcatattcaataacccttaatataacttcgtataatgtatgctatacgaagttattagGTCTGAAGAGGAGTTTACGTC
CCATATGGAGTCCCGCttaatcagcttgcttacgcaggaatgctgggatatccagataatccggctctttcgcagtttgcggc
gcattgtcattcacgactttagcaaccggcttctgctcctgggtcagcggagccatcccgtgctgctggtagcgatccatcac
tggctgctgaacctgcttattagtcaccagagtgatttcaggacgtttgtccatgccgatacctgtcgcaacaacagttacgc
gcagctcgtcattcatatccgggtcaagagaagtaccgataaccacggtcgcgttgtcggaagcaaatgcacggatggtgtta
cctaccgtttcgaactcatccagacgcaggtcgaagcccgctgtgatgtttaccagtacgccgcgtgcgccagacaggtcgat
atcttccagcagcggagaagagatagccatttcagcagcttcttccgcacgatcttcaccgctcgccacaccagaacccatca
ttgcgtagcccatttcagacattacggtgcgtacgtctgcaaagtccacgttcatcaaacccggacgagtaatcagttcggcg
ataccttgcacagcgccttttagtacatcgttcgctgcgccaaacgcatccagcagggagataccacggcccagaactttcag
cagtttgtcgttcgggatagtgatcagagagtccacatgcttggacagttcagtgatcccctgctccgcgaatgccatacgct
tcttgccttcaaagttgaaaggcttagtgacgacagcaacggtcaggatacccaaatcttttgccacttcagcgacgactggt
gctgcacctgtaccggtaccacccatacccgcagcaataaagaccatgtctgcaccttccagcgccgcacgcagtgcatc
gcgatcctcatcagccgcattgcggccaacttctggattagcgccagcgcccagtcctttggtgataccgctaccgatttgaa
tcgtctgtccaaccgctgttttacgcagcgcttgtgcatcggtatttaccgcgaagaattcaacaccttcaatgcgctcacgc
accatgtgttcaacagcattaccgccgccgccgacgccgatgactttaatcaccgcgtcattggtaagttccattggttc
aaacatAGATCATCGACCCCTCATTTTATTGCCAGATCAGAAGAACTcgtcaagaaggcgatagaaggcgatgcgctgcgaat
cgggagcggcgataccgtaaagcacgaggaagcggtcagcccattcgccgccaagctcttcagcaatatcacgggtagccaac
gctatgtcctgatagcggtccgccacacccagccggccacagtcgatgaatccagaaaagcggccattttccaccatgatatt
cggcaagcaggcatcgccatgggtcacgacgagatcctcgccgtcgggcatgcgcgccttgagcctggcgaacagttcggcCg
gcgcgagcccctgatgctcttcgtccagatcatcctgatcgacaagaccggcttccatccgagtacgtgctcgctcgatgcga
tgtttcgcttggtggtcgaatgggcaggtagccggatcaagcgtatgcagccgccgcattgcatcagccatgatggatacttt
ctcggcaggagcaaggtgagatgacaggagatcctgccccggcacttcgcccaatagcagccagtcccttcccgcttcagtga
caacgtcgagcacagctgcgcaaggaacgcccgtcgtggccagccacgatagccgcgctgcctcgtcctgcagttcattcagg
gcaccggacaggtcggtcttgacaaaaagaaccgggcgcccctgcgctgacagccggaacacggcggcatcagagcagccgat
tgtctgttgtgcccagtcatagccgaatagcctctccacccaagcggccggagaacctgcgtgcaatccatcttgttcaatca
tgcgaaacgatcctcatcctgtctcttgatcagatcCtgatcccctgcgccatcagatccttggcggcaagaaagccatccag
tttactttgcagggcttcccaaccttaccagagggcgccccagctggcaattccactagAGGACTTCGATTAGGCCAAATAGG
CCGTTCAGATCCTTCCGTATTTAGCCAGTATGTTCTCTAGTGTGGTTCGTTGTTTTTGCGTGAGCCATGAGAACGAACCATTG
AGATCATGCTTACTTTGCATGTCACTCAAAAATTTTGCCTCAAAACTGGTGAGCTGAATTTTTGCAGTTAAAGCATCGTGTAG
TGTTTTTCTTAGTCCGTTACGTAGGTAGGAATCTGATGTAATGGTTGTTGGTATTTTGTCACCATTCATTTTTATCTGGTTGT
TCTCAAGTTCGGTTACGAGATCCATTTGTCTATCTAGTTCAACTTGGAAAATCAACGTATCAGTCGGGCGGCCTCGCTTATCA
ACCACCAATTTCATATTGCTGTAAGTGTTTAAATCTTTACTTATTGGTTTCAAAACCCATTGGTTAAGCCTTTTAAACTCATG
GTAGTTATTTTCAAGCATTAACATGAACTTAAATTCATCAAGGCTAATCTCTATATTTGCCTTGTGAGTTTTCTTTTGTGTTA
GTTCTTTTAATAACCACTCATAAATCCTCATAGAGTATTTGTTTTCAAAAGACTTAACATGTTCCAGATTATATTTTATGAAT
TTTTTTAACTGGAAAAGATAAGGCAATATCTCTTCACTAAAAACTAATTCTAATTTTTCGCTTGAGAACTTGGCATAGTTTGT
CCACTGGAAAATCTCAAAGCCTTTAACCAAAGGATTCCTGATTTCCACAGTTCTCGTCATCAGCTCTCTGGTTGCTTTAGCTA
ATACACCATAAGCATTTTCCCTACTGATGTTCATCATCTGAGCGTATTGGTTATAAGTGAACGATACCGTCCGTTCTTTCCTT
GTAGGGTTTTCAATCGTGGGGTTGAGTAGTGCCACACAGCATAAAATTAGCTTGGTTTCATGCTCCGTTAAGTCATAGCGACT
AATCGCTAGTTCATTTGCTTTGAAAACAACTAATTCAGACATACATCTCAATTGGTCTAGGTGATTTTAATCACTATACCAAT
TGAGATGGGCTAGTCAATGATAATTACTAGTCCTTTTCCTTTGAGTTGTGGGTATCTGTAAATTCTGCTAGACCTTTGCTGGA
AAACTTGTAAATTCTGCTAGACCCTCTGTAAATTCCGCTAGACCTTTGTGTGTTTTTTTTGTTTATATTCAAGTGGTTATAAT
TTATAGAATAAAGAAAGAATAAAAAAAGATAAAAAGAATAGATCCCAGCCCTGTGTATAACTCACTACTTTAGTCAGTTCCGC
AGTATTACAAAAGGATGTCGCAAACGCTGTTTGCTCCTCTACAAAACAGACCTTAAAACCCTAAAGGCTTAAGTAGCACCCTC
GCAAGCTCGGGCAAATCGCTGAATATTCCTTTTGTCTCCGACCATCAGGCACCTGAGTCGCTGTCTTTTTCGTGACATTCAGT
TCGCTGCGCTCACGGCTCTGGCAGTGAATGGGGGTAAATGGCACTACAGGCGCCTTTTATGGATTCATGCAAGGAAACTACCC
ATAATACAAGAAAAGCCCGTCACGGGCTTCTCAGGGCGTTTTATGGCGGGTCTGCTATGTGGTGCTATCTGACTTTTTGCTGT
TCAGCAGTTCCTGCCCTCTGATTTTCCAGTCTGACCACTTCGGATTATCCCGTGACAGGTCATTCAGACTGGCTAATGCACCC
AGTAAGGCAGCGGTATCATCAACAGGCTTACCCGTCTTACTGTCAAGAGGACATCCGGTCAtgagcgcgcgTTGACAATTAAT
CATCGGCTCGTATAATGcctctagaaataatTCACACAGGAAAGtataCACATCATGAAATACCTGCTGCCGACCGCTGCTGC
TGGTCTGCTGCTCCTCGCTGCCCAGCCGGCGATGGCCGATGATGATGATGATAGCCCGGGTCAGGGTACGCAGAGCGAAAACA
GCTGTACGCATTTTCCGGGTAACCTGCCGAATATGCTGCGTGATCTGCGTGATGCATTTAGCCGTGTGAAAACATTTTTTCAG
ATGAAAGATCAGCTGGATAATCTGCTGCTGAAAGAAAGCCTGCTGGAAGATTTTAAAGGTTATCTGGGTTGTCAGGCCCTGAG
CGAAATGATTCAGTTTTATCTGGAAGAAGTTATGCCGCAGGCAGAAAATCAGGATCCTGATATTAAAGCACATGTTAATAGCC
TGGGTGAAAATCTGAAAACACTGCGTCTGCGTCTGCGCCGTTGCCATCGCTTTCTGCCGTGTGAAAATAAAAGCAAAGCAGTG
GAACAGGTTAAAAACGCATTTAATAAACTGCAGGAAAAAGGTATCTATAAAGCCATGTCAGAATTTGATATCTTTATCAATTA
TATCGAAGCATATATGACCATGAAAATCCGTAATCACCACCACCACCACCACTGAGATCCGGCTGCTAACAAAGCCCGAAAGG
ctgctaacaaagcccgaaaggaagctgagttggctgctgccaccgctgagcaataactagcataaccccttggggcctctaaa
cgg
SEQ ID NO: 8: plasmid-pSC101-ptac-pelB-huil10-KanR 
gtcttgaggggttttttgctgaaaggaggaactatatccggattggcgaatggggcgaattgggtaccgggccccccctcgag
ccgatcatattcaataacccttaatataacttcgtataatgtatgctatacgaagttattagGTCTGAAGAGGAGTTTACGTC
CCATATGGAGTCCCGCttaatcagcttgcttacgcaggaatgctgggatatccagataatccggctctttcgcagtttgcggc
gcattgtcattcacgactttagcaaccggcttctgctcctgggtcagcggagccatcccgtgctgctggtagcgatccatcac
tggctgctgaacctgcttattagtcaccagagtgatttcaggacgtttgtccatgccgatacctgtcgcaacaacagttacgc
 
gcagctcgtcattcatatccgggtcaagagaagtaccgataaccacggtcgcgttgtcggaagcaaatgcacggatggtgtta
cctaccgtttcgaactcatccagacgcaggtcgaagcccgctgtgatgtttaccagtacgccgcgtgcgccagacaggtcgat
atcttccagcagcggagaagagatagccatttcagcagcttcttccgcacgatcttcaccgctcgccacaccagaacccatca
ttgcgtagcccatttcagacattacggtgcgtacgtctgcaaagtccacgttcatcaaacccggacgagtaatcagttcggcg
ataccttgcacagcgccttttagtacatcgttcgctgcgccaaacgcatccagcagggagataccacggcccagaactttcag
cagtttgtcgttcgggatagtgatcagagagtccacatgcttggacagttcagtgatcccctgctccgcgaatgccatacgct
tcttgccttcaaagttgaaaggcttagtgacgacagcaacggtcaggatacccaaatcttttgccacttcagcgacgactggt
gctgcacctgtaccggtaccacccatacccgcagcaataaagaccatgtctgcaccttccagcgccgcacgcagtgcatc
gcgatcctcatcagccgcattgcggccaacttctggattagcgccagcgcccagtcctttggtgataccgctaccgatttgaa
tcgtctgtccaaccgctgttttacgcagcgcttgtgcatcggtatttaccgcgaagaattcaacaccttcaatgcgctcacgc
accatgtgttcaacagcattaccgccgccgccgacgccgatgactttaatcaccgcgtcattggtaagttccattggttc
aaacatAGATCATCGACCCCTCATTTTATTGCCAGATCAGAAGAACTcgtcaagaaggcgatagaaggcgatgcgctgcgaat
cgggagcggcgataccgtaaagcacgaggaagcggtcagcccattcgccgccaagctcttcagcaatatcacgggtagccaac
gctatgtcctgatagcggtccgccacacccagccggccacagtcgatgaatccagaaaagcggccattttccaccatgatatt
cggcaagcaggcatcgccatgggtcacgacgagatcctcgccgtcgggcatgcgcgccttgagcctggcgaacagttcggcCg
gcgcgagcccctgatgctcttcgtccagatcatcctgatcgacaagaccggcttccatccgagtacgtgctcgctcgatgcga
tgtttcgcttggtggtcgaatgggcaggtagccggatcaagcgtatgcagccgccgcattgcatcagccatgatggatacttt
ctcggcaggagcaaggtgagatgacaggagatcctgccccggcacttcgcccaatagcagccagtcccttcccgcttcagtga
caacgtcgagcacagctgcgcaaggaacgcccgtcgtggccagccacgatagccgcgctgcctcgtcctgcagttcattcagg
gcaccggacaggtcggtcttgacaaaaagaaccgggcgcccctgcgctgacagccggaacacggcggcatcagagcagccgat
tgtctgttgtgcccagtcatagccgaatagcctctccacccaagcggccggagaacctgcgtgcaatccatcttgttcaatca
tgcgaaacgatcctcatcctgtctcttgatcagatcCtgatcccctgcgccatcagatccttggcggcaagaaagccatccag
tttactttgcagggcttcccaaccttaccagagggcgccccagctggcaattccactagAGGACTTCGATTAGGCCAAATAGG
CCGTTCAGATCCTTCCGTATTTAGCCAGTATGTTCTCTAGTGTGGTTCGTTGTTTTTGCGTGAGCCATGAGAACGAACCATTG
AGATCATGCTTACTTTGCATGTCACTCAAAAATTTTGCCTCAAAACTGGTGAGCTGAATTTTTGCAGTTAAAGCATCGTGTAG
TGTTTTTCTTAGTCCGTTACGTAGGTAGGAATCTGATGTAATGGTTGTTGGTATTTTGTCACCATTCATTTTTATCTGGTTGT
TCTCAAGTTCGGTTACGAGATCCATTTGTCTATCTAGTTCAACTTGGAAAATCAACGTATCAGTCGGGCGGCCTCGCTTATCA
ACCACCAATTTCATATTGCTGTAAGTGTTTAAATCTTTACTTATTGGTTTCAAAACCCATTGGTTAAGCCTTTTAAACTCATG
GTAGTTATTTTCAAGCATTAACATGAACTTAAATTCATCAAGGCTAATCTCTATATTTGCCTTGTGAGTTTTCTTTTGTGTTA
GTTCTTTTAATAACCACTCATAAATCCTCATAGAGTATTTGTTTTCAAAAGACTTAACATGTTCCAGATTATATTTTATGAAT
TTTTTTAACTGGAAAAGATAAGGCAATATCTCTTCACTAAAAACTAATTCTAATTTTTCGCTTGAGAACTTGGCATAGTTTGT
CCACTGGAAAATCTCAAAGCCTTTAACCAAAGGATTCCTGATTTCCACAGTTCTCGTCATCAGCTCTCTGGTTGCTTTAGCTA
ATACACCATAAGCATTTTCCCTACTGATGTTCATCATCTGAGCGTATTGGTTATAAGTGAACGATACCGTCCGTTCTTTCCTT
GTAGGGTTTTCAATCGTGGGGTTGAGTAGTGCCACACAGCATAAAATTAGCTTGGTTTCATGCTCCGTTAAGTCATAGCGACT
AATCGCTAGTTCATTTGCTTTGAAAACAACTAATTCAGACATACATCTCAATTGGTCTAGGTGATTTTAATCACTATACCAAT
TGAGATGGGCTAGTCAATGATAATTACTAGTCCTTTTCCTTTGAGTTGTGGGTATCTGTAAATTCTGCTAGACCTTTGCTGGA
AAACTTGTAAATTCTGCTAGACCCTCTGTAAATTCCGCTAGACCTTTGTGTGTTTTTTTTGTTTATATTCAAGTGGTTATAAT
TTATAGAATAAAGAAAGAATAAAAAAAGATAAAAAGAATAGATCCCAGCCCTGTGTATAACTCACTACTTTAGTCAGTTCCGC
AGTATTACAAAAGGATGTCGCAAACGCTGTTTGCTCCTCTACAAAACAGACCTTAAAACCCTAAAGGCTTAAGTAGCACCCTC
GCAAGCTCGGGCAAATCGCTGAATATTCCTTTTGTCTCCGACCATCAGGCACCTGAGTCGCTGTCTTTTTCGTGACATTCAGT
TCGCTGCGCTCACGGCTCTGGCAGTGAATGGGGGTAAATGGCACTACAGGCGCCTTTTATGGATTCATGCAAGGAAACTACCC
ATAATACAAGAAAAGCCCGTCACGGGCTTCTCAGGGCGTTTTATGGCGGGTCTGCTATGTGGTGCTATCTGACTTTTTGCTGT
TCAGCAGTTCCTGCCCTCTGATTTTCCAGTCTGACCACTTCGGATTATCCCGTGACAGGTCATTCAGACTGGCTAATGCACCC
AGTAAGGCAGCGGTATCATCAACAGGCTTACCCGTCTTACTGTCAAGAGGACATCCGGTCAtgagcgcgcgTTGACAATTAAT
CATCGGCTCGTATAATGcctctagaaataatTCACACAGGAAAGtataCACATcATGAAATACCTGCTGCCGACCGCTGCTGC
TGGTCTGCTGCTCCTCGCTGCCCAGCCGGCGATGGCCAGCCCGGGTCAGGGTACGCAGAGCGAAAACAGCTGTACGCATTTTC
CGGGTAACCTGCCGAATATGCTGCGTGATCTGCGTGATGCATTTAGCCGTGTGAAAACATTTTTTCAGATGAAAGATCAGCTG
GATAATCTGCTGCTGAAAGAAAGCCTGCTGGAAGATTTTAAAGGTTATCTGGGTTGTCAGGCCCTGAGCGAAATGATTCAGTT
TTATCTGGAAGAAGTTATGCCGCAGGCAGAAAATCAGGATCCTGATATTAAAGCACATGTTAATAGCCTGGGTGAAAATCTGA
AAACACTGCGTCTGCGTCTGCGCCGTTGCCATCGCTTTCTGCCGTGTGAAAATAAAAGCAAAGCAGTGGAACAGGTTAAAAAC
GCATTTAATAAACTGCAGGAAAAAGGTATCTATAAAGCCATGTCAGAATTTGATATCTTTATCAATTATATCGAAGCATATAT
GACCATGAAAATCCGTAATCACCACCACCACCACCACTGAGATCCGGCTGCTAACAAAGCCCGAAAGGctgctaacaaagccc
gaaaggaagctgagttggctgctgccaccgctgagcaataactagcataaccccttggggcctctaaacgg
SEQ ID NO: 9: pelB-D5-huil10-monomer 
ATGAAATACCTGCTGCCGACCGCTGCTGCTGGTCTGCTGCTCCTCGCTGCCCAGCCGGCGATGGCCGATGATGATGATGATAG
CCCGGGTCAGGGTACGCAGAGCGAAAACAGCTGTACGCATTTTCCGGGTAACCTGCCGAATATGCTGCGTGATCTGCGTGATG
CATTTAGCCGTGTGAAAACATTTTTTCAGATGAAAGATCAGCTGGATAATCTGCTGCTGAAAGAAAGCCTGCTGGAAGATTTT
AAAGGTTATCTGGGTTGTCAGGCCCTGAGCGAAATGATTCAGTTTTATCTGGAAGAAGTTATGCCGCAGGCAGAAAATCAGGA
TCCTGATATTAAAGCACATGTTAATAGCCTGGGTGAAAATCTGAAAACACTGCGTCTGCGTCTGCGCCGTTGCCATCGCTTTC
TGCCGTGTGAAAATggcggtggcagcggtggcAAAAGCAAAGCAGTGGAACAGGTTAAAAACGCATTTAATAAACTGCAGGAA
AAAGGTATCTATAAAGCCATGTCAGAATTTGATATCTTTATCAATTATATCGAAGCATATATGACCATGAAAATCCGTAATCA
CCACCACCATCACCACTAA
SEQ ID NO: 10: pelB-D5-super-huil10 
ATGAAATACCTGCTGCCGACCGCTGCTGCTGGTCTGCTGCTCCTCGCTGCCCAGCCGGCGATGGCCGATGATGATGATGATAG
CCCGGGTCAGGGTACGCAGAGCGAAAACAGCTGTACGCATTTTCCGGGTTATCTGCCGAATATGCTGCGTGATCTGCGTGATG
CATTTAGCCGTGTGAAAACATTTTTTCAGATGAAAGATCAGCTGGATAATCTGCTGCTGAAAGAAAGCCTGCTGGAAGATTTT
AAAGGTTATCTGGGTTGTCAGGCCCTGAGCGAAATGATTCAGTTTTATCTGGAAGAAGTTATGCCGCAGGCAGAAAATCAGGA
TCCTGATATTAAAGCACATGTTCAGAGCCTGGGTGAAAATCTGAAAGATCTGCGTCTGTGGCTGCGCCGTTGCCATCGCTTTC
TGCCGTGTGAAAATAAAAGCAAAGCAGTGGAACAGGTTAAAAACGCATTTAATAAACTGCAGGAAAAAGGTATCTATAAAGCC
 
ATGTCAGAATTTGATATCTTTATCAATTATATCGAAGCATATATGACCATGAAAATCCGTAAT
CACCACCACCACCACCACTAG
SEQ ID NO: 11: pelB-D5-super-huil10-monomer 
ATGAAATACCTGCTGCCGACCGCTGCTGCTGGTCTGCTGCTCCTCGCTGCCCAGCCGGCGATGGCCGATGATGATGATGATAG
CCCGGGTCAGGGTACGCAGAGCGAAAACAGCTGTACGCATTTTCCGGGTTATCTGCCGAATATGCTGCGTGATCTGCGTGATG
CATTTAGCCGTGTGAAAACATTTTTTCAGATGAAAGATCAGCTGGATAATCTGCTGCTGAAAGAAAGCCTGCTGGAAGATTTT
AAAGGTTATCTGGGTTGTCAGGCCCTGAGCGAAATGATTCAGTTTTATCTGGAAGAAGTTATGCCGCAGGCAGAAAATCAGGA
TCCTGATATTAAAGCACATGTTCAGAGCCTGGGTGAAAATCTGAAAGATCTGCGTCTGTGGCTGCGCCGTTGCCATCGCTTTC
TGCCGTGTGAAAATggcggtggcagcggtggcAAAAGCAAAGCAGTGGAACAGGTTAAAAACGCATTTAATAAACTGCAGGAA
AAAGGTATCTATAAAGCCATGTCAGAATTTGATATCTTTATCAATTATATCGAAGCATATATGACCATGAAAATCCGTAAT
SEQ ID NO: 12: huIL-10 
SPGQGTQSENSCTHFPGNLPNMLRDLRDAFSRVKTFFQMKDQLDNLLLKESLLEDFKGYLGCQALSEMIQFYLEEVMPQAENQ
DPDIKAHVNSLGENLKTLRLRLRRCHRFLPCENKSKAVEQVKNAFNKLQEKGIYKAMSEFDIFINYIEAYMTMKIRN
SEQ ID NO: 13: huIL-10 monomer 
SPGQGTQSENSCTHFPGNLPNMLRDLRDAFSRVKTFFQMKDQLDNLLLKESLLEDFKGYLGCQALSEMIQFYLEEVMPQAENQ
DPDIKAHVNSLGENLKTLRLRLRRCHRFLPCENGGGSGGKSKAVEQVKNAFNKLQEKGIYKAMSEFDIFINYIEAYMTMKIRN
SEQ ID NO: 14: super-huIL-10 
SPGQGTQSENSCTHFPGYLPNMLRDLRDAFSRVKTFFQMKDQLDNLLLKESLLEDFKGYLGCQALSEMIQFYLEEVMPQAENQ
DPDIKAHVQSLGENLKDLRLWLRRCHRFLPCENKSKAVEQVKNAFNKLQEKGIYKAMSEFDIFINYIEAYMTMKIRN
SEQ ID NO: 15: super-huIL-10-monomer 
SPGQGTQSENSCTHFPGYLPNMLRDLRDAFSRVKTFFQMKDQLDNLLLKESLLEDFKGYLGCQALSEMIQFYLEEVMPQAENQ
DPDIKAHVQSLGENLKDLRLWLRRCHRFLPCENGGGSGGKSKAVEQVKNAFNKLQEKGIYKAMSEFDIFINYIEAYMTMKIRN
SEQ ID NO: 16: pelB signal peptide
   MKYLLPTAAAGLLLLAAQPAMA
SEQ ID NO: 17: pelB-D5-huIL-10 
MKYLLPTAAAGLLLLAAQPAMADDDDD
SPGQGTQSENSCTHFPGNLPNMLRDLRDAFSRVKTFFQMKDQLDNLLLKESLLEDFKGYLGCQALSEMIQFYLEEVMPQAENQ
DPDIKAHVNSLGENLKTLRLRLRRCHRFLPCENKSKAVEQVKNAFNKLQEKGIYKAMSEFDIFINYIEAYMTMKIRN
SEQ ID NO: 18: pelB-D5-huIL-10 monomer 
MKYLLPTAAAGLLLLAAQPAMADDDDD
SPGQGTQSENSCTHFPGNLPNMLRDLRDAFSRVKTFFQMKDQLDNLLLKESLLEDFKGYLGCQALSEMIQFYLEEVMPQAENQ
DPDIKAHVNSLGENLKTLRLRLRRCHRFLPCENGGGSGGKSKAVEQVKNAFNKLQEKGIYKAMSEFDIFINYIEAYMTMKIRN
SEQ ID NO: 19: pelB-D5-super-huIL-10 
MKYLLPTAAAGLLLLAAQPAMADDDDD
SPGQGTQSENSCTHFPGYLPNMLRDLRDAFSRVKTFFQMKDQLDNLLLKESLLEDFKGYLGCQALSEMIQFYLEEVMPQAENQ
DPDIKAHVQSLGENLKDLRLWLRRCHRFLPCENKSKAVEQVKNAFNKLQEKGIYKAMSEFDIFINYIEAYMTMKIRN
SEQ ID NO: 20: pelB-D5-super-huIL-10-monomer 
MKYLLPTAAAGLLLLAAQPAMADDDDD
SPGQGTQSENSCTHFPGYLPNMLRDLRDAFSRVKTFFQMKDQLDNLLLKESLLEDFKGYLGCQALSEMIQFYLEEVMPQAENQ
DPDIKAHVQSLGENLKDLRLWLRRCHRFLPCENGGGSGGKSKAVEQVKNAFNKLQEKGIYKAMSEFDIFINYIEAYMTMKIRN
SEQ ID NO: 21: pelB-D4-huIL-10 
MKYLLPTAAAGLLLLAAQPAMADDDD
SPGQGTQSENSCTHFPGNLPNMLRDLRDAFSRVKTFFQMKDQLDNLLLKESLLEDFKGYLGCQALSEMIQFYLEEVMPQAENQ
DPDIKAHVNSLGENLKTLRLRLRRCHRFLPCENKSKAVEQVKNAFNKLQEKGIYKAMSEFDIFINYIEAYMTMKIRN
SEQ ID NO: 22: pelB-D4-huIL-10 monomer 
MKYLLPTAAAGLLLLAAQPAMADDDD
SPGQGTQSENSCTHFPGNLPNMLRDLRDAFSRVKTFFQMKDQLDNLLLKESLLEDFKGYLGCQALSEMIQFYLEEVMPQAENQ
DPDIKAHVNSLGENLKTLRLRLRRCHRFLPCENGGGSGGKSKAVEQVKNAFNKLQEKGIYKAMSEFDIFINYIEAYMTMKIRN
SEQ ID NO: 23: pelB-D4-super-huIL-10 
MKYLLPTAAAGLLLLAAQPAMADDDD
SPGQGTQSENSCTHFPGYLPNMLRDLRDAFSRVKTFFQMKDQLDNLLLKESLLEDFKGYLGCQALSEMIQFYLEEVMPQAENQ
DPDIKAHVQSLGENLKDLRLWLRRCHRFLPCENKSKAVEQVKNAFNKLQEKGIYKAMSEFDIFINYIEAYMTMKIRN
SEQ ID NO: 24: pelB-D4-super-huIL-10-monomer 
MKYLLPTAAAGLLLLAAQPAMADDDD
SPGQGTQSENSCTHFPGYLPNMLRDLRDAFSRVKTFFQMKDQLDNLLLKESLLEDFKGYLGCQALSEMIQFYLEEVMPQAENQ
DPDIKAHVQSLGENLKDLRLWLRRCHRFLPCENGGGSGGKSKAVEQVKNAFNKLQEKGIYKAMSEFDIFINYIEAYMTMKIRN
SEQ ID NO: 50 pUC-ptac-bba0032-KanR 
ccaccgctgagcaataactagcataaccccttggggcctctaaacgggtcttgaggggttttttgctgaaaggaggaactata
tccggattggcgaatgggTCAGAAGAACTcgtcaagaaggcgatagaaggcgatgcgctgcgaatcgggagcggcgataccgt
aaagcacgaggaagcggtcagcccattcgccgccaagctcttcagcaatatcacgggtagccaacgctatgtcctgatagcgg
tccgccacacccagccggccacagtcgatgaatccagaaaagcggccattttccaccatgatattcggcaagcaggcatcgcc
atgggtcacgacgagatcctcgccgtcgggcatgcgcgccttgagcctggcgaacagttcggcCggcgcgagcccctgatgct
cttcgtccagatcatcctgatcgacaagaccggcttccatccgagtacgtgctcgctcgatgcgatgtttcgcttggtggtcg
aatgggcaggtagccggatcaagcgtatgcagccgccgcattgcatcagccatgatggatactttctcggcaggagcaaggtg
agatgacaggagatcctgccccggcacttcgcccaatagcagccagtcccttcccgcttcagtgacaacgtcgagcacagctg
cgcaaggaacgcccgtcgtggccagccacgatagccgcgctgcctcgtcctgcagttcattcagggcaccggacaggtcggtc
ttgacaaaaagaaccgggcgcccctgcgctgacagccggaacacggcggcatcagagcagccgattgtctgttgtgcccagtc
atagccgaatagcctctccacccaagcggccggagaacctgcgtgcaatccatcttgttcaatcatgcgaaacgatcctcatc
ctgtctcttgatcagatcCtgatcccctgcgccatcagatccttggcggcaagaaagccatccagtttactttgcagggcttc
ccaaccttaccagagggcgccccagctggcaattccactagAGGACTTCGATTAGGCCAAATAGcctttttgataatctcatg
accaaaatcccttaacgtgagttttcgttccactgagcgtcagaccccgtagaaaagatcaaaggatcttcttgagatccttt
ttttctgcgcgtaatctgctgcttgcaaacaaaaaaaccaccgctaccagcggtggtttgtttgccggatcaagagctaccaa
ctctttttccgaaggtaactggcttcagcagagcgcagataccaaatactgttcttctagtgtagccgtagttaggccaccac
ttcaagaactctgtagcaccgcctacatacctcgctctgctaatcctgttaccagtggctgctgccagtggcgataagtcgtg
tcttaccgggttggactcaagacgatagttaccggataaggcgcagcggtcgggctgaacggggggttcgtgcacacagccca
gcttggagcgaacgacctacaccgaactgagatacctacagcgtgagctatgagaaagcgccacgcttcccgaagggagaaag
gcggacaggtatccggtaagcggcagggtcggaacaggagagcgcacgagggagcttccagggggaaacgcctggtatcttta
tagtcctgtcgggtttcgccacctctgacttgagcgtcgatttttgtgatgctcgtcaggggggcggagcctatggaaaaacg
ccagcaacgcggcctttttacggttcctggccttttgctggccttttgctcacatgttctttcctgcgttatcccctgattct
gtggACAAGAGGACATCCGGTCAtgagcgcgcgTTGACAATTAATCATCGGCTCGTATAATGcctctagaaataatTCACACA
GGAAAGtataCACA
SEQ ID NO: 51 plasmid-pUC-ptac-pelB-D5-huil10-KanR 
GTGTGAAAACATTTTTTCAGATGAAAGATCAGCTGGATAATCTGCTGCTGAAAGAAAGCCTGCTGGAAGATTTTAAAGGTTAT
CTGGGTTGTCAGGCCCTGAGCGAAATGATTCAGTTTTATCTGGAAGAAGTTATGCCGCAGGCAGAAAATCAGGATCCTGATAT
TAAAGCACATGTTAATAGCCTGGGTGAAAATCTGAAAACACTGCGTCTGCGTCTGCGCCGTTGCCATCGCTTTCTGCCGTGTG
AAAATAAAAGCAAAGCAGTGGAACAGGTTAAAAACGCATTTAATAAACTGCAGGAAAAAGGTATCTATAAAGCCATGTCAGAA
TTTGATATCTTTATCAATTATATCGAAGCATATATGACCATGAAAATCCGTAATCACCACCACCACCACCACTGAGATCCGGC
TGCTAACAAAGCCCGAAAGGctgctaacaaagcccgaaaggaagctgagttggctgctgccaccgctgagcaataactagcat
aaccccttggggcctctaaacgggtcttgaggggttttttgctgaaaggaggaactatatccggattggcgaatgggTCAGAA
GAACTcgtcaagaaggcgatagaaggcgatgcgctgcgaatcgggagcggcgataccgtaaagcacgaggaagcggtcagccc
attcgccgccaagctcttcagcaatatcacgggtagccaacgctatgtcctgatagcggtccgccacacccagccggccacag
tcgatgaatccagaaaagcggccattttccaccatgatattcggcaagcaggcatcgccatgggtcacgacgagatcctcgcc
gtcgggcatgcgcgccttgagcctggcgaacagttcggcCggcgcgagcccctgatgctcttcgtccagatcatcctgatcga
caagaccggcttccatccgagtacgtgctcgctcgatgcgatgtttcgcttggtggtcgaatgggcaggtagccggatcaagc
gtatgcagccgccgcattgcatcagccatgatggatactttctcggcaggagcaaggtgagatgacaggagatcctgccccgg
cacttcgcccaatagcagccagtcccttcccgcttcagtgacaacgtcgagcacagctgcgcaaggaacgcccgtcgtggcca
gccacgatagccgcgctgcctcgtcctgcagttcattcagggcaccggacaggtcggtcttgacaaaaagaaccgggcgcccc
tgcgctgacagccggaacacggcggcatcagagcagccgattgtctgttgtgcccagtcatagccgaatagcctctccaccca
agcggccggagaacctgcgtgcaatccatcttgttcaatcatgcgaaacgatcctcatcctgtctcttgatcagatcCtgatc
ccctgcgccatcagatccttggcggcaagaaagccatccagtttactttgcagggcttcccaaccttaccagagggcgcccca
gctggcaattccactagAGGACTTCGATTAGGCCAAATAGcctttttgataatctcatgaccaaaatcccttaacgtgagttt
tcgttccactgagcgtcagaccccgtagaaaagatcaaaggatcttcttgagatcctttttttctgcgcgtaatctgctgctt
gcaaacaaaaaaaccaccgctaccagcggtggtttgtttgccggatcaagagctaccaactctttttccgaaggtaactggct
tcagcagagcgcagataccaaatactgttcttctagtgtagccgtagttaggccaccacttcaagaactctgtagcaccgcct
acatacctcgctctgctaatcctgttaccagtggctgctgccagtggcgataagtcgtgtcttaccgggttggactcaagacg
atagttaccggataaggcgcagcggtcgggctgaacggggggttcgtgcacacagcccagcttggagcgaacgacctacaccg
aactgagatacctacagcgtgagctatgagaaagcgccacgcttcccgaagggagaaaggcggacaggtatccggtaagcggc
agggtcggaacaggagagcgcacgagggagcttccagggggaaacgcctggtatctttatagtcctgtcgggtttcgccacct
ctgacttgagcgtcgatttttgtgatgctcgtcaggggggcggagcctatggaaaaacgccagcaacgcggcctttttacggt
tcctggccttttgctggccttttgctcacatgttctttcctgcgttatcccctgattctgtggACAAGAGGACATCCGGTCAt
gagcgcgcgTTGACAATTAATCATCGGCTCGTATAATGcctctagaaataatTCACACAGGAAAGtataCACATCATGAAATA
CCTGCTGCCGACCGCTGCTGCTGGTCTGCTGCTCCTCGCTGCCCAGCCGGCGATGGCCGATGATGATGATGATAGCCCGGGTC
AGGGTACGCAGAGCGAAAACAGCTGTACGCATTTTCCGGGTAACCTGCCGAATATGCTGCGTGATCTGCGTGATGCATTTAGC
C
SEQ ID NO: 52 plasmid-pUC-ptac-pelB-D5-mil10-KanR 
CACCACCACCACCACCACTGAGATCCGGCTGCTAACAAAGCCCGAAAGGctgctaacaaagcccgaaaggaagctgagttggc
tgctgccaccgctgagcaataactagcataaccccttggggcctctaaacgggtcttgaggggttttttgctgaaaggaggaa
ctatatccggattggcgaatgggTCAGAAGAACTcgtcaagaaggcgatagaaggcgatgcgctgcgaatcgggagcggcgat
accgtaaagcacgaggaagcggtcagcccattcgccgccaagctcttcagcaatatcacgggtagccaacgctatgtcctgat
agcggtccgccacacccagccggccacagtcgatgaatccagaaaagcggccattttccaccatgatattcggcaagcaggca
tcgccatgggtcacgacgagatcctcgccgtcgggcatgcgcgccttgagcctggcgaacagttcggcCggcgcgagcccctg
atgctcttcgtccagatcatcctgatcgacaagaccggcttccatccgagtacgtgctcgctcgatgcgatgtttcgcttggt
ggtcgaatgggcaggtagccggatcaagcgtatgcagccgccgcattgcatcagccatgatggatactttctcggcaggagca
aggtgagatgacaggagatcctgccccggcacttcgcccaatagcagccagtcccttcccgcttcagtgacaacgtcgagcac
agctgcgcaaggaacgcccgtcgtggccagccacgatagccgcgctgcctcgtcctgcagttcattcagggcaccggacaggt
 
cggtcttgacaaaaagaaccgggcgcccctgcgctgacagccggaacacggcggcatcagagcagccgattgtctgttgtgcc
cagtcatagccgaatagcctctccacccaagcggccggagaacctgcgtgcaatccatcttgttcaatcatgcgaaacgatcc
tcatcctgtctcttgatcagatcCtgatcccctgcgccatcagatccttggcggcaagaaagccatccagtttactttgcagg
gcttcccaaccttaccagagggcgccccagctggcaattccactagAGGACTTCGATTAGGCCAAATAGcctttttgataatc
tcatgaccaaaatcccttaacgtgagttttcgttccactgagcgtcagaccccgtagaaaagatcaaaggatcttcttgagat
cctttttttctgcgcgtaatctgctgcttgcaaacaaaaaaaccaccgctaccagcggtggtttgtttgccggatcaagagct
accaactctttttccgaaggtaactggcttcagcagagcgcagataccaaatactgttcttctagtgtagccgtagttaggcc
accacttcaagaactctgtagcaccgcctacatacctcgctctgctaatcctgttaccagtggctgctgccagtggcgataag
tcgtgtcttaccgggttggactcaagacgatagttaccggataaggcgcagcggtcgggctgaacggggggttcgtgcacaca
gcccagcttggagcgaacgacctacaccgaactgagatacctacagcgtgagctatgagaaagcgccacgcttcccgaaggga
gaaaggcggacaggtatccggtaagcggcagggtcggaacaggagagcgcacgagggagcttccagggggaaacgcctggtat
ctttatagtcctgtcgggtttcgccacctctgacttgagcgtcgatttttgtgatgctcgtcaggggggcggagcctatggaa
aaacgccagcaacgcggcctttttacggttcctggccttttgctggccttttgctcacatgttctttcctgcgttatcccctg
attctgtggACAAGAGGACATCCGGTCAtgagcgcgcgTTGACAATTAATCATCGGCTCGTATAATGcctctagaaataatTC
ACACAGGAAAGtataCACATCATGAAATACCTGCTGCCGACCGCTGCTGCTGGTCTGCTGCTCCTCGCTGCCCAGCCGGCGAT
GGCCGATGATGATGATGATAGCCGCGGCCAGTACAGCCGCGAGGACAATAACTGCACCCATTTTCCGGTGGGCCAGAGCCATA
TGCTGCTGGAACTGCGCACCGCGTTTAGCCAAGTTAAGACGTTCTTCCAGACCAAAGACCAGCTGGATAACATTTTACTGACC
GACTCTTTAATGCAAGATTTTAAGGGCTATCTGGGCTGCCAAGCTTTAAGCGAGATGATCCAGTTCTATTTAGTGGAAGTGAT
GCCGCAAGCTGAAAAACACGGCCCGGAGATCAAAGAACATTTAAACTCTTTAGGCGAGAAACTGAAGACTTTACGCATGCGTT
TACGTCGTTGCCATCGCTTTCTGCCGTGCGAGAACAAAAGCAAAGCGGTGGAGCAAGTTAAGAGCGACTTCAACAAACTGCAA
GATCAAGGTGTGTATAAGGCGATGAACGAATTCGATATCTTTATCAACTGCATCGAAGCGTATATGATGATTAAAATGAAAAG
C
SEQ ID NO:53: PsseA-hlyA-loxp-KnaR-loxp 
TTCACAGAAAAGTGTTGCCCCCTTCCATGGCGGAAGGGGGACAAAGGTGAATTTGTCCTACTCAGGAGAGCGTTCACCGACAA
ACAACAGATAAAACGAAAGGCCCAGTCTTTCGACTGAGCCTTTCGTTTTATTTGATGCACTAGTGCCGATCATATTCAATAAC
CCTTAATATAACTTCGTATAATGTATGCTATACGAAGTTATTAGGTCTGAAGAGGAGTTTACGTCCCATATGGAGTCCCGCTC
AGAAGAACTCGTCAAGAAGGCGATAGAAGGCGATGCGCTGCGAATCGGGAGCGGCGATACCGTAAAGCACGAGGAAGCGGTCA
GCCCATTCGCCGCCAAGCTCTTCAGCAATATCACGGGTAGCCAACGCTATGTCCTGATAGCGGTCCGCCACACCCAGCCGGCC
ACAGTCGATGAATCCAGAAAAGCGGCCATTTTCCACCATGATATTCGGCAAGCAGGCATCGCCATGGGTCACGACGAGATCCT
CGCCGTCGGGCATGCGCGCCTTGAGCCTGGCGAACAGTTCGGCCGGCGCGAGCCCCTGATGCTCTTCGTCCAGATCATCCTGA
TCGACAAGACCGGCTTCCATCCGAGTACGTGCTCGCTCGATGCGATGTTTCGCTTGGTGGTCGAATGGGCAGGTAGCCGGATC
AAGCGTATGCAGCCGCCGCATTGCATCAGCCATGATGGATACTTTCTCGGCAGGAGCAAGGTGAGATGACAGGAGATCCTGCC
CCGGCACTTCGCCCAATAGCAGCCAGTCCCTTCCCGCTTCAGTGACAACGTCGAGCACAGCTGCGCAAGGAACGCCCGTCGTG
GCCAGCCACGATAGCCGCGCTGCCTCGTCCTGCAGTTCATTCAGGGCACCGGACAGGTCGGTCTTGACAAAAAGAACCGGGCG
CCCCTGCGCTGACAGCCGGAACACGGCGGCATCAGAGCAGCCGATTGTCTGTTGTGCCCAGTCATAGCCGAATAGCCTCTCCA
CCCAAGCGGCCGGAGAACCTGCGTGCAATCCATCTTGTTCAATCATGCGAAACGATCCTCATCCTGTCTCTTGATCAGATCCT
GATCCCCTGCGCCATCAGATCCTTGGCGGCAAGAAAGCCATCCAGTTTACTTTGCAGGGCTTCCCAACCTTACCAGAGGGCGC
CCCAGCTGGCAATTCCGACGTCATTGCGTTGCTAGCCAAGCTTAGGATCCGGAACCCTTAATATAACTTCGTATAATGTATGC
TATACGAAGTTATTAGGTCCCTCGAAGAGGTTCACTAGCTATCTAGAGCTTGGACTCCTGTTGATAGATCCAGTAATGACCTC
AGAACTCCATCTGGATTTGTTCAGAACGCTCGGTTGCCGCCGGGCGTTTTTTATTGGTGAGAATCCAAGCCCGGGAAACGGGA
TCCTGATCTTGATCAGGCTGTGGGTCAGGCCACTCTTGGCATCGACACCAATGTGGGCCTTCATGCCAAAGTGCCACTGATTG
CCTTTCTTGGTCTGATGCATCTCCGGATCGCGTTGCTGCTCTTTGTTCTTGGTCGAGCTGGGTGCCTCAATGATGGTGGCATC
GACCAAGGTGCCTTGAGTCATCATGACGCCTGCTTCGGCCAGCCAGCGATTGATGGTCTTGAACAATTGGCGGGCCAGTTGAT
GCTGCTCCAGCAGGTGGCGGAAATTCATGATGGTGGTGCGGTCCGGCAAGGCGCTATCCAGGGATAACCGGGCAAACAGACGC
ATGGAGGCGATTTCGTACAGAGCATCTTCCATCGCGCCATCGCTCAGGTTGTACCAATGCTGCATGCAGTGAATGCGTAGCAT
GGTTTCCAGCGGATAAGGTCGCCGGCCATTACCAGCCTTGGGGTAAAACGGCTCGATGACTTCCACCATGTTTTGCCATGGCA
GAATCTGCTCCATGCGGGACAAGAAAATCTCTTTTCTGGTCTGACGGCGCTTACTGCTGAATTCACTGTCGGCGAAGGTAAGT
TGATGACTCATGATGAACCCTGTTCTATGGCTCCAGATGACAAACATGATCTCATATCAGGGACTTGTTCGCACCTTCCTTAG
CACGTTAATTATCTATCGTGTATATGGAGGGGATCTAGAATGAAAAAAATAATGCTAGTTTTTATTACACTTATATTAGTTAG
TCTACCAATTGCGCAACAAACTGAAGCAAAGGATGCATCTGCATTCAATAAAGAAAATTCAATTTCATCCATGGCACCACCAG
CATCTCCGCCTGCAAGTCCTAAGACGCCAATCGAAAAGAAACACGCGGATGAAATCGATAAGTATATACAAGGATTGGACTAC
AATAAAAACAATGTATTAGTATACCACGGAGATGCAGTGACAAATGTGCCGCCAAGAAAAGGTTACAAAGATGGAAATGAATA
TATTGTTGTGGAGAAAAAGAAGAAATCCATCAATCAAAATAATGCAGACATTCAAGTTGTGAATGCAATTTCGAGCCTAACCT
ATCCAGGTGCTCTCGTAAAAGCGAATTCGGAATTAGTAGAAAATCAACCAGATGTTCTCCCTGTAAAACGTGATTCATTAACA
CTCAGCATTGATTTGCCAGGTATGACTAATCAAGACAATAAAATCGTTGTAAAAAATGCCACTAAATCAAACGTTAACAACGC
AGTAAATACATTAGTGGAAAGATGGAATGAAAAATATGCTCAAGCTTATCCAAATGTAAGTGCAAAAATTGATTATGATGACG
AAATGGCTTACAGTGAATCACAATTAATTGCGAAATTTGGTACAGCATTTAAAGCTGTAAATAATAGCTTGAATGTAAACTTC
GGCGCAATCAGTGAAGGGAAAATGCAAGAAGAAGTCATTAGTTTTAAACAAATTTACTATAACGTGAATGTTAATGAACCTAC
AAGACCTTCCAGATTTTTCGGCAAAGCTGTTACTAAAGAGCAGTTGCAAGCGCTTGGAGTGAATGCAGAAAATCCTCCTGCAT
ATATCTCAAGTGTGGCGTATGGCCGTCAAGTTTATTTGAAATTATCAACTAATTCCCATAGTACTAAAGTAAAAGCTGCTTTT
GATGCTGCCGTAAGCGGAAAATCTGTCTCAGGTGATGTAGAACTAACAAATATCATCAAAAATTCTTCCTTCAAAGCCGTAAT
TTACGGAGGTTCCGCAAAAGATGAAGTTCAAATCATCGACGGCAACCTCGGAGACTTACGAGATATTTTGAAAAAAGGCGCTA
CTTTTAATCGAGAAACACCAGGAGTTCCCATTGCTTATACAACAAACTTCCTAAAAGACAATGAATTAGCTGTTATTAAAAAC
AACTCAGAATATATTGAAACAACTTCAAAAGCTTATACAGATGGAAAAATTAACATCGATCACTCTGGAGGATACGTTGCTCA
ATTCAACATTTCTTGGGATGAAGTAAATTATGATCCTGAAGGTAACGAAATTGTTCAACATAAAAACTGGAGCGAAAACAATA
AAAGTAAGCTAGCTCATTTCACATCGTCCATCTATTTGCCAGGTAACGCGAGAAATATTAATGTTTACGCTAAAGAATGCACT
GGTTTAGCTTGGGAATGGTGGAGAACGGTAATTGATGACCGGAACTTACCACTTGTGAAAAATAGAAATATCTCCATCTGGGG
CACCACGCTTTATCCGAAATATAGTAATAAAGTAGATAATCCAATCGAATAAGTCGACCTCGAGGACTAGTGAACCTCTTCGA
GGGACCTAATAACTTCGTATAGCATACATTATACGAAGTTATATTAAGGGTTATTGAATATGATCGGCTCGAGATTACTTCGC
 
ATTGCAATCAGCTACGGACTCAAGCTTACGTCAGGTCATACGATGAAGGCGATAAGTCGCGTAAGTTGCTAGCCCACGCGGCT
CACCTTCACGGGTGGGCCTTTCTTCGGTGTTTCAATCTCGATTAACAGGTAACGCAAAATTGCTGTGTACGTCAGA
SEQ ID NO:54: Pssbp1-dapE-Cm 
TTGGTTTCAGTGAATCCCGTTATCAGCAGTTTTTTGATGAGGTGTAGTCTATTTGTCCTACTCAGGAGAGCGTTCACCGACAA
ACAACAGATAAAACGAAAGGCCCAGTCTTTCGACTGAGCCTTTCGTTTTATTTGATGCACTAGTGCCGATCATATTCAATAAC
CCTTAATATAACTTCGTATAATGTATGCTATACGAAGTTATTAGGTCTGAAGAGGAGTTTACGTCCCATATGGAGTCCCGCTT
ACGCCCCGCCCTGCCACTCATCGCAGTACTGTTGTAATTCATTAAGCATTCTGCCGACATGGAAGCCATCACAGACGGCATGA
TGAACCTGAATCGCCAGCGGCATCAGCACCTTGTCGCCTTGCGTATAATATTTGCCCATGGTGAAAACGGGGGCGAAGAAGTT
GTCCATATTGGCCACGTTTAAATCAAAACTGGTGAAACTCACCCAGGGATTGGCTGAGACGAAAAACATATTCTCAATAAACC
CTTTAGGGAAATAGGCCAGGTTTTCACCGTAACACGCCACATCTTGCGAATATATGTGTAGAAACTGCCGGAAATCGTCGTGG
TATTCACTCCAGAGCGATGAAAACGTTTCAGTTTGCTCATGGAAAACGGTGTAACAAGGGTGAACACTATCCCATATCACCAG
CTCACCGTCTTTCATTGCCATACGAAATTCCGGATGAGCATTCATCAGGCGGGCAAGAATGTGAATAAAGGCCGGATAAAACT
TGTGCTTATTTTTCTTTACGGTCTTTAAAAAGGCCGTAATATCCAGCTGAACGGTCTGGTTATAGGTACATTGAGCAACTGAC
TGAAATGCCTCAAAATGTTCTTTACGATGCCATTGGGATATATCAACGGTGGTATATCCAGTGATTTTTTTCTCCATGCGAAA
CGATCCTCATCCTGTCTCTTGATCAGATCCTGATCCCCTGCGCCATCAGATCCTTGGCGGCAAGAAAGCCATCCAGTTTACTT
TGCAGGGCTTCCCAACCTTACCAGAGGGCGCCCCAGCTGGCAATTCCGACGTCATTGCGTTGCTAGCCAAGCTTAGGATCCGG
AACCCTTAATATAACTTCGTATAATGTATGCTATACGAAGTTATTAGGTCCCTCGAAGAGGTTCACTAGCTATCTAGAGCTTG
GACTCCTGTTGATAGATCCAGTAATGACCTCAGAACTCCATCTGGATTTGTTCAGAACGCTCGGTTGCCGCCGGGCGTTTTTT
ATTGGTGAGAATCCAAGCCCGGGAAACGGGATCCTGATCTTGATCAACCGAGGTCACAACATAGTAAAAGCGCTATTGGTAAT
GGTACAATCGCGCGTTTACACTTATTCAGAACGATTTTTTTCAGGAGGGCCTACTTGTCACACAGGACGGTAATGTCGTGCCC
GGTTATTGAGCTGACACAGCAACTTATTCGTCGCCCCTCCCTGAGTCCGGATGACGCAGGCTGTCAGGCATTAATGATTGAAC
GCCTGCGTAAAATCGGTTTTACCATTGAGCATATGGATTTTGGCGACACGCAAAATTTTTGGGCATGGCGTGGGCGTGGCGAA
ACGCTGGCGTTCGCCGGGCATACTGACGTCGTACCGGCTGGCGATGTCGATCGCTGGATCAATCCGCCGTTTGAGCCTACGAT
TCGCGACGGAATGTTGTTCGGTCGCGGCGCGGCGGATATGAAAGGTTCACTGGCGGCCATGGTGGTGGCTGCTGAACGTTTTG
TTGCGCAGCATCCTCATCACCGGGGCCGCCTCGCATTTTTGATTACCTCTGATGAGGAAGCCAGCGCAAAAAATGGCACCGTA
AAGGTGGTTGAAGCGCTGATGGCCCGTAACGAGCGGCTGGATTACTGTCTGGTCGGCGAACCTTCCAGTACTGAAATTGTGGG
CGATGTCGTCAAAAACGGGCGTCGCGGTTCTCTCACCTGTAACCTCACTATCCATGGCGTTCAGGGGCACGTCGCCTATCCGC
ATCTGGCGGATAACCCTGTTCATCGCGCCGCGCCTTTTCTTAATGAACTGGTCGCTATCGAGTGGGATCGGGGCAATGACTTT
TTCCCGGCCACCAGTATGCAGGTAGCCAATATCCAGGCAGGCACCGGCAGCAATAATGTCATTCCCGGCGAACTGTTCGTGCA
GTTTAACTTCCGTTTCAGCACGGAACTGACCGATGAGATGATCAAAGAGCGGGTACATGCGCTGCTTGAAAAGCATCAGCTAC
GCTATACCGTAGACTGGTGGCTTTCCGGGCAGCCGTTCCTGACCGCACGCGGCAAGCTGGTGGACGCGGTGGTAAACGCCATT
GAGCACTATAATGAAATTAAACCGCAATTACTGACGACGGGCGGTACGTCCGACGGACGTTTTATCGCCCGTATGGGAGCGCA
GGTGGTAGAACTGGGGCCGGTGAACGCCACCATTCATAAAATTAATGAATGTGTGAATGCCGCCGATCTCCAGCTACTGGCCC
GTATGTATCAACGCATTATGGAACAGCTCGTCGCCCATCACCATCACCATCACTAAGATTACTTCGCATTGCAATCAGCTACG
GACTCAAGCTTACGTCAGGTCATACGATGAAGGCGATAAGTCGCGTAAGTTGCTAGCCCACGCGGCTCACCTTCACGGGTGGG
CCTTTCTTCGTGAGCGTTCCGGTAAGAGGAAATAAGCATGGACTGGCTGGCAAAATATTG
SEQ ID NO:55: PsseA-hlyA-loxp-KnaR-loxp plasmid 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCGACTTCCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
 
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTACTGTCTAGGGCGGCGGATTTGTCCTACTCAGGAGAGCGTTCACCGACAAACAACAGATA
AAACGAAAGGCCCAGTCTTTCGACTGAGCCTTTCGTTTTATTTGATGCACTAGTGCCGATCATATTCAATAACCCTTAATATA
ACTTCGTATAATGTATGCTATACGAAGTTATTAGGTCTGAAGAGGAGTTTACGTCCCATATGGAGTCCCGCTCAGAAGAACTC
GTCAAGAAGGCGATAGAAGGCGATGCGCTGCGAATCGGGAGCGGCGATACCGTAAAGCACGAGGAAGCGGTCAGCCCATTCGC
CGCCAAGCTCTTCAGCAATATCACGGGTAGCCAACGCTATGTCCTGATAGCGGTCCGCCACACCCAGCCGGCCACAGTCGATG
AATCCAGAAAAGCGGCCATTTTCCACCATGATATTCGGCAAGCAGGCATCGCCATGGGTCACGACGAGATCCTCGCCGTCGGG
CATGCGCGCCTTGAGCCTGGCGAACAGTTCGGCCGGCGCGAGCCCCTGATGCTCTTCGTCCAGATCATCCTGATCGACAAGAC
CGGCTTCCATCCGAGTACGTGCTCGCTCGATGCGATGTTTCGCTTGGTGGTCGAATGGGCAGGTAGCCGGATCAAGCGTATGC
AGCCGCCGCATTGCATCAGCCATGATGGATACTTTCTCGGCAGGAGCAAGGTGAGATGACAGGAGATCCTGCCCCGGCACTTC
GCCCAATAGCAGCCAGTCCCTTCCCGCTTCAGTGACAACGTCGAGCACAGCTGCGCAAGGAACGCCCGTCGTGGCCAGCCACG
ATAGCCGCGCTGCCTCGTCCTGCAGTTCATTCAGGGCACCGGACAGGTCGGTCTTGACAAAAAGAACCGGGCGCCCCTGCGCT
GACAGCCGGAACACGGCGGCATCAGAGCAGCCGATTGTCTGTTGTGCCCAGTCATAGCCGAATAGCCTCTCCACCCAAGCGGC
CGGAGAACCTGCGTGCAATCCATCTTGTTCAATCATGCGAAACGATCCTCATCCTGTCTCTTGATCAGATCCTGATCCCCTGC
GCCATCAGATCCTTGGCGGCAAGAAAGCCATCCAGTTTACTTTGCAGGGCTTCCCAACCTTACCAGAGGGCGCCCCAGCTGGC
AATTCCGACGTCATTGCGTTGCTAGCCAAGCTTAGGATCCGGAACCCTTAATATAACTTCGTATAATGTATGCTATACGAAGT
TATTAGGTCCCTCGAAGAGGTTCACTAGCTATCTAGAGCTTGGACTCCTGTTGATAGATCCAGTAATGACCTCAGAACTCCAT
CTGGATTTGTTCAGAACGCTCGGTTGCCGCCGGGCGTTTTTTATTGGTGAGAATCCAAGCCCGGGAAACGGGATCCTGATCTT
GATCAGGCTGTGGGTCAGGCCACTCTTGGCATCGACACCAATGTGGGCCTTCATGCCAAAGTGCCACTGATTGCCTTTCTTGG
TCTGATGCATCTCCGGATCGCGTTGCTGCTCTTTGTTCTTGGTCGAGCTGGGTGCCTCAATGATGGTGGCATCGACCAAGGTG
CCTTGAGTCATCATGACGCCTGCTTCGGCCAGCCAGCGATTGATGGTCTTGAACAATTGGCGGGCCAGTTGATGCTGCTCCAG
CAGGTGGCGGAAATTCATGATGGTGGTGCGGTCCGGCAAGGCGCTATCCAGGGATAACCGGGCAAACAGACGCATGGAGGCGA
TTTCGTACAGAGCATCTTCCATCGCGCCATCGCTCAGGTTGTACCAATGCTGCATGCAGTGAATGCGTAGCATGGTTTCCAGC
GGATAAGGTCGCCGGCCATTACCAGCCTTGGGGTAAAACGGCTCGATGACTTCCACCATGTTTTGCCATGGCAGAATCTGCTC
CATGCGGGACAAGAAAATCTCTTTTCTGGTCTGACGGCGCTTACTGCTGAATTCACTGTCGGCGAAGGTAAGTTGATGACTCA
TGATGAACCCTGTTCTATGGCTCCAGATGACAAACATGATCTCATATCAGGGACTTGTTCGCACCTTCCTTAGCACGTTAATT
ATCTATCGTGTATATGGAGGGGATCTAGAATGAAAAAAATAATGCTAGTTTTTATTACACTTATATTAGTTAGTCTACCAATT
GCGCAACAAACTCAACCAAAGCATGCATCTGCATTCAATAAACAAAATTCAATTTCATCCATGGCACCACCAGCATCTCCGCC
TGCAAGTCCTAAGACGCCAATCGAAAAGAAACACGCGGATGAAATCGATAAGTATATACAAGGATTGGACTACAATAAAAACA
ATGTATTAGTATACCACGGAGATGCAGTGACAAATGTGCCGCCAAGAAAAGGTTACAAAGATGGAAATGAATATATTGTTGTG
GAGAAAAAGAAGAAATCCATCAATCAAAATAATGCAGACATTCAAGTTGTGAATGCAATTTCGAGCCTAACCTATCCAGGTGC
TCTCGTAAAAGCGAATTCGGAATTAGTAGAAAATCAACCAGATGTTCTCCCTGTAAAACGTGATTCATTAACACTCAGCATTG
ATTTGCCAGGTATGACTAATCAAGACAATAAAATCGTTGTAAAAAATGCCACTAAATCAAACGTTAACAACGCAGTAAATACA
TTAGTGGAAAGATGGAATGAAAAATATGCTCAAGCTTATCCAAATGTAAGTGCAAAAATTGATTATGATGACGAAATGGCTTA
CAGTGAATCACAATTAATTGCGAAATTTGGTACAGCATTTAAAGCTGTAAATAATAGCTTGAATGTAAACTTCGGCGCAATCA
GTGAAGGGAAAATGCAAGAAGAAGTCATTAGTTTTAAACAAATTTACTATAACGTGAATGTTAATGAACCTACAAGACCTTCC
AGATTTTTCGGCAAAGCTGTTACTAAAGAGCAGTTGCAAGCGCTTGGAGTGAATGCAGAAAATCCTCCTGCATATATCTCAAG
TGTGGCGTATGGCCGTCAAGTTTATTTGAAATTATCAACTAATTCCCATAGTACTAAAGTAAAAGCTGCTTTTGATGCTGCCG
TAAGCGGAAAATCTGTCTCAGGTGATGTAGAACTAACAAATATCATCAAAAATTCTTCCTTCAAAGCCGTAATTTACGGAGGT
TCCGCAAAAGATGAAGTTCAAATCATCGACGGCAACCTCGGAGACTTACGAGATATTTTGAAAAAAGGCGCTACTTTTAATCG
AGAAACACCAGGAGTTCCCATTGCTTATACAACAAACTTCCTAAAAGACAATGAATTAGCTGTTATTAAAAACAACTCAGAAT
ATATTGAAACAACTTCAAAAGCTTATACAGATGGAAAAATTAACATCGATCACTCTGGAGGATACGTTGCTCAATTCAACATT
TCTTGGGATGAAGTAAATTATGATCCTGAAGGTAACGAAATTGTTCAACATAAAAACTGGAGCGAAAACAATAAAAGTAAGCT
AGCTCATTTCACATCGTCCATCTATTTGCCAGGTAACGCGAGAAATATTAATGTTTACGCTAAAGAATGCACTGGTTTAGCTT
GGGAATGGTGGAGAACGGTAATTGATGACCGGAACTTACCACTTGTGAAAAATAGAAATATCTCCATCTGGGGCACCACGCTT
TATCCGAAATATAGTAATAAAGTAGATAATCCAATCGAATAAGTCGACCTCGAGGACTAGTGAACCTCTTCGAGGGACCTAAT
AACTTCGTATAGCATACATTATACGAAGTTATATTAAGGGTTATTGAATATGATCGGCTCGAGATTACTTCGCATTGCAATCA
GCTACGGACTCAAGCTTACGTCAGGTCATACGATGAAGGCGATAAGTCGCGTAAGTTGCTAGCCCACGCGGCTCACCTTCACG
GGTGGGCCTTTCTTCGCGATCCACATG
SEQ ID NO:56: Pssbp1-dapE-Cm plasmid 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCGACTTCCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
 
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTACTGTCTAGGGCGGCGGATTTGTCCTACTCAGGAGAGCGTTCACCGACAAACAACAGATA
AAACGAAAGGCCCAGTCTTTCGACTGAGCCTTTCGTTTTATTTGATGCACTAGTGCCGATCATATTCAATAACCCTTAATATA
ACTTCGTATAATGTATGCTATACGAAGTTATTAGGTCTGAAGAGGAGTTTACGTCCCATATGGAGTCCCGCTTACGCCCCGCC
CTGCCACTCATCGCAGTACTGTTGTAATTCATTAAGCATTCTGCCGACATGGAAGCCATCACAGACGGCATGATGAACCTGAA
TCGCCAGCGGCATCAGCACCTTGTCGCCTTGCGTATAATATTTGCCCATGGTGAAAACGGGGGCGAAGAAGTTGTCCATATTG
GCCACGTTTAAATCAAAACTGGTGAAACTCACCCAGGGATTGGCTGAGACGAAAAACATATTCTCAATAAACCCTTTAGGGAA
ATAGGCCAGGTTTTCACCGTAACACGCCACATCTTGCGAATATATGTGTAGAAACTGCCGGAAATCGTCGTGGTATTCACTCC
AGAGCGATGAAAACGTTTCAGTTTGCTCATGGAAAACGGTGTAACAAGGGTGAACACTATCCCATATCACCAGCTCACCGTCT
TTCATTGCCATACGAAATTCCGGATGAGCATTCATCAGGCGGGCAAGAATGTGAATAAAGGCCGGATAAAACTTGTGCTTATT
TTTCTTTACGGTCTTTAAAAAGGCCGTAATATCCAGCTGAACGGTCTGGTTATAGGTACATTGAGCAACTGACTGAAATGCCT
CAAAATGTTCTTTACGATGCCATTGGGATATATCAACGGTGGTATATCCAGTGATTTTTTTCTCCATTTTAGCTTCCTTAGCT
CCTGAAAATCTCGATAACTCAAAAAATACGCCCGGTAGTGATCTTATTTCATTATGGTGAAAGTTGGAACCTCTTACGTGCCG
ATCAGACGTCATTGCGTTGCTAGCCAAGCTTAGGATCCGGAACCCTTAATATAACTTCGTATAATGTATGCTATACGAAGTTA
TTAGGTCCCTCGAAGAGGTTCACTAGCTATCTAGAGCTTGGACTCCTGTTGATAGATCCAGTAATGACCTCAGAACTCCATCT
GGATTTGTTCAGAACGCTCGGTTGCCGCCGGGCGTTTTTTATTGGTGAGAATCCAAGCCCGGGAAACGGGATCCTGATCTTGA
TCAACCGAGGTCACAACATAGTAAAAGCGCTATTGGTAATGGTACAATCGCGCGTTTACACTTATTCAGAACGATTTTTTTCA
GGAGGGCCTACTTGTCACACAGGACGGTAATGTCGTGCCCGGTTATTGAGCTGACACAGCAACTTATTCGTCGCCCCTCCCTG
AGTCCGGATGACGCAGGCTGTCAGGCATTAATGATTGAACGCCTGCGTAAAATCGGTTTTACCATTGAGCATATGGATTTTGG
CGACACGCAAAATTTTTGGGCATGGCGTGGGCGTGGCGAAACGCTGGCGTTCGCCGGGCATACTGACGTCGTACCGGCTGGCG
ATGTCGATCGCTGGATCAATCCGCCGTTTGAGCCTACGATTCGCGACGGAATGTTGTTCGGTCGCGGCGCGGCGGATATGAAA
GGTTCACTGGCGGCCATGGTGGTGGCTGCTGAACGTTTTGTTGCGCAGCATCCTCATCACCGGGGCCGCCTCGCATTTTTGAT
TACCTCTGATGAGGAAGCCAGCGCAAAAAATGGCACCGTAAAGGTGGTTGAAGCGCTGATGGCCCGTAACGAGCGGCTGGATT
ACTGTCTGGTCGGCGAACCTTCCAGTACTGAAATTGTGGGCGATGTCGTCAAAAACGGGCGTCGCGGTTCTCTCACCTGTAAC
CTCACTATCCATGGCGTTCAGGGGCACGTCGCCTATCCGCATCTGGCGGATAACCCTGTTCATCGCGCCGCGCCTTTTCTTAA
TGAACTGGTCGCTATCGAGTGGGATCGGGGCAATGACTTTTTCCCGGCCACCAGTATGCAGGTAGCCAATATCCAGGCAGGCA
CCGGCAGCAATAATGTCATTCCCGGCGAACTGTTCGTGCAGTTTAACTTCCGTTTCAGCACGGAACTGACCGATGAGATGATC
AAAGAGCGGGTACATGCGCTGCTTGAAAAGCATCAGCTACGCTATACCGTAGACTGGTGGCTTTCCGGGCAGCCGTTCCTGAC
CGCACGCGGCAAGCTGGTGGACGCGGTGGTAAACGCCATTGAGCACTATAATGAAATTAAACCGCAATTACTGACGACGGGCG
GTACGTCCGACGGACGTTTTATCGCCCGTATGGGAGCGCAGGTGGTAGAACTGGGGCCGGTGAACGCCACCATTCATAAAATT
AATGAATGTGTGAATGCCGCCGATCTCCAGCTACTGGCCCGTATGTATCAACGCATTATGGAACAGCTCGTCGCCCATCACCA
TCACCATCACTAAGATTACTTCGCATTGCAATCAGCTACGGACTCAAGCTTACGTCAGGTCATACGATGAAGGCGATAAGTCG
CGTAAGTTGCTAGCCCACGCGGCTCACCTTCACGGGTGGGCCTTTCTTCGCGATCCACATG
SEQ ID NO:57: PsseA-hlyA-loxp-KnaR-loxp forwad primer
   TTCACAGAAAAGTGTTGCCCCCTTCCATGGCGGAAGGGGGACAAAGGTGAATTTGTCCTACTCAGGAGAGCGTTCA
SEQ ID NO:58: PsseA-hlyA-loxp-KnaR-loxp reverse primer
   TCTGACGTACACAGCAATTTTGCGTTACCTGTTAATCGAGATTGAAACACCGAAGAAAGGCCCACCCG
SEQ ID NO:59: Pssbp1-dapE-Cm forwad primer
   TTGGTTTCAGTGAATCCCGTTATCAGCAGTTTTTTGATGAGGTGTAGTCTATTTGTCCTACTCAGGAGAGCG
SEQ ID NO:60: Pssbp1-dapE-Cm reverse primer
   CAATATTTTGCCAGCCAGTCCATGCTTATTTCCTCTTACCGGAACGCTCACGAAGAAAGGCCCACCCG

## Claims

1. A modified bacterium, comprising a polynucleotide or an expression vector encoding a fusion polypeptide, wherein the fusion polypeptide comprises a signal peptide, an interleukin 10 (IL-10) polypeptide, and a peptide linker linking the signal peptide and the IL-10 polypeptide, wherein the peptide linker consists of 4 or 5 aspartic acid residues, and wherein the modified bacterium is capable of expressing and secreting the IL-10 polypeptide.

2. The modified bacterium of claim 1, wherein the IL-10 polypeptide is a human IL-10 polypeptide or a variant thereof.

3. The modified bacteria of claim 1 or 2, wherein the IL-10 polypeptide comprises the amino acid sequence of SEQ ID NO: 12; 13, 14 or 15.

4. The modified bacterium any one of claim 1-3, wherein the signal peptide comprises an amino acid sequence of SEQ ID NO: 16.

5. The modified bacterium any one of claim 1-4, wherein the fusion polypeptide comprises an amino acid sequence of SEQ ID NO: 17, 18, 19, 20, 21, 22, 23, or 24.

6. The modified bacterium any one of claim 1-5, wherein the bacterium further comprises one or more expression cassettes for expressing an essential gene, the expression of the essential gene is controlled by a strictly hypoxia inducible promoter, and the bacterium is deficient in at least one gene involved in or regulating an endogenous anti-oxidative stress response pathway or a functional expression product thereof, as compared to an unmodified starting strain.

7. The modified bacterium of claim 6, wherein the strictly hypoxia inducible promoter is ssbp1 promoter.

8. The modified bacterium of claim 6 or 7, wherein the expression product of the essential gene is responsible for the synthesis of 2,6-diaminopimelic acid (DAP) within the bacterium, and when cultured under aerobic conditions, the growth of the bacterium is dependent on the additional DAP or an analog thereof added to the medium.

9. The modified bacterium of claim 8, wherein the essential gene is selected from *dapA, dapB, dapD, dapE, argD, dapF,* and any combination thereof.

10. The modified bacterium of claim 8, wherein the essential gene is selected from *dapA* and *dapE.*

11. The modified bacterium of any one of claims 6-10, wherein the gene involved in or regulating the endogenous anti-oxidative stress response pathway is a gene of HtrA serine protease family.

12. The modified bacterium of any one of claims 6-11, wherein the essential gene is a gene naturally occurring in the bacterial chromosome, and the natural promoter of the essential gene is functionally replaced by the strictly hypoxia inducible promoter, whereby the expression of the essential gene in the bacterium is completely under the control of the strictly hypoxia inducible promoter.

13. The modified bacterium of any one of claims 6-11, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

14. The modified bacterium of any one of claims 6-13, which further comprises a pH regulated expression cassette, wherein the pH regulated expression cassette comprises a gene *hlyA* or *hlyE* encoding a bacteria-derived hemolysin protein under the control of a promoter that is active under acid pH condition.

15. The modified bacterium of claim 14, wherein the *hlyA* or *hlyE* is derived from *Listeria monocytogenes, Vibrio cholerae,* or *Escherichia coli.*

16. The modified bacterium of claim 14 or 15, wherein the promoter that is active under acid pH condition is sseA.

17. The modified bacterium of any one of claims 1-16, wherein the unmodified starting strain is a facultative anaerobic bacterium.

18. The modified bacterium of any one of claims 1-17, wherein the bacterium is a bacterium of *Enterobacteriaceae.*

19. The modified bacterium of claim 1-18, wherein the bacterium is *E. coli* or *Salmonella typhimurium.*

20. The modified bacteria of claim 19, wherein the starting strain is an *E. coli* Nissle strain or a *Salmonella typhimurium* SL7207 strain.

21. A pharmaceutical composition for treating a malignant tumor, comprising the modified bacterium of any one of claims 1-20.

22. A method for treating a malignant tumor, comprising administering an effective amount of the modified bacterium of any one of claims 1-20 or the pharmaceutical composition of claim 21 to a subject having the malignant tumor.

23. Use of the modified bacterium of any one of claims 1-20 or the pharmaceutical composition of claim 21 in the preparation of a medicament for treating a malignant tumor.
